(19) Europäisches Patentamt
European Patent Office
Office européen des brevets

(11) EP 3 689 867 B1

(12) EUROPEAN PATENT SPECIFICATION

(45) Date of publication and mention of the grant of the patent:
**15.12.2021 Bulletin 2021/50**

(21) Application number: **19154592.0**

(22) Date of filing: **30.01.2019**

(51) Int Cl.:
*C07D 405/14* (2006.01) *C07D 403/14* (2006.01)
*C07D 409/14* (2006.01) *C07D 493/04* (2006.01)
*H01L 51/50* (2006.01)

# (54) COMPOSITION, ORGANIC SEMICONDUCTOR LAYER AND ELECTRONIC DEVICE

ZUSAMMENSETZUNG, ORGANISCHE HALBLEITERSCHICHT UND ELEKTRONISCHE VORRICHTUNG

COMPOSITION, COUCHE SEMI-CONDUCTRICE ORGANIQUE ET DISPOSITIF ÉLECTRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**05.08.2020 Bulletin 2020/32**

(73) Proprietors:
- **Novaled GmbH**
  **01099 Dresden (DE)**
- **Samsung SDI Co., Ltd.**
  **Gyeonggi-do 17084 (KR)**

(72) Inventors:
- **GALAN, Elena**
  **01099 Dresden (DE)**
- **SCHULZE, Benjamin**
  **01099 Dresden (DE)**
- **CARDINALI, Francois**
  **01099 Dresden (DE)**
- **KIM, Hyungsun**
  **Gyeonggi-do 16678 (KR)**
- **LEE, Seungjae**
  **Gyeonggi-do 16678 (KR)**

(74) Representative: **Michalski Hüttermann & Partner Patentanwälte mbB**
**Speditionstraße 21**
**40221 Düsseldorf (DE)**

(56) References cited:
**WO-A1-2018/110958** **WO-A1-2019/009591**
**US-A1- 2015 171 340**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a composition, in particular to an organic semiconductor layer comprising the composition, suitable for use as an organic semiconductor layer for electronic devices.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

US 2015/0171340 A1 refers to condensed-cyclic compounds and organic light emitting devices including the condensed-cyclic compounds.

WO 2019/009591 A1 refers to organic compounds which has excellent thermal stability, electron transporting ability and light emitting ability, and to an organic electroluminescent device containing the same.

WO 2018/110958 A1 refers to compounds having an excellent light-emitting ability; and an organic electroluminescent element comprising the same in one or more organic material layers.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic electronic device, such as an organic light emitting diode, may be applied to a large-size flat panel display.

Further, development of an organic semiconductor layer being capable to have an extended life span at higher current density and thereby at higher brightness is needed.

There remains a need to improve performance of organic semiconductor materials, organic semiconductor layers, as well as organic electronic devices thereof, in particular to achieve increased lifetime through improving the characteristics of the compounds comprised therein.

### DISCLOSURE

An aspect of the present invention provides a composition comprising:

a) a compound of formula 1:

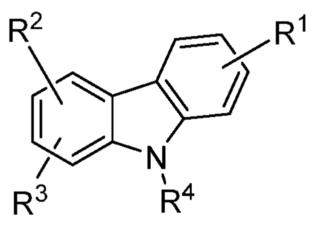

(1),

wherein

$R^1$ and $R^2$ are independently selected from H, D, CN, halogen, $C_1$ to $C_{12}$ alkyl, $C_1$ to $C_{12}$ alkoxy, substituted or unsubstituted $C_6$ to $C_{18}$ aryl, substituted or unsubstituted $C_3$ to $C_{24}$ heteroaryl, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl,

$C_6$ to $C_{24}$ aryl, $C_3$ to $C_{24}$ heteroaryl;

$R^3$ or $R^4$ has the general formula 2:

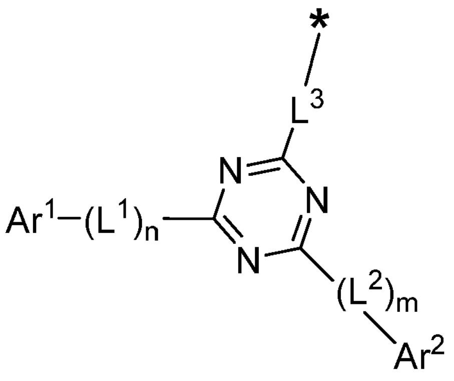

(2),

wherein the asterisk symbol "*" represents the binding position of the group $R^3$ or $R^4$;

$L^1$, $L^2$ and $L^3$ are independently selected from substituted or unsubstituted $C_6$ to $C_{13}$ arylene or substituted or unsubstituted $C_3$ to $C_{12}$ heteroarylene, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen;

n is 1 or 0;

m is 1 or 0;

$Ar^1$ is selected from substituted or unsubstituted $C_5$ to $C_{24}$ heteroaryl, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl; wherein $Ar^1$ is free of a spiro-group;

$Ar^2$ has the general formula 3:

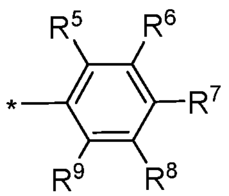

(3),

wherein the asterisk symbol "*" represents the binding position of the group $Ar^2$ to the moiety $L^2$; $R^5$ to $R^9$ are independently H, D, CN, halogen, $C_1$ to $C_{12}$ alkyl, $C_1$ to $C_{12}$ alkoxy, substituted or unsubstituted $C_6$ to $C_{12}$ aryl, substituted or unsubstituted $C_3$ to $C_{12}$ heteroaryl,
wherein the substituted aryl ring or substituted heteroaryl ring is substituted with one or more $C_1$ to $C_{12}$ alkyl, $C_6$ to $C_{12}$ aryl or $C_3$ to $C_{12}$ heteroaryl groups, wherein the substituents are bonded by a single bond to the substituted aryl ring or substituted heteroaryl ring;
or

$Ar^2$ is selected from a fused ring system comprising one to four substituted or unsubstituted 6 membered aryl rings and zero to two substituted or unsubstituted 5 to 7 membered heteroaryl rings, wherein the substituted aryl ring is substituted with $C_1$ to $C_{12}$ alkyl groups and the substituted heteroaryl ring is substituted with one or more $C_1$ to $C_{12}$ alkyl, $C_6$ to $C_{12}$ aryl or $C_3$ to $C_{12}$ heteroaryl groups;

with the provision that:

i) $R^3$ is formula 2 and $R^4$ is selected from $C_1$ to $C_{12}$ alkyl, substituted or unsubstituted $C_6$ to $C_{18}$ aryl, substituted

or unsubstituted $C_3$ to $C_{24}$ heteroaryl,
wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR',
wherein R' and R" are independently selected from $C_1$ to $C_{16}$ alkyl, $C_6$ to $C_{24}$ aryl, $C_3$ to $C_{24}$ heteroaryl;
or
ii) $R^4$ is formula 2 and $R^3$ is H or D; and

b) at least one organic metal complex, wherein the metal of the organic metal complex is selected from the group comprising alkali, alkaline earth or rare earth metal.

Hetero atoms, if not otherwise stated, can be individually selected from N, O, S, B, Si, P, Se, preferably from N, O and S and more preferred is N.

According to one embodiment, the composition comprising:

a) a compound of formula 1:

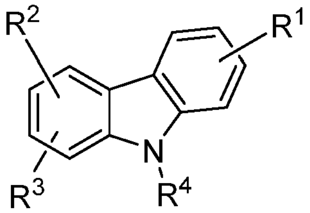

(1),

wherein

$R^1$ and $R^2$ are independently selected from H, D, CN, halogen, $C_1$ to $C_{12}$ alkyl, $C_1$ to $C_{12}$ alkoxy, substituted or unsubstituted $C_6$ to $C_{18}$ aryl comprising 1 to 3 of a 6-member aryl ring, substituted or unsubstituted $C_3$ to $C_{24}$ heteroaryl comprising 1 to 4 of a 6-member heteroaryl ring or at least one 6-member heteroaryl ring and 1 to 3 of a 6-member aryl ring, wherein rings can be fused, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl;

$R^3$ or $R^4$ has the general formula 2:

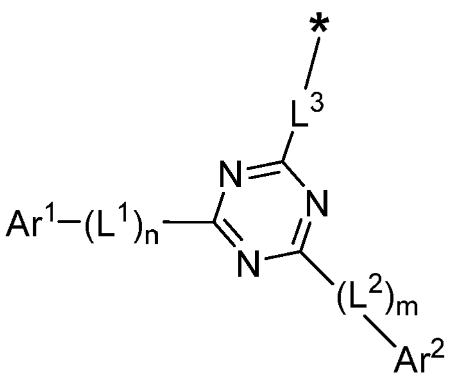

(2),

wherein the asterisk symbol "*" represents the binding position of the group $R^3$ or $R^4$;

$L^1$, $L^2$ and $L^3$ are independently selected from substituted or unsubstituted $C_6$ to $C_{13}$ arylene comprising 1 to 3 of a 6-member aryl ring, or substituted or unsubstituted $C_3$ to $C_{12}$ heteroarylene comprising 1 to 2 of a 6-member heteroaryl ring or one 6-member heteroaryl ring and one 6-member aryl ring, wherein rings thereof can be fused, wherein the substituents are selected from $C_1$ to $C_{12}$ alkyl, perhalogen-

ated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen;

n is 1 or 0;

m is 1 or 0;

$Ar^1$ is selected from substituted or unsubstituted $C_5$ to $C_{24}$ heteroaryl comprising at least 1 to 3 of a heteroaryl ring and 1 to 4 of an aryl ring, preferably at least 1 to 2 of a heteroaryl ring and 1 to 4 of an aryl ring, more preferred at least 1 to 2 of a 5-member heteroaryl ring and 1 to 2 of an 6-member aryl ring and in addition preferred one 5-member heteroaryl ring and 1 to 4 of an 6-member aryl ring, wherein rings thereof can be fused, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl; wherein $Ar^1$ is free of a spiro-group;

$Ar^2$ has the general formula 3:

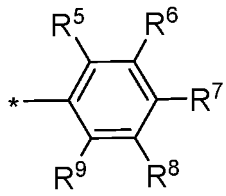

(3),

wherein the asterisk symbol "*" represents the binding position of the group $Ar^2$ to the moiety $L^2$;

$R^5$ to $R^9$ are independently H, D, CN, halogen, $C_1$ to $C_{12}$ alkyl, $C_1$ to $C_{12}$ alkoxy, substituted or unsubstituted $C_6$ to $C_{12}$ aryl comprising 1 to 2 of a 6-member aryl ring, substituted or unsubstituted $C_3$ to $C_{12}$ heteroaryl comprising 1 to 2 of a heteroaryl ring or at least one heteroaryl ring and one 6-member aryl ring, wherein rings thereof can be fused, wherein the substituted aryl ring or substituted heteroaryl ring is substituted with one or more $C_1$ to $C_{12}$ alkyl, $C_6$ to $C_{12}$ aryl or $C_3$ to $C_{12}$ heteroaryl groups, wherein the substituents are bonded by a single bond to the substituted aryl ring or substituted heteroaryl ring;

or

$Ar^2$ is selected from a fused ring system comprising one to four substituted or unsubstituted 6 membered aryl rings and zero to two substituted or unsubstituted 5 to 7 membered heteroaryl rings, wherein rings thereof can be fused, wherein the substituted aryl ring is substituted with $C_1$ to $C_{12}$ alkyl groups and the substituted heteroaryl ring is substituted with one or more $C_1$ to $C_{12}$ alkyl, $C_6$ to $C_{12}$ aryl or $C_3$ to $C_{12}$ heteroaryl groups;

with the provision that:

i) $R^3$ is formula 2 and $R^4$ is selected from $C_1$ to $C_{12}$ alkyl, substituted or unsubstituted $C_6$ to $C_{18}$ aryl, substituted or unsubstituted $C_3$ to $C_{24}$ heteroaryl,
wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR',
wherein R' and R" are independently selected from $C_1$ to $C_{16}$ alkyl, $C_6$ to $C_{24}$ aryl, $C_3$ to $C_{24}$ heteroaryl;
or
ii) $R^4$ is formula 2 and $R^3$ is H or D; and

b) at least one organic metal complex, wherein the metal of the organic metal complex is selected from the group comprising alkali, alkaline earth or rare earth metal

If not otherwise stated H can represent hydrogen or deuterium.

The composition can be an organic semiconductor.

According to one embodiment, the composition comprising:

a) a compound of formula 1:

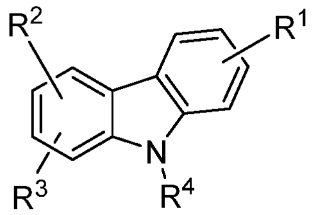

(1),

wherein

$R^1$ and $R^2$ are independently selected from H, D, CN, halogen, $C_1$ to $C_{12}$ alkyl, $C_1$ to $C_{12}$ alkoxy, substituted or unsubstituted $C_6$ to $C_{18}$ aryl, substituted or unsubstituted $C_3$ to $C_{24}$ heteroaryl, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl;

$R^3$ or $R^4$ has the general formula 2:

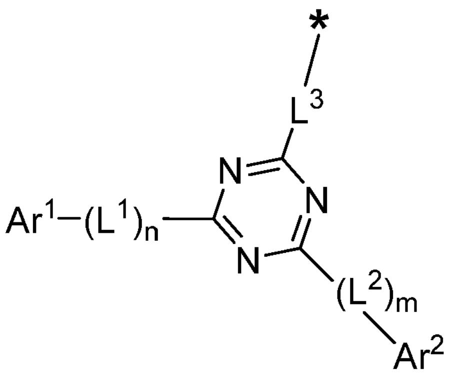

(2),

wherein the asterisk symbol "*" represents the binding position of the group $R^3$ or $R^4$;

$L^1$, $L^2$ and $L^3$ are independently selected from substituted or unsubstituted $C_6$ to $C_{13}$ arylene or substituted or unsubstituted $C_3$ to $C_{12}$ heteroarylene, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen;

n is 1 or 0;

m is 1 or 0;

$Ar^1$ is selected from substituted or unsubstituted $C_5$ to $C_{24}$ heteroaryl, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl;

$Ar^2$ has the general formula 3:

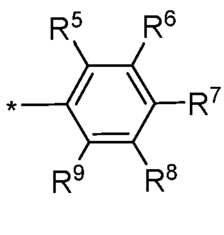

(3),

wherein the asterisk symbol "*" represents the binding position of the group $Ar^2$ to the moiety $L^2$;
$R^5$ to $R^9$ are independently H, D, CN, halogen, $C_1$ to $C_{12}$ alkyl, $C_1$ to $C_{12}$ alkoxy, substituted or unsubstituted $C_6$ to $C_{12}$ aryl, substituted or unsubstituted $C_3$ to $C_{12}$ heteroaryl,
wherein the substituted aryl ring or substituted heteroaryl ring is substituted with one or more $C_1$ to $C_{12}$ alkyl, $C_6$ to $C_{12}$ aryl or $C_3$ to $C_{12}$ heteroaryl groups, wherein the substituents are bonded by a single bond to the substituted aryl ring or substituted heteroaryl ring;
or

$Ar^2$ is selected from a fused ring system comprising one to four substituted or unsubstituted 6 membered aryl rings and zero to two substituted or unsubstituted 5 to 7 membered heteroaryl rings, wherein the substituted aryl ring is substituted with $C_1$ to $C_{12}$ alkyl groups and the substituted heteroaryl ring is substituted with one or more $C_1$ to $C_{12}$ alkyl, $C_6$ to $C_{12}$ aryl or $C_3$ to $C_{12}$ heteroaryl groups;

with the provision that:

i) $R^3$ is formula 2 and $R^4$ is selected from $C_1$ to $C_{12}$ alkyl, substituted or unsubstituted $C_6$ to $C_{18}$ aryl, substituted or unsubstituted $C_3$ to $C_{24}$ heteroaryl,
wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR',
wherein R' and R" are independently selected from $C_1$ to $C_{16}$ alkyl, $C_6$ to $C_{24}$ aryl, $C_3$ to $C_{24}$ heteroaryl;
or
ii) $R^4$ is formula 2 and $R^3$ is H or D;

wherein the compound of formula 1 is free of a spiro-group; and
b) at least one organic metal complex, wherein the metal of the organic metal complex is selected from the group comprising alkali, alkaline earth or rare earth metal.

According to one embodiment, the composition comprising:

a) a compound of formula 1:

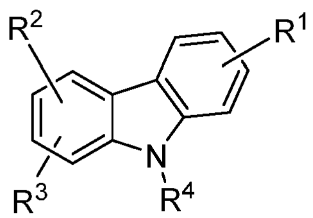
(1),

wherein

$R^1$ and $R^2$ are independently selected from H, D, CN, halogen, $C_1$ to $C_{12}$ alkyl, $C_1$ to $C_{12}$ alkoxy, substituted or unsubstituted $C_6$ to $C_{18}$ aryl, substituted or unsubstituted $C_3$ to $C_{24}$ heteroaryl, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl;
$R^3$ or $R^4$ has the general formula 2:

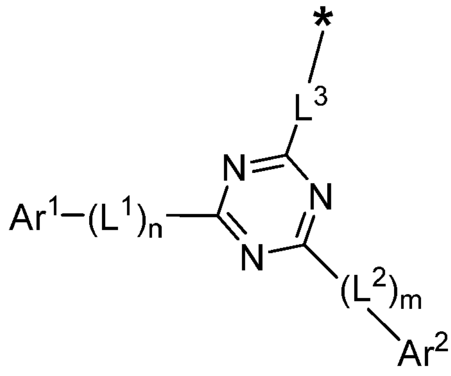

(2),

wherein the asterisk symbol "*" represents the binding position of the group $R^3$ or $R^4$;

$L^1$, $L^2$ and $L^3$ are independently selected from substituted or unsubstituted $C_6$ to $C_{13}$ arylene or substituted or unsubstituted $C_3$ to $C_{12}$ heteroarylene, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen;

n is 1 or 0;

m is 1 or 0;

$Ar^1$ is selected from substituted or unsubstituted $C_5$ to $C_{24}$ heteroaryl, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl,

$Ar^2$ perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl; has the general formula 3:

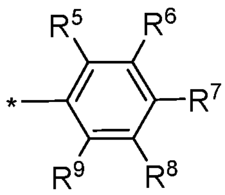

(3),

wherein the asterisk symbol "*" represents the binding position of the group $Ar^2$ to the moiety $L^2$;
$R^5$ to $R^9$ are independently H, D, CN, halogen, $C_1$ to $C_{12}$ alkyl, $C_1$ to $C_{12}$ alkoxy, unsubstituted $C_6$ to $C_{12}$ aryl, or unsubstituted $C_3$ to $C_{12}$ heteroaryl;
or

$Ar^2$ is selected from a fused ring system comprising one to four unsubstituted 6 membered aryl rings and zero to two unsubstituted 5 to 7 membered heteroaryl rings;

with the provision that:

i) $R^3$ is formula 2 and $R^4$ is selected from $C_1$ to $C_{12}$ alkyl, substituted or unsubstituted $C_6$ to $C_{18}$ aryl, substituted or unsubstituted $C_3$ to $C_{24}$ heteroaryl,
wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR',
wherein R' and R" are independently selected from $C_1$ to $C_{16}$ alkyl, $C_6$ to $C_{24}$ aryl, $C_3$ to $C_{24}$ heteroaryl;
or
ii) $R^4$ is formula 2 and $R^3$ is H or D;

wherein the compound of formula 1 is free of a spiro-group; and

b) at least one organic metal complex, wherein the metal of the organic metal complex is selected from the group comprising alkali, alkaline earth or rare earth metal.

According to one embodiment, the composition comprising:

a) a compound of formula 1:

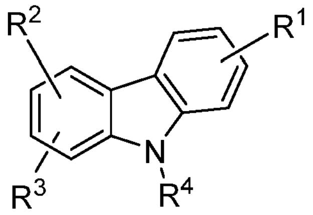

(1),

wherein

| | |
|---|---|
| $R^1$ and $R^2$ | are independently selected from H, D, CN, halogen, $C_1$ to $C_{12}$ alkyl, $C_1$ to $C_{12}$ alkoxy, unsubstituted $C_6$ to $C_{18}$ aryl, unsubstituted $C_3$ to $C_{24}$ heteroaryl; |
| $R^3$ or $R^4$ | has the general formula 2: |

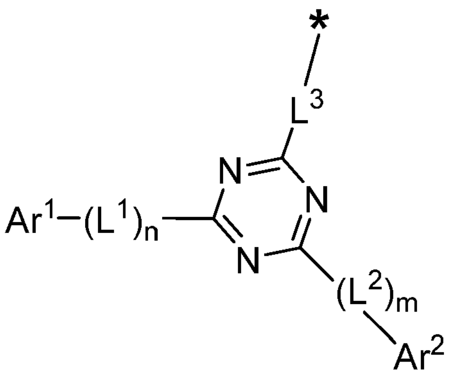

(2),

wherein the asterisk symbol "*" represents the binding position of the group $R^3$ or $R^4$;

| | |
|---|---|
| $L^1$, $L^2$ and $L^3$ | are independently selected from unsubstituted $C_6$ to $C_{13}$ arylene or unsubstituted $C_3$ to $C_{12}$ heteroarylene; |
| n | is 1 or 0; |
| m | is 1 or 0; |
| $Ar^1$ | is selected from unsubstituted $C_5$ to $C_{24}$ heteroaryl; |

wherein $Ar^1$ is free of a spiro-group or the compound of formula 1 is free of a spiro-group;

$Ar^2$ has the general formula 3:

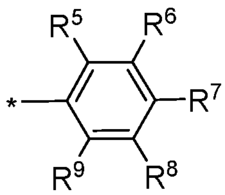

(3),

wherein the asterisk symbol "*" represents the binding position of the group $Ar^2$ to the moiety $L^2$;

$R^5$ to $R^9$ are independently H, D, CN, halogen, $C_1$ to $C_{12}$ alkyl, $C_1$ to $C_{12}$ alkoxy, unsubstituted $C_6$ to $C_{12}$ aryl, unsubstituted $C_3$ to $C_{12}$ heteroaryl;

or

$Ar^2$ is selected from a fused ring system comprising one to four unsubstituted 6 membered aryl rings and zero to two unsubstituted 5 to 7 membered heteroaryl rings;

with the provision that:

iii) $R^3$ is formula 2 and $R^4$ is selected from $C_1$ to $C_{12}$ alkyl, unsubstituted $C_6$ to $C_{18}$ aryl, unsubstituted $C_3$ to $C_{24}$ heteroaryl;
or
iv) $R^4$ is formula 2 and $R^3$ is H or D; and
b) at least one organic metal complex, wherein the metal of the organic metal complex is selected from the group comprising alkali, alkaline earth or rare earth metal.

According to another embodiment of the compound of formula 1, wherein the hetero atom of the $C_3$ to $C_{24}$ heteroaryl, $C_3$ to $C_{12}$ heteroaryl, $C_3$ to $C_{12}$ heteroarylene, may be selected from N, O or S.

According to another embodiment of the compound of formula 1, wherein the hetero atom of the $C_3$ to $C_{24}$ heteroaryl, $C_3$ to $C_{12}$ heteroaryl, $C_3$ to $C_{12}$ heteroarylene, may be selected from N or O.

According to another embodiment of the compound of formula 1, wherein $R^1$, $R^2$ may be independently selected from substituted or unsubstituted $C_1$ to $C_{16}$ alkyl, substituted or unsubstituted $C_6$ to $C_{12}$ aryl, substituted or unsubstituted $C_3$ to $C_{17}$ heteroaryl, wherein the substituents of the substituted $C_6$ to $C_{12}$ aryl and substituted $C_3$ to $C_{17}$ heteroarylene are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl.

According to another embodiment of the compound of formula 1, wherein $R^1$, $R^2$ may be independently selected from H, D, unsubstituted $C_6$ to $C_{18}$ aryl, or unsubstituted $C_3$ to $C_{24}$ heteroaryl. According to another embodiment of the compound of formula 1, wherein $R^1$, $R^2$ may be preferably independently selected from H, D, or unsubstituted $C_6$ to $C_{18}$ aryl. According to another embodiment of the compound of formula 1, wherein $R^1$, $R^2$ may be further preferred independently selected from H, D, or unsubstituted $C_6$ aryl. According to another embodiment of the compound of formula 1, wherein $R^1$ may in addition preferred independently selected from H or D and $R^2$ is a unsubstituted $C_6$ aryl. According to another embodiment of the compound of formula 1, wherein $R^2$ is in addition preferred selected from H or D and $R^1$ is an unsubstituted $C_6$ aryl.

According to another embodiment of the compound of formula 1, wherein $R^1$, $R^2$ are independently selected from H, D, phenyl, biphenyl, terphenyl naphthyl, phenanthrenyl, pyridyl, quinolinyl, quinazolinyl.

According to another embodiment of the compound of formula 1, wherein $R^1$, $R^2$ are independently selected from H, D, phenyl, biphenyl, terphenyl, naphthyl, phenanthrenyl.

According to another embodiment of the compound of formula 1, wherein $R^1$, $R^2$ are independently selected from H, D, unsubstituted $C_6$ to $C_{12}$ aryl, unsubstituted $C_3$ to $C_{17}$ heteroaryl, and preferably from H, D, phenyl and biphenyl and more preferred from H, D, and phenyl.

According to another embodiment of the compound of formula 1, wherein $Ar^1$ may be selected from substituted or unsubstituted $C_5$ to $C_{24}$ heteroaryl, wherein the substituents of the substituted $C_5$ to $C_{24}$ heteroaryl may be selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl.

According to another embodiment of the compound of formula 1, wherein $Ar^1$ may be selected from substituted or unsubstituted $C_5$ to $C_{18}$ heteroaryl, wherein the substituents of the substituted $C_5$ to $C_{18}$ heteroaryl may be selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl.

According to another embodiment of the compound of formula 1, wherein $Ar^1$ may be selected from substituted or unsubstituted $C_5$ to $C_{18}$ heteroaryl, wherein the substituents of the substituted $C_5$ to $C_{18}$ heteroaryl may be selected from $C_1$ to $C_{12}$ alkyl, perhalogenated $C_1$ to $C_{12}$ alkyl, $C_6$ to $C_{24}$ aryl, perhalogenated $C_6$ to $C_{24}$ aryl, $C_3$ to $C_{24}$ heteroaryl, CN, halogen.

According to another embodiment of the compound of formula 1, wherein $Ar^1$ may be selected from substituted

or unsubstituted $C_5$ to $C_{18}$ heteroaryl, wherein the substituents of the substituted $C_5$ to $C_{18}$ heteroaryl may be selected from $C_1$ to $C_{12}$ alkyl, $C_6$ to $C_{24}$ aryl, $C_3$ to $C_{24}$ heteroaryl.

According to another embodiment of the compound of formula 1, wherein $Ar^1$ may be selected from unsubstituted $C_5$ to $C_{24}$ heteroaryl.

According to another embodiment of the compound of formula 1, wherein $Ar^1$ may be selected from substituted or unsubstituted $C_5$ to $C_{24}$ heteroaryl comprising at least one heteroatom selected from O, S, Se or N, preferably the at least one heteroatom is selected from O, S, Se and in addition preferred the at least one heteroatom is O.

According to another embodiment of the compound of formula 1, wherein $Ar^1$ may be selected from unsubstituted $C_5$ to $C_{18}$ heteroaryl.

According to another embodiment of the compound of formula 1, wherein $Ar^1$ may have the general formula 4:

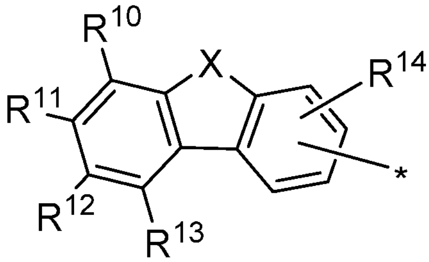
(4),

wherein the asterisk symbol "*" represents the binding position of the group $Ar^1$ to the moiety $L^1$;

| | |
|---|---|
| X | is selected from O, S, Se or $NR^{15}$, wherein $R^{15}$ is independently selected from $C_1$ to $C_{12}$ alkyl, perhalogenated $C_1$ to $C_{12}$ alkyl, $C_6$ to $C_{24}$ aryl, perhalogenated $C_6$ to $C_{24}$ aryl, $C_3$ to $C_{24}$ heteroaryl; |
| $R^{10}$ to $R^{14}$ | are independently selected from H, D, CN, halogen, $C_1$ to $C_{12}$ alkyl, $C_1$ to $C_{12}$ alkoxy, $C_6$ to $C_{18}$ aryl or $C_3$ to $C_{24}$ heteroaryl; |

wherein preferably $R^{10}$ and $R^{11}$, $R^{11}$ and $R^{12}$ or $R^{12}$ and $R^{13}$ form a 5 to 7 membered substituted or unsubstituted aryl ring, or a 5 to 7 membered substituted or unsubstituted heteroaryl ring, and in addition preferred $R^{10}$ and $R^{11}$, $R^{11}$ and $R^{12}$ or $R^{12}$ and $R^{13}$ form two fused 5 to 7 membered substituted or unsubstituted aryl rings, or two fused 5 to 7 membered substituted or unsubstituted heteroaryl rings,
wherein the substituents are selected from $C_1$ to $C_{12}$ alkyl, $C_6$ to $C_{18}$ aryl or $C_3$ to $C_{24}$ heteroaryl groups.

According to another embodiment of the compound of formula 1, wherein X in general formula 4 can be selected from O, S or $NR^{15}$, more preferred X is selected from O or S, and also preferred X is O.

According to another embodiment of the compound of formula 1, wherein X in general formula 4 can be selected from O, S or $NR^{15}$, and $R^{10}$ to $R^{14}$ are H.

According to another embodiment of the compound of formula 1, wherein $Ar^1$ may be independently selected from the group comprising D1 to D16:

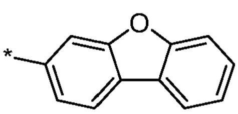
D1,

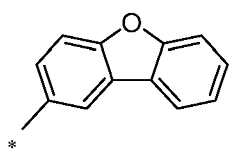
D2,

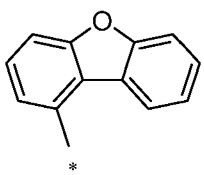
D3,

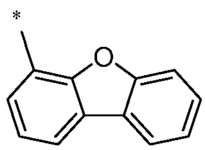
D4,

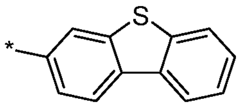
D5,

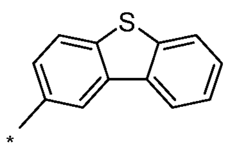
D6,

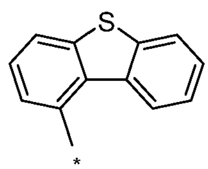
D7,

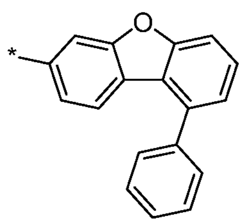
D8,

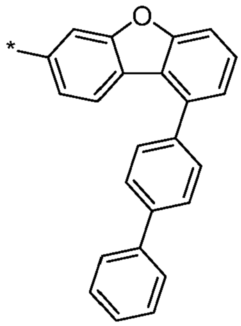
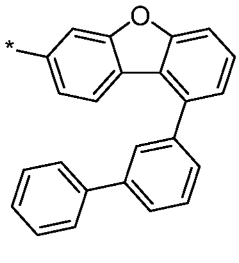
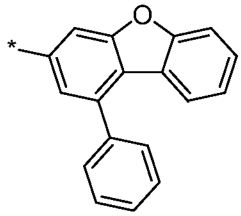
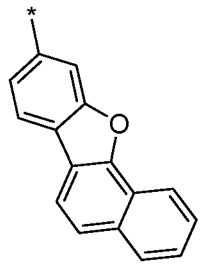

D9, D10, D11, D12,

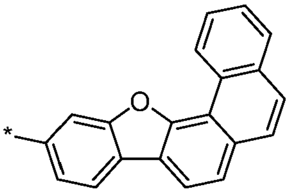
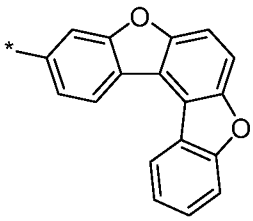
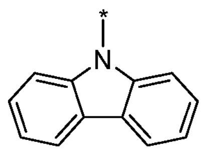
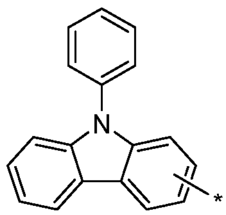

D13, D14, D15, D16.

In another embodiment, $Ar^1$ may be selected from D1 to D14. In another embodiment, $Ar^1$ may be preferably selected from D1, D3, D5, D7, D8 to D14. In another embodiment, $Ar^1$ may be more preferably selected from D1 to D7. In another embodiment, $Ar^1$ may be more preferably selected from D1 or D3. In another embodiment, $Ar^1$ may be furthermore preferably selected from D1.

According to another embodiment of the compound of formula 1, wherein $Ar^2$ may have the general formula 3:

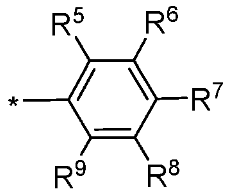

(3),

wherein the asterisk symbol "*" represents the binding position of the group $Ar^2$ to the moiety $L^2$;
$R^5$ to $R^9$ are independently H, D, CN, halogen, $C_1$ to $C_{12}$ alkyl, $C_1$ to $C_{12}$ alkoxy, substituted or unsubstituted $C_6$ to $C_{12}$ aryl, substituted or unsubstituted $C_3$ to $C_{12}$ heteroaryl,

wherein the substituted aryl ring or substituted heteroaryl ring is substituted with one or more $C_1$ to $C_{12}$ alkyl, $C_6$ to $C_{12}$ aryl or $C_3$ to $C_{12}$ heteroaryl groups,
wherein the substituents are bonded by a single bond to the substituted aryl ring or substituted heteroaryl ring.

According to another embodiment of the compound of formula 1, wherein $Ar^2$ may have the general formula 3:

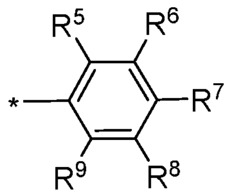

(3),

wherein the asterisk symbol "*" represents the binding position of the group $Ar^2$ to the moiety $L^2$;
$R^5$ to $R^9$ are independently H, D, CN, halogen, $C_1$ to $C_{12}$ alkyl, $C_1$ to $C_{12}$ alkoxy, substituted or unsubstituted $C_6$ to $C_{12}$ aryl, substituted or unsubstituted $C_3$ to $C_{12}$ heteroaryl,

wherein the substituted aryl ring or substituted heteroaryl ring is substituted with one or more $C_1$ to $C_{12}$ alkyl, $C_6$ to $C_{12}$ aryl or $C_3$ to $C_{12}$ heteroaryl groups,
wherein the substituents are bonded by a single bond to the substituted aryl ring or substituted heteroaryl ring; and wherein the group $Ar^2$ can be free of a fluorene group.

According to another embodiment of the compound of formula 1, wherein $Ar^2$ may be selected from a fused ring system comprising one to four substituted or unsubstituted 6 membered aryl rings and zero to two substituted or unsubstituted 5 to 7 membered heteroaryl rings,
wherein the substituted aryl ring is substituted with $C_1$ to $C_{12}$ alkyl groups and the substituted heteroaryl ring is substituted with one or more $C_1$ to $C_{12}$ alkyl, $C_6$ to $C_{12}$ aryl or $C_3$ to $C_{12}$ heteroaryl groups;
and $R^3$ is formula 2 and $R^4$ is selected from $C_1$ to $C_{12}$ alkyl, substituted or unsubstituted $C_6$ to $C_{18}$ aryl, substituted or unsubstituted $C_3$ to $C_{24}$ heteroaryl,
wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR',
wherein R' and R" are independently selected from $C_1$ to $C_{16}$ alkyl, $C_6$ to $C_{24}$ aryl, $C_3$ to $C_{24}$ heteroaryl.

According to another embodiment of the compound of formula 1, wherein $Ar^2$ may be selected from a fused ring system comprising one to four substituted or unsubstituted 6 membered aryl rings and zero to two substituted or unsubstituted 5 to 7 membered heteroaryl rings,
wherein the substituted aryl ring is substituted with $C_1$ to $C_{12}$ alkyl groups and the substituted heteroaryl ring is substituted with one or more $C_1$ to $C_{12}$ alkyl, $C_6$ to $C_{12}$ aryl or $C_3$ to $C_{12}$ heteroaryl groups;
and $R^4$ is formula 2 and $R^3$ is H or D.

According to another embodiment of the compound of formula 1, wherein $Ar^2$ is free of a fluorene group.

According to another embodiment of the compound of formula 1, wherein $Ar^2$ can be selected from the group comprising E1 to E44:

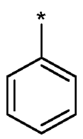
E1,
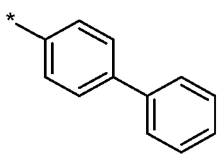
E2,
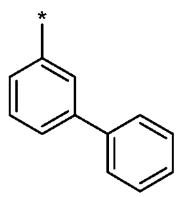
E3,
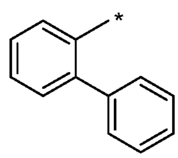
E4,
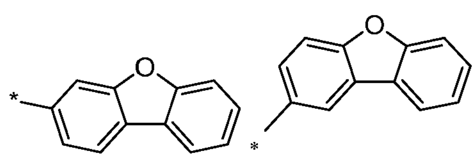
E5, E6,

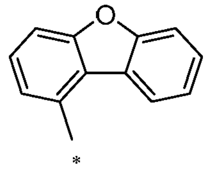
E7,
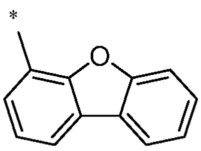
E8,
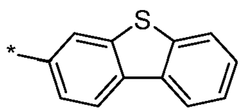
E9,
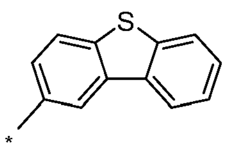
E10,
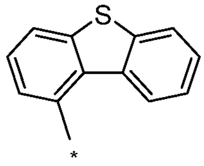
E11,

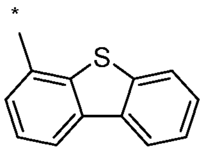 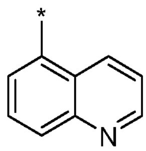 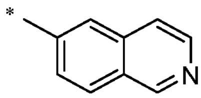 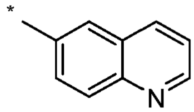 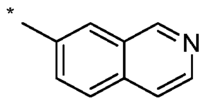

E12, E13, E14, E15, E16,

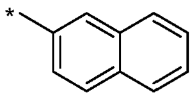 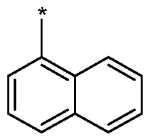 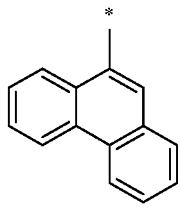 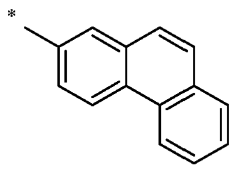 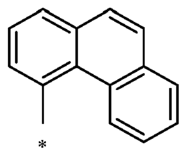

E17, E18, E19, E20, E21,

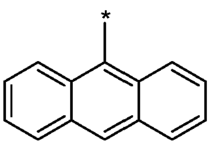 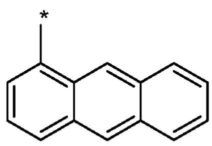 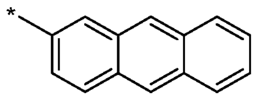 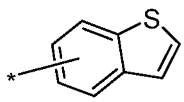 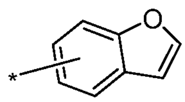

E22, E23, E24, E25, E26,

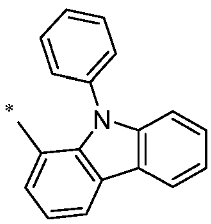 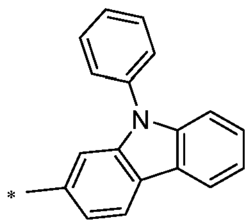 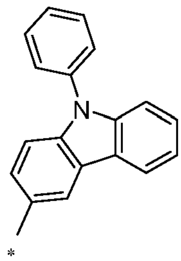 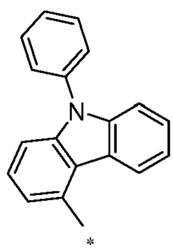 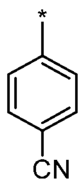 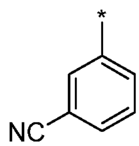

E27, E28, E29, E30, E31, E32,

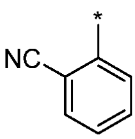 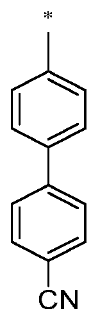 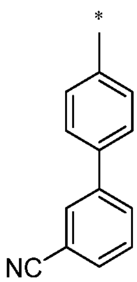 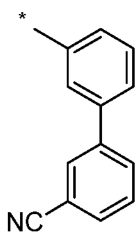 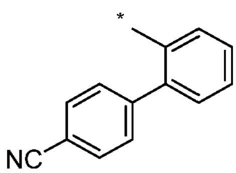 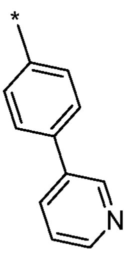 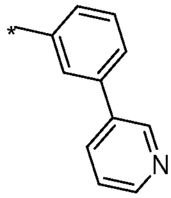

E33, E34, E35, E36, E37, E38, E39,

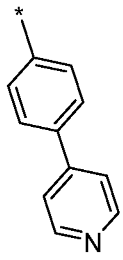 E40, 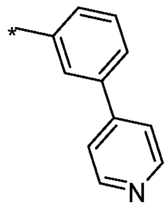 E41, 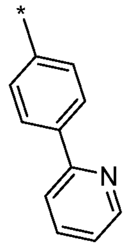 E42, 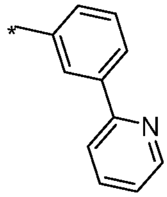 E43, 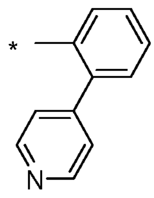 E44.

According to another embodiment of the compound of formula 1, wherein $Ar^2$ can be preferably selected from the group comprising E1 to E26 and E31 to E44. According to another embodiment of the compound of formula 1, wherein $Ar^2$ can be more preferably selected from the group comprising E1 to E12, E17 to E19 and E31 to E37. According to another embodiment of the compound of formula 1, wherein $Ar^2$ can be also preferred selected from the group comprising E1 to E12, E17 to E19 and E31 to E37. According to another embodiment of the compound of formula 1, wherein $Ar^2$ can be most preferred selected from the group comprising E1 to E4 and E17 to E18.

According to another embodiment of the compound of formula 1, wherein

$L^1$, $L^2$ and $L^3$ are independently selected from substituted or unsubstituted $C_6$ to $C_{13}$ arylene or substituted or unsubstituted $C_3$ to $C_{12}$ heteroarylene, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen.

According to another embodiment of the compound of formula 1, wherein

$L^1$, $L^2$ and $L^3$ are independently selected from substituted or unsubstituted $C_6$ arylene or substituted or unsubstituted $C_3$ to $C_5$ heteroarylene, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen.

According to another embodiment of the compound of formula 1, wherein

$L^1$, $L^2$ and $L^3$ are independently selected from unsubstituted $C_6$ to $C_{13}$ arylene or unsubstituted $C_3$ to $C_{12}$ heteroarylene, preferably independently selected from unsubstituted $C_6$ arylene or unsubstituted $C_3$ to $C_5$ heteroarylene.

According to another embodiment of the compound of formula 1, wherein

a) $L^1$, $L^2$ and/or $L^3$ may be independently selected from the group comprising F1 to F11:

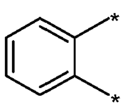 (F1), 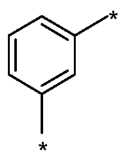 (F2), 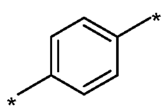 (F3), 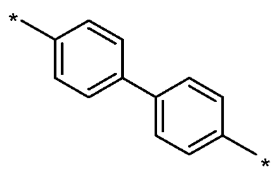 (F4),

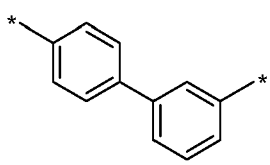 (F5), 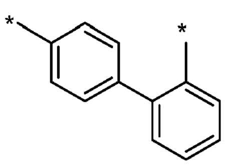 (F6), 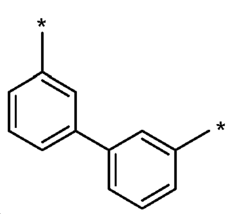 (F7), 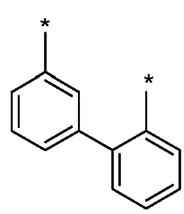 (F8),

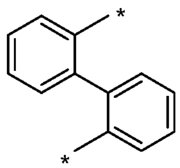 (F9), 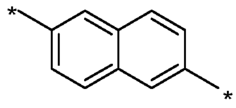 (F10), 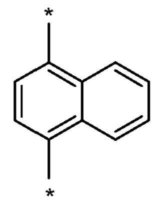 (F11);

preferably $L^1$, $L^2$ and/or $L^3$ can be selected from F2, F3, F4, F5 or F7, more preferably $L^1$, $L^2$ and/or $L^3$ can be selected from F2 , F3, F5 or F7, more preferred $L^1$, $L^2$ and/or $L^3$ can be selected from F2 or F3; or

b) for $L^1$ n is 1 and for $L^2$ m is 0, $L^1$ may be selected from F1 to F11, preferably $L^1$ can be selected from F2, F3, F4, F5 or F7, more preferably $L^1$ can be selected from F2 , F3, F5 or F7, more preferred $L^1$ can be selected from F2 or F3; or

c) for $L^1$ n is 0 and for $L^2$ m can be 1, $L^2$ is selected from F1 to F11, preferably $L^2$ can be selected from F2, F3, F4, F5 or F7, more preferably $L^2$ can be selected from F2 , F3, F5 or F7, more preferred $L^2$ can be selected from F2 or F3; or

d) for $L^1$ n is 0 and for $L^2$ m is 0 and $L^3$ may be selected from F1 to F11, preferably $L^3$ can be selected from F2, F3, F4, F5 or F7, more preferably $L^3$ can be selected from F2 , F3, F5 or F7, more preferred $L^3$ is selected from F2 or F3.

According to another embodiment of the compound of formula 1, wherein n = 1 and m = 0. According to another embodiment of the compound of formula 1, wherein n = 0 and m = 1. According to another more preferred embodiment of the compound of formula 1, wherein n = 0 and m = 0.

According to another embodiment of the compound of formula 1, wherein n = 1 and m = 0 and $L^3$ is a substituted or unsubstituted phenylene or substituted or unsubstituted biphenylene group, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen. According to another embodiment of the compound of formula 1, wherein n = 0 and m = 1 and $L^3$ is a substituted or unsubstituted phenylene or substituted or unsubstituted biphenylene group, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen. According to another more preferred embodiment of the compound of formula 1, wherein n = 0 and m = 0 and $L^3$ is a substituted or unsubstituted phenylene or substituted or unsubstituted biphenylene group, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen.

According to another embodiment of the compound of formula 1, wherein n = 1 and m = 0 and $L^3$ is a unsubstituted phenylene or unsubstituted biphenylene group. According to another embodiment of the compound of formula 1, wherein n = 0 and m = 1 and $L^3$ is a unsubstituted phenylene or unsubstituted biphenylene group. According to another more preferred embodiment of the compound of formula 1, wherein n = 0 and m = 0 and $L^3$ is a unsubstituted phenylene or unsubstituted biphenylene group.

According to another embodiment of the compound of formula 1 may be selected from G1 to G31:

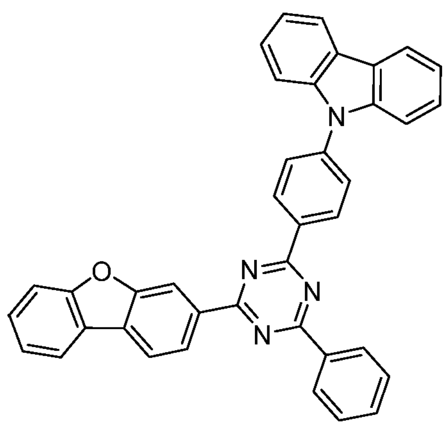

G1,

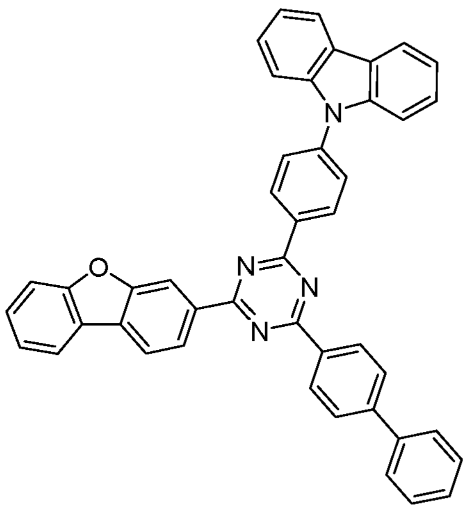

G2,

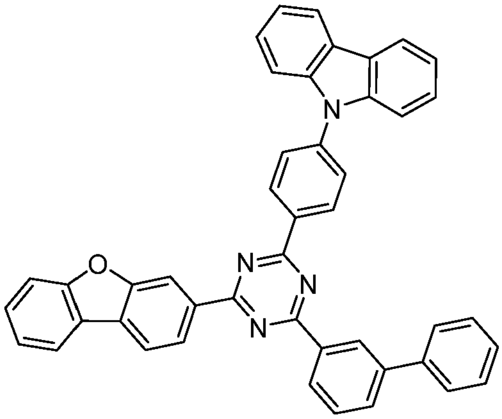
G3,

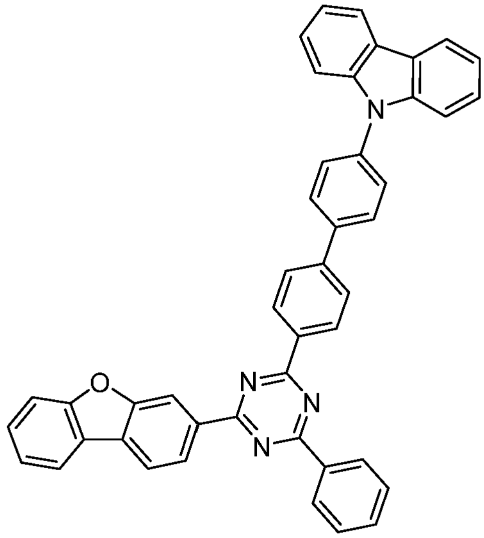
G4,

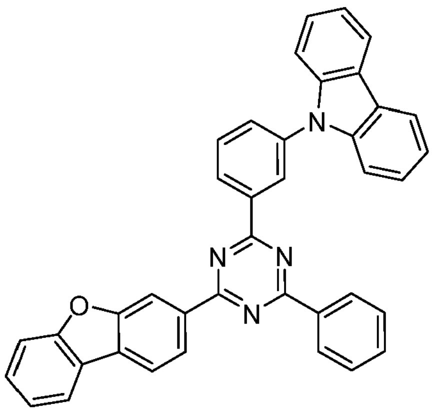
G5,

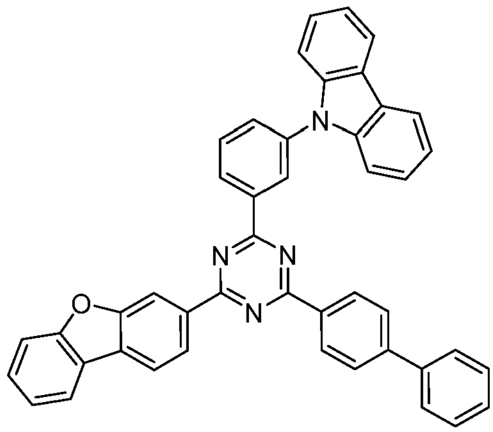
G6,

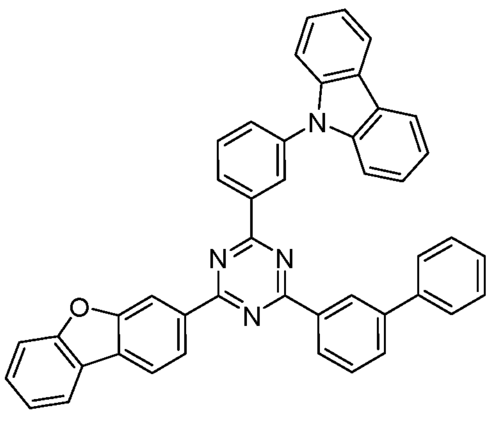
G7,

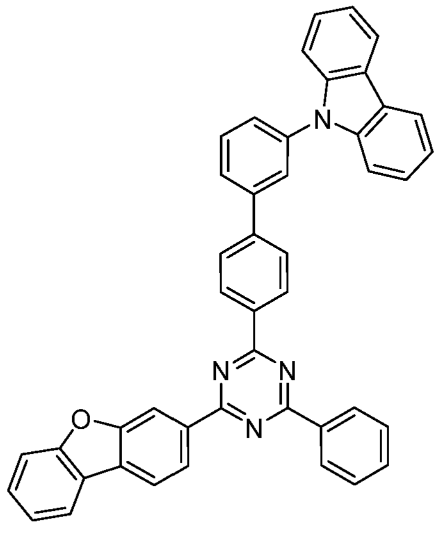
G8,

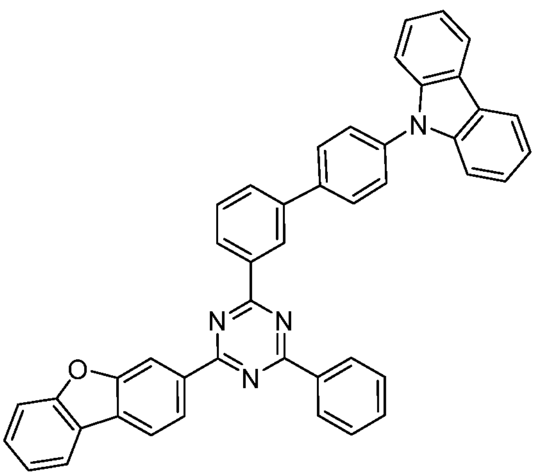

G9,

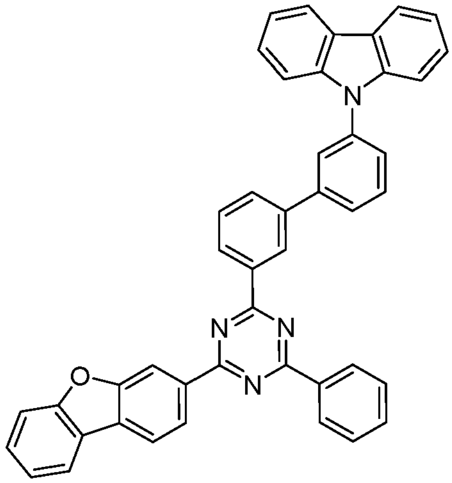

G10,

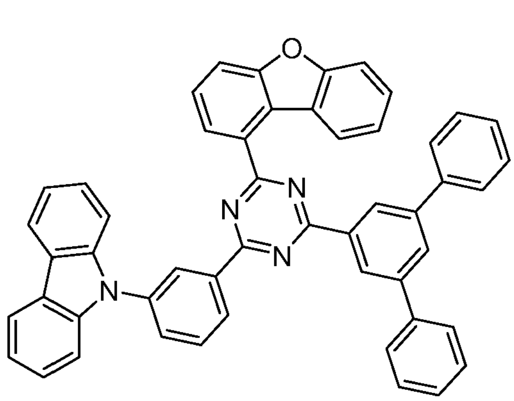

G11,

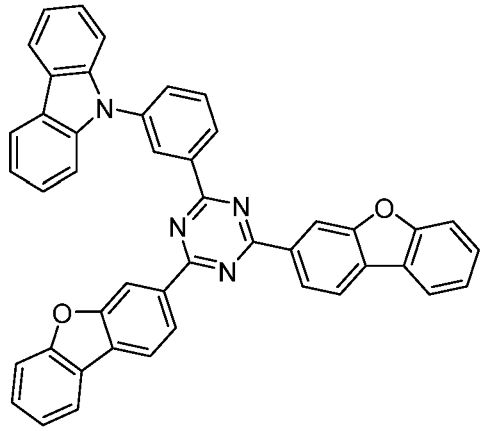

G12,

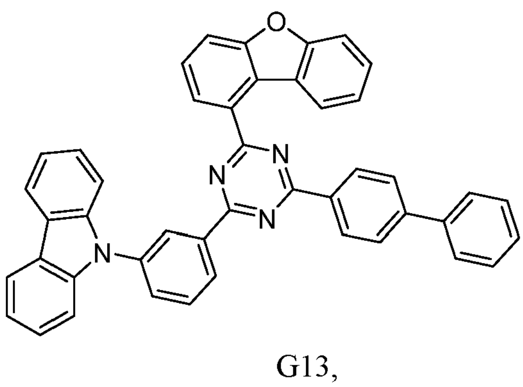

G13,

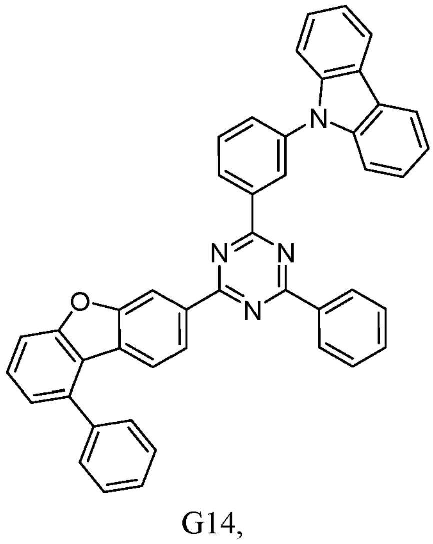

G14,

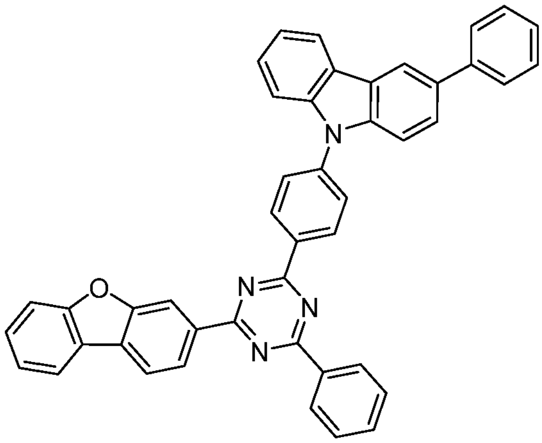

G15,

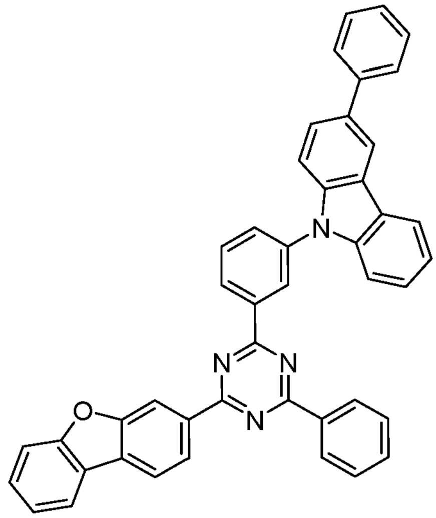

G16,

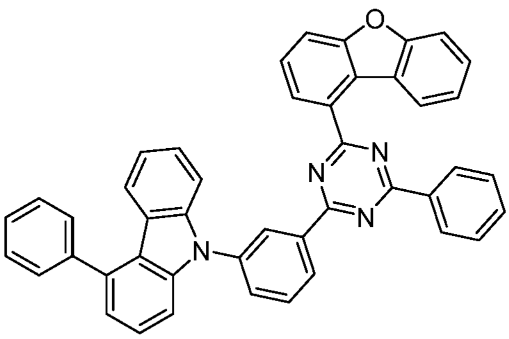

G17,

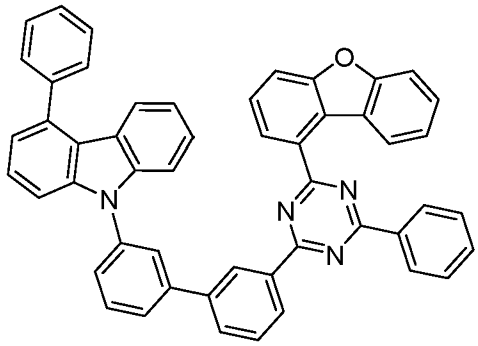

G18,

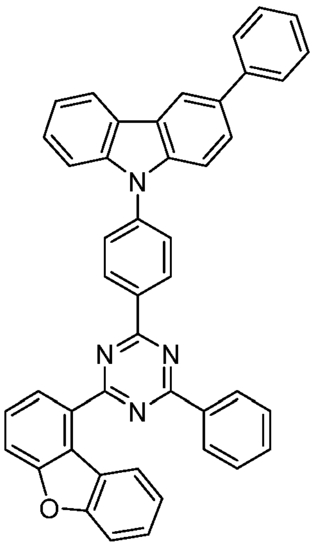

G19,

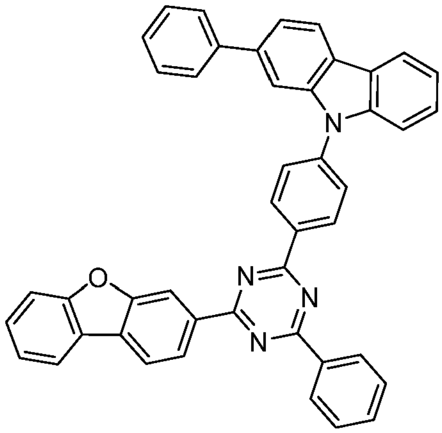

G20,

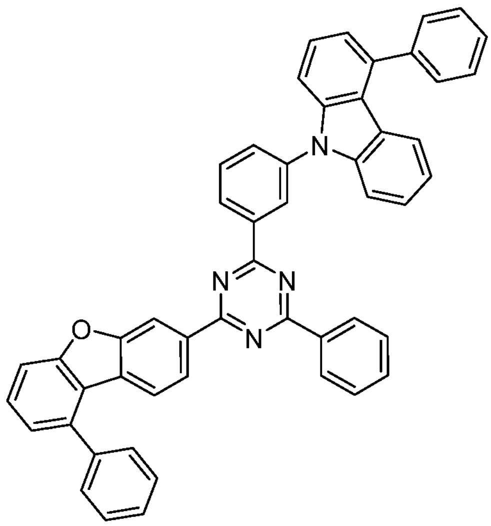

G21,

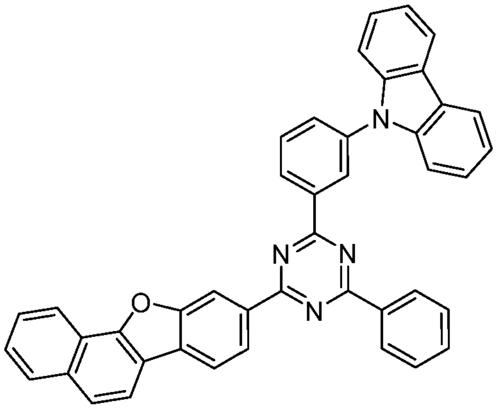

G22,

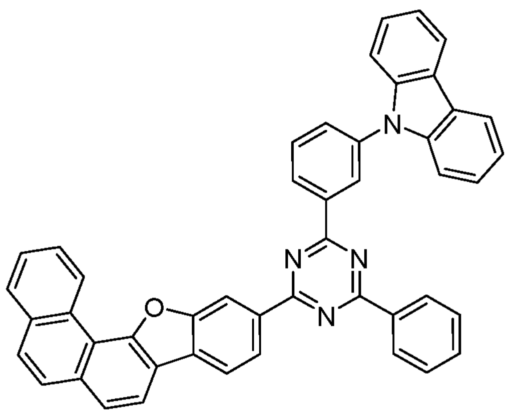

G23,

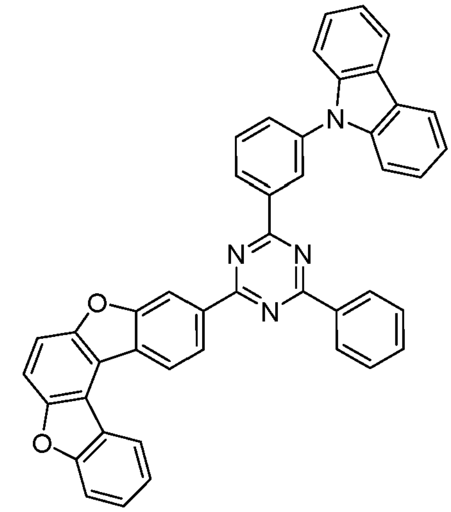

G24,

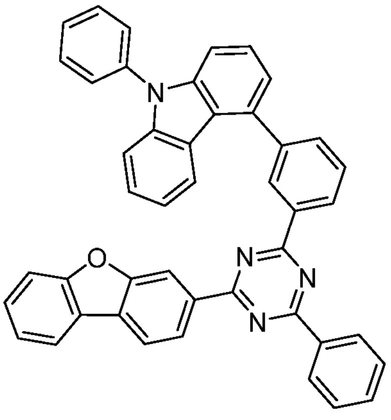

G25,

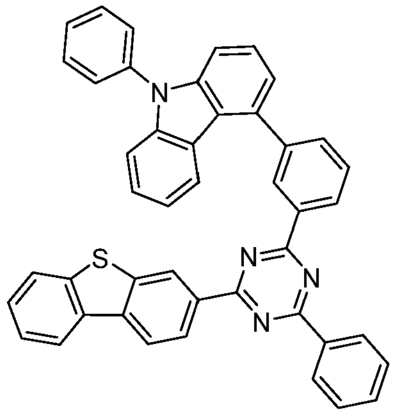

G26,

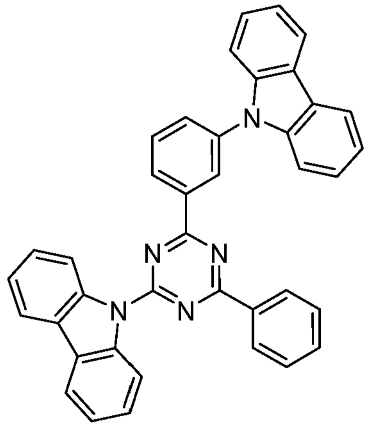

G27,

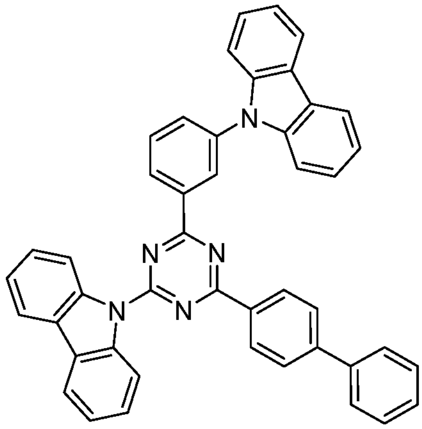

G28,

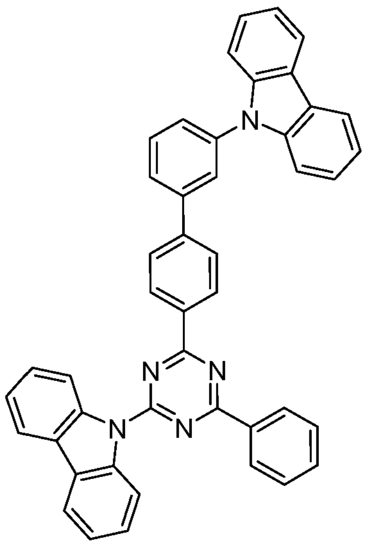

G29,

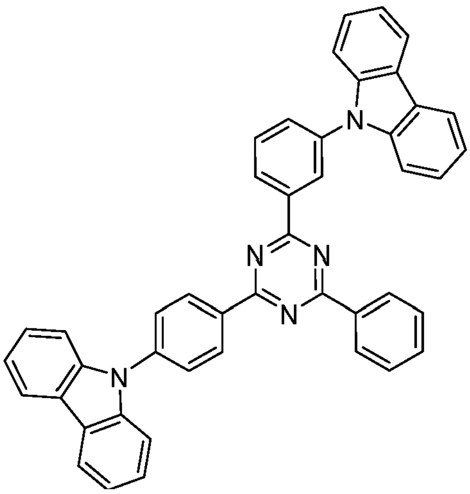

G30,

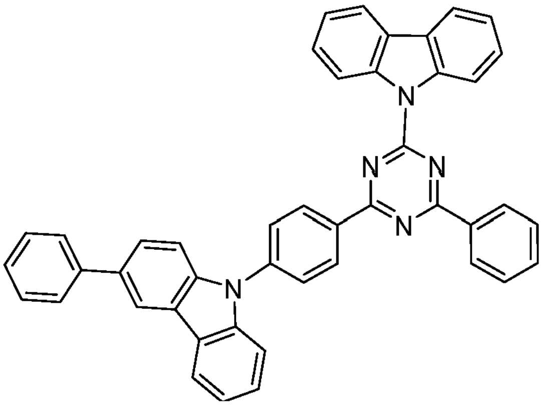

G31.

According to one embodiment, the composition comprises b) at least one organic metal complex, wherein the metal of the organic metal complex is selected from alkali or alkaline earth metal, more preferably the metal is lithium, magnesium or calcium.

According to one embodiment, the composition comprises one organic metal complex.

According to one embodiment, the composition consists of a compound of formula (1) and a organic metal complex, wherein the metal of the organic metal complex is selected from alkali, alkaline earth or rare earth metal, more preferably the metal is an alkali or alkaline earth metal.

According to one embodiment, the composition comprises b) at least one organic metal complex, wherein the organic metal complex comprises at least one ligand, wherein the ligand is selected from a quinolate or borate group.

Quinolates that can be suitable used are disclosed in WO 2013079217 A1.

Borate groups that can be suitable used are disclosed in WO 2013079676 A1.

According to one embodiment, the composition comprises at least one organic metal complex, wherein the organic metal complex the following Formula 5

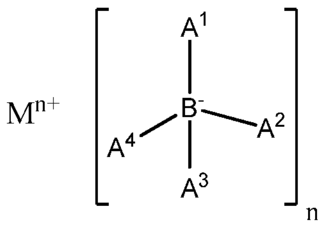

Formula (5)

wherein M is a metal ion, each of $A^1$- $A^4$ is independently selected from H, substituted or unsubstituted $C_6$ to $C_{20}$ aryl and substituted or unsubstituted $C_2$ to $C_{20}$ heteroaryl and n is valency of the metal ion.

According to one embodiment of formula (5), wherein n is 1 or 2.

According to one embodiment of formula (5), wherein M is an alkali metal, an alkaline earth metal or a rare earth metal, alternatively an alkali metal or alkaline earth metal, alternatively selected from lithium, magnesium or calcium.

According to one embodiment of formula (5), wherein at least three groups selected from $A^1$ to $A^4$ are nitrogen containing heteroaryl.

According to one embodiment, wherein the heteroaryl of Formula (5) contains a nitrogen and the nitrogen containing heteroaryl is bound to the central boron atom via a N-N bond, preferably the heteroaryl in Formula (5) is pyrazolyl.

The compound of formula (1) and/or the organic metal complex may be essentially non-emissive.

According to another aspect an organic semiconductor layer may comprises at least one composition of the present invention.

The organic semiconductor layer comprising the composition of the present invention may be essentially non-emissive.

The thickness of the organic semiconductor layer may be from about 0.5 nm to about 100 nm, for example about 2 nm to about 40 nm. When the thickness of the organic semiconductor layer is within these ranges, the organic semiconductor layer may have improved charge transport ability without a substantial increase in operating voltage.

The organic semiconductor layer comprising the composition of the present invention may have strong electron transport characteristics to increase charge mobility and/or stability.

According to another aspect an electronic device may comprises at least one organic semiconductor layer of the present invention.

According to another aspect an electronic device may comprises at least one anode and at least one cathode, preferably the organic semiconductor layer is arranged between the anode and the cathode.

The organic semiconductor layer comprising a composition of the present invention may have strong electron transport characteristics to increase charge mobility and/or stability and thereby to improve luminance efficiency, voltage characteristics, and/or lifetime characteristics of an electronic device.

The electronic device of the present invention may further comprise a photoactive layer, wherein the organic semiconductor layer of the present invention is arranged between the photoactive layer and the cathode layer, preferably between an emission layer or light-absorbing layer and the cathode layer, preferably the organic semiconductor layer is an electron transport layer.

An organic electronic device according to one embodiment comprises the organic semiconductor layer of the present invention, at least one anode layer, at least one cathode layer and at least one emission layer, wherein the organic semiconductor layer is preferably arranged between the emission layer and the cathode layer.

An organic electronic device according to one embodiment can be a light emitting device, thin film transistor, a battery, a display device or a photovoltaic cell, and preferably a light emitting device. A light emitting device can be an OLED.

According to one embodiment the OLED may have the following layer structure, wherein the layers having the following order:
an anode layer, a hole injection layer, optional a first hole transport layer, optional a second hole transport layer, an emission layer, an electron transport layer comprising the composition according to the invention, an electron injection layer, and a cathode layer.

A method of manufacturing an organic electronic device uses:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating. for example, a method uses
- a first deposition source to release the compound of formula 1 according to the invention, and
- a second deposition source to release the at least one organic metal complex; the method comprising the steps of forming the electron transport layer stack; whereby for an organic light-emitting diode (OLED):

  - the first electron transport layer is formed by releasing the compound of formula 1 from the first deposition source and the organic metal complex from the second deposition source.

The method may further include forming on the anode electrode an emission layer and at least one layer selected from the group consisting of forming a hole injection layer, forming a hole transport layer, or forming a hole blocking layer, between the anode electrode and the organic semiconductor layer.

The method may further include the steps for forming an organic light-emitting diode (OLED), wherein

- on a substrate a first anode electrode is formed,
- on the first anode electrode an emission layer is formed,
- on the emission layer an electron transport layer stack is formed, preferably a first electron transport layer is formed on the emission layer and a second electron transport layer is formed on the first electron transport layer and the second electron transport layer comprises the composition according to the invention,
- and finally a cathode electrode is formed,
- optional a hole injection layer, a hole transport layer, and a hole blocking layer, formed in that order between the first anode electrode and the emission layer,
- optional an electron injection layer is formed between the electron transport layer stack and the cathode electrode.

The method may further include forming an electron injection layer on a first electron transport layer. However, according to various embodiments of the OLED of the present invention, the OLED may not comprise an electron injection layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

The term "organic metal complex" means a compound which comprises one or more metal and one or more organic groups. The metal may be bound to the organic group via a covalent or ionic bond. The organic group means a group comprising mainly covalently bound carbon and hydrogen atoms. The organic group may further comprise heteroatoms selected from N, O, S, B, Si, P, Se, preferably from B, N, O and S.

In the context of the present specification the term "essentially non-emissive" or "non-emitting" means that the visible emission spectrum from the composition or a layer of a) the compound of formula 1 and b) at least one organic metal complex, wherein the metal of the organic metal complex is selected from the group comprising alkali, alkaline earth or rare earth metal in a device is less than 10 %, preferably less than 5 %, further preferred less than 1 %, relative to the visible emission spectrum. The visible emission spectrum is an emission spectrum with a wavelength of about $\geq$ 380 nm to about $\leq$ 780 nm. Preferably, an organic semiconductor layer or a device comprising a layer, which comprises a) the compound of formula 1 and b) at least one organic metal complex, wherein the metal of the organic metal complex is selected from the group comprising alkali, alkaline earth or rare earth metal, is essentially non-emissive or non-emitting.

The term "free of', "does not contain", "does not comprise" does not exclude impurities which may be present in the compounds prior to deposition. Impurities have no technical effect with respect to the object achieved by the present invention.

The operating voltage, also named U, is measured in Volt (V) at 10 milliAmpere per square centimeter (mA/cm2).

The candela per Ampere efficiency, also named cd/A efficiency, is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The rate onset temperature is measured in °C and describes the VTE source temperature at which measurable evaporation of a compound commences at a pressure of less than $10^{-5}$ mbar.

The term "OLED", "organic light emitting diode", "organic light emitting device", "organic optoelectronic device"

and "organic light-emitting diode" are simultaneously used and have the same meaning.

The term "transition metal" means and comprises any element in the d-block of the periodic table, which comprises groups 3 to 12 elements on the periodic table.

The term "group III to VI metal" means and comprises any metal in groups III to VI of the periodic table.

As used herein, "weight percent", "wt.-%", "percent by weight", "% by weight", and variations thereof refer to a composition, component, substance or agent as the weight of that composition, component, substance or agent of the respective electron transport layer divided by the total weight of the composition thereof and multiplied by 100. It is understood that the total weight percent amount of all components, substances or agents of the respective electron transport layer are selected such that it does not exceed 100 wt.-%.

As used herein, "volume percent", "vol.-%", "percent by volume", "% by volume", and variations thereof refer to an elemental metal, a composition, component, substance or agent as the volume of that elemental metal, component, substance or agent of the respective electron transport layer divided by the total volume of the respective electron transport layer thereof and multiplied by 100. It is understood that the total volume percent amount of all elemental metal, components, substances or agents of the respective cathode electrode layer are selected such that it does not exceed 100 vol.-%.

All numeric values are herein assumed to be modified by the term "about", whether or not explicitly indicated. As used herein, the term "about" refers to variation in the numerical quantity that can occur.

Whether or not modified by the term "about", the claims include equivalents to the quantities.

It should be noted that, as used in this specification and the appended claims, the singular forms "a", "an", and "the" include plural referents unless the content clearly dictates otherwise.

It should be noted that, as used in this specification and the appended claims, "*" if not otherwise defined indicates the chemical bonding position.

The anode electrode and cathode electrode may be described as anode electrode / cathode electrode or anode electrode / cathode electrode or anode electrode layer / cathode electrode layer.

In the present specification, when a definition is not otherwise provided, an "alkyl group" may refer to an aliphatic hydrocarbon group. The alkyl group may refer to "a saturated alkyl group" without any double bond or triple bond. The alkyl group may be a linear, cyclic or branched alkyl group.

The alkyl group may be a $C_1$ to $C_{16}$ alkyl group, or preferably a $C_1$ to $C_{12}$ alkyl group. More specifically, the alkyl group may be a $C_1$ to $C_{14}$ alkyl group, or preferably a $C_1$ to $C_{10}$ alkyl group or a $C_1$ to $C_6$ alkyl group. For example, a $C_1$ to $C_4$ alkyl group comprises 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a tert-butyl group, a pentyl group, a hexyl group, a cyclopropyl group, a cyclobutyl group, a cyclopentyl group, a cyclohexyl group, and the like.

In the present specification, "aryl" and "arylene group" may refer to a group comprising at least one hydrocarbon aromatic moiety, and all the elements of the hydrocarbon aromatic moiety may have p-orbitals which form conjugation, for example a phenyl group, a naphthyl group, an anthracenyl group, a phenanthrenyl group, a pyrenyl group, a fluorenyl group and the like.

The term "heteroaryl" and "heteroarylene" may refer to aromatic heterocycles with at least one heteroatom, and all the elements of the aromatic heterocycle may have p-orbitals which form conjugation, for example a pyridyl, pyrimidyl, pyrazinyl, triazinyl, pyrrolyl, carbazolyl, furanyl, benzofuranyl, dibenzofuranyl, thiophenyl, benzothiophenyl, dibenzothiophenyl group and the like. Preferably, the aromatic heterocycles are free of $sp^3$-hybridised carbon atoms.

The term "substituted or unsubstituted heteroaryl", "substituted or unsubstituted $C_5$ to $C_{24}$ heteroaryl", "substituted or unsubstituted $C_3$ to $C_{24}$ heteroaryl", "substituted or unsubstituted $C_5$ to $C_{18}$ heteroaryl", "substituted or unsubstituted $C_5$ to $C_{17}$ heteroaryl", "substituted or unsubstituted $C_3$ to $C_{17}$ heteroaryl", "substituted or unsubstituted $C_3$ to $C_{12}$ heteroarylene" and the like means that the substituted or unsubstituted heteroaryl comprises at least one heteroaryl ring; or at least one heteroaryl ring and at least one non-heteroaryl ring; or at least two heteroaryl rings and at least one non-heteroaryl ring; or at least three heteroaryl rings and at least one non-heteroaryl ring; or at least one heteroaryl ring and at least two non-heteroaryl rings. The rings of the substituted or unsubstituted heteroaryl can be a fused.

The term "hetero-fluorene ring" refers to a dibenzo[d,d]furanyl, dibenzo[b,d]thiophenyl or dibenzo[b,d]selenophenyl group.

The heteroatom may be selected from N, O, S, B, Si, P, Se, preferably from N, O and S.

A heteroarylene ring may comprise at least 1 to 3 heteroatoms. Preferably a heteroarylene ring may comprise at least 1 to 3 heteroatoms individually selected from N, S and/or O.

Further preferred in addition to the compounds of formula 1 at least one additional heteroaryl/ene ring may comprise at least 1 to 3 N-atoms, or at least 1 to 2-N atoms or at least one N-atom.

Further preferred in addition to the compounds of formula 1 at least one additional heteroaryl/ene ring may comprise at least 1 to 3 O-atoms, or at least 1 to 2 O-atoms or at least one O-atom.

Further preferred in addition to the compounds of formula 1 at least one additional heteroaryl/ene ring may comprise at least 1 to 3 S-atoms, or at least 1 to 2 S-atoms or at least one S-atom.

According to another preferred embodiment the compound according to formula 1 may comprise:

- at least 6 to 25 aromatic rings, preferably at least 7 to 22 aromatic rings, further preferred at least 8 to 20 aromatic rings, in addition preferred at least 9 to 15 aromatic rings and more preferred at least 10 to 14 aromatic rings; wherein
- at least 2 to 5, preferably 3 to 4 or 2 to 3 are heteroaromatic rings.

According to one embodiment the compound according to formula 1:

- comprises at least about 6 to about 20 aromatic rings, preferably at least about 7 to about 18 aromatic rings, further preferred at least about 9 to about 16 aromatic rings, in addition preferred at least about 10 to about 15 aromatic rings and more preferred at least about 11 to about 14 aromatic rings; and/or
- the compound of formula 1 comprises at least about 2 to about 6, preferably about 3 to about 5 or about 2 to about 4, hetero aromatic rings, wherein the hetero atoms can be selected from N, O, S.

According to one embodiment the compound according to formula 1 can be free of a fluorene ring and free of a hetero-fluorene ring.

According to one embodiment the compound according to formula 1 can be free of of a spiro-group.

According to a further preferred embodiment the compound of formula 1 comprises at least 2 to 7, preferably 2 to 5, or 2 to 3 hetero aromatic rings.

According to a further preferred embodiment the compound of formula 1 comprises at least 2 to 7, preferably 2 to 5, or 2 to 3 hetero aromatic rings, wherein at least one of the aromatic rings is a five member hetero aromatic ring.

According to a further preferred embodiment the compound of formula 1 comprises at least 3 to 7, preferably 3 to 6, or 3 to 5 hetero aromatic rings, wherein at least two of the hetero aromatic rings are five member hetero-aromatic-rings.

According to one embodiment the compound according to formula 1 may comprise at least 6 to 12 non-hetero aromatic rings and 2 to 3 hetero aromatic rings.

According to one preferred embodiment the compound according to formula 1 may comprise at least 7 to 12 non-hetero aromatic rings and 2 to 5 hetero aromatic rings.

According to one preferred embodiment the compound according to formula 1 may comprise at least 7 to 11 non-hetero aromatic rings and 2 to 3 hetero aromatic rings.

## Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

According to another embodiment the compound of formula 1 may have a melting point of about ≥ 250° C and about ≤ 380° C, preferably about ≥ 260° C and about ≤ 370° C, further preferred about ≥ 265° C and about ≤ 360° C.

## Glass transition temperature

The glass transition temperature is measured under nitrogen and using a heating rate of 10 K per min in a Mettler Toledo DSC 822e differential scanning calorimeter as described in DIN EN ISO 11357, published in March 2010.

According to another embodiment the compound of formula 1 may have a glass transition temperature Tg of about ≥ 105° C and about ≤ 380° C, preferably about ≥ 110° C and about ≤ 350° C.

## Rate onset temperature

The rate onset temperature is determined by loading 100 mg compound into a VTE source. As VTE source a point source for organic materials is used as supplied by Kurt J. Lesker Company (www.lesker.com) or CreaPhys GmbH (http://www.creaphys.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Ångstrom per second. To determine the rate onset temperature, the deposition rate is plotted against the VTE source temperature. The rate onset is the temperature at which noticeable deposition on

the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low takt time and decomposition of the organic compound in VTE source may occur due to prolonged exposure to elevated temperatures.

The rate onset temperature is an indirect measure of the volatility of a compound. The higher the rate onset temperature the lower is the volatility of a compound.

According to another embodiment the compound of formula 1 may have a rate onset temperature $T_{RO}$ of about ≥ 200° C and about ≤ 260° C, preferably about ≥ 220° C and about ≤ 260° C, further preferred about ≥ 220° C and about ≤ 260° C, in addition preferred about ≥ 230° C and about ≤ 255° C.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

According to one embodiment the compounds according to formula 1 may have a dipole moment (Debye) in the range from about ≥ 1.2 to about ≤ 4, preferably from about ≥ 1.3 to about ≤ 3.8, further preferred from about ≥ 1.4 to about ≤ 3.6.

Calculated HOMO and LUMO

The HOMO and LUMO are calculated with the program package TURBOMOLE V6.5. The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

According to one embodiment the compounds according to formula 1 may have a LUMO energy level (eV) in the range from about - 2.20 eV to about - 1.90 eV, preferably from about - 2.1 eV to about - 1.91 eV, further preferred from about - 2.08 eV to about - 1.92 eV, also preferred from about - 2.06 eV to about - 1.95 eV.

Technical effect

Surprisingly, it was found that the composition according to invention and the inventive organic electronic devices solve the problem underlying the present invention by being superior over the organic electroluminescent devices and compositions known in the art, in particular with respect to lifetime. At the same time the operating voltage is kept at a similar or even improved level which is important for reducing power consumption and increasing battery life, for example of a mobile display device. Long lifetime at high current density is important for the longevity of a device which is run at high brightness.

Additionally, it was surprisingly found that the calculated LUMO level of compounds of formula 1 is significantly more negative than the LUMO of the state of the art. A more negative LUMO may be beneficial for improved electron transfer from the cathode to the emission layer.

Furthermore, it was surprisingly found that the rate onset temperature of compounds of formula 1 is significantly lower than of the state of the art. A lower rate onset temperature may be beneficial for mass production as the deposition rate can be increased without increasing decomposition of the compound in the VTE source.

The inventors have surprisingly found that particular good performance can be achieved when using the organic electroluminescent device as a fluorescent blue device.

The specific arrangements mentioned herein as preferred were found to be particularly advantageous.

Likewise, some compounds falling within the scope of the broadest definition of the present invention have surprisingly be found to be particularly well performing with respect to the mentioned property of cd/A efficiency and/or lifetime. These compounds are discussed herein to be particularly preferred.

Further an organic optoelectronic device having high efficiency and/or long lifetime may be realized.

## Anode

A material for the anode may be a metal or a metal oxide, or an organic material, preferably a material with work function above about 4.8 eV, more preferably above about 5.1 eV, most preferably above about 5.3 eV. Preferred metals are noble metals like Pt, Au or Ag, preferred metal oxides are transparent metal oxides like ITO or IZO which may be advantageously used in bottom-emitting OLEDs having a reflective cathode.

In devices comprising a transparent metal oxide anode or a reflective metal anode, the anode may have a thickness from about 50 nm to about 100 nm, whereas semitransparent metal anodes may be as thin as from about 5 nm to about 15 nm, and non-transparent metal anodes may have a thickness from about 15 nm to about 150nm.

## Hole injection layer (HIL)

The hole injection layer may improve interface properties between the anode and an organic material used for the hole transport layer, and is applied on a non-planarized anode and thus may planarize the surface of the anode. For example, the hole injection layer may include a material having a median value of the energy level of its highest occupied molecular orbital (HOMO) between the work function of the anode material and the energy level of the HOMO of the hole transport layer, in order to adjust a difference between the work function of the anode and the energy level of the HOMO of the hole transport layer.

When the hole transport region comprises a hole injection layer 36, the hole injection layer may be formed on the anode by any of a variety of methods, for example, vacuum deposition, spin coating, casting, Langmuir-Blodgett (LB) method, or the like.

When hole injection layer is formed using vacuum deposition, vacuum deposition conditions may vary depending on the material that is used to form the hole injection layer, and the desired structure and thermal properties of the hole injection layer to be formed and for example, vacuum deposition may be performed at a temperature of about 100 °C to about 500 °C, a pressure of about $10^{-6}$ Pa to about $10^{-1}$ Pa, and a deposition rate of about 0.1 to about 10 nm/sec, but the deposition conditions are not limited thereto.

When the hole injection layer is formed using spin coating, the coating conditions may vary depending on the material that is used to form the hole injection layer, and the desired structure and thermal properties of the hole injection layer to be formed. For example, the coating rate may be in the range of about 2000 rpm to about 5000 rpm, and a temperature at which heat treatment is performed to remove a solvent after coating may be in a range of about 80 °C to about 200 °C, but the coating conditions are not limited thereto.

The hole injection layer may further comprise a p-dopant to improve conductivity and/or hole injection from the anode.

## p-dopant

In another aspect, the p-dopant may be homogeneously dispersed in the hole injection layer.

In another aspect, the p-dopant may be present in the hole injection layer in a higher concentration closer to the anode and in a lower concentration closer to the cathode.

The p-dopant may be one of a quinone derivative or a radialene compound but not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), 4,4',4"-((1E,1'E,1"E)-cyclopropane-1,2,3-triylidenetris(cyanomethanylylidene))-tris(2,3,5,6-tetrafluorobenzonitrile).

According to another embodiment, an organic electronic device comprising an organic semiconductor layer comprising a composition according to invention may additional comprise a layer comprising a radialene compound and/or a quinodimethane compound.

In another embodiment, the radialene compound and/or the quinodimethane compound may be substituted

with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have formula (XX) and/or the quinodimethane compound may have formula (XXIa) or (XXIb):

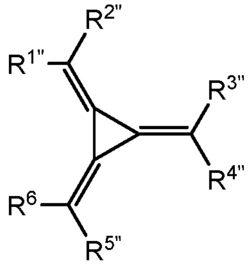
(XX)

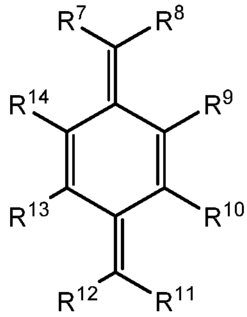
(XXIa)

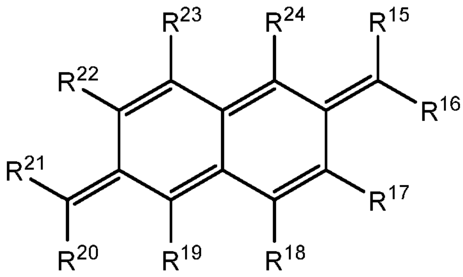
(XXIb),

wherein $R^{1"}$, $R^{2"}$, $R^{3"}$, $R^{4"}$, $R^{5"}$, $R^6$, $R^7$, $R^8$, $R^{11}$, $R^{12}$, $R^{15}$, $R^{16}$, $R^{20}$, $R^{21}$ are independently selected from an electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and electron withdrawing groups. Electron withdrawing group/s that can be suitable used are above mentioned.

Hole transport layer (HTL)

Conditions for forming the hole transport layer and the electron blocking layer may be defined based on the above-described formation conditions for the hole injection layer.

A thickness of the hole transport part of the charge transport region may be from about 10 nm to about 1000 nm, for example, about 10 nm to about 100 nm. When the hole transport part of the charge transport region comprises the hole injection layer and the hole transport layer, a thickness of the hole injection layer may be from about 10 nm to about 1000 nm, for example about 10 nm to about 100 nm and a thickness of the hole transport layer may be from about 5 nm to about 200 nm, for example about 10 nm to about 150 nm. When the thicknesses of the hole transport part of the charge transport region, the HIL, and the HTL are within these ranges, satisfactory hole transport characteristics may be obtained without a substantial increase in operating voltage.

Hole transport matrix materials used in the hole transport region are not particularly limited. Preferred are covalent compounds comprising a conjugated system of at least 6 delocalized electrons, preferably organic compounds comprising at least one aromatic ring, more preferably organic compounds comprising at least two aromatic rings, even more preferably organic compounds comprising at least three aromatic rings, most preferably organic compounds comprising at least four aromatic rings. Typical examples of hole transport matrix materials which are widely used in hole

transport layers are polycyclic aromatic hydrocarbons, triarylene amine compounds and heterocyclic aromatic compounds. Suitable ranges of frontier orbital energy levels of hole transport matrices useful in various layer of the hole transport region are well-known. In terms of the redox potential of the redox couple HTL matrix/ cation radical of the HTL matrix, the preferred values (if measured for example by cyclic voltammetry against ferrocene/ferrocenium redox couple as reference) may be in the range 0.0 - 1.0 V, more preferably in the range 0.2 - 0.7 V, even more preferably in the range 0.3 - 0.5 V.

Buffer layer

The hole transport part of the charge transport region may further include a buffer layer.
Buffer layer that can be suitable used are disclosed in US 6 140 763, US 6 614 176 and in US2016/248022.
The buffer layer may compensate for an optical resonance distance of light according to a wavelength of the light emitted from the EML, and thus may increase efficiency.

Emission layer (EML)

The emission layer may be formed on the hole transport region by using vacuum deposition, spin coating, casting, LB method, or the like. When the emission layer is formed using vacuum deposition or spin coating, the conditions for deposition and coating may be similar to those for the formation of the hole injection layer, though the conditions for the deposition and coating may vary depending on the material that is used to form the emission layer. The emission layer may include an emitter host (EML host) and an emitter dopant (further only emitter).
A thickness of the emission layer may be about 100Å to about 1000Å, for example about 200Å to about 600Å. When the thickness of the emission layer is within these ranges, the emission layer may have improved emission characteristics without a substantial increase in operating voltage.

Emitter host

According to another embodiment, the emission layer comprises compound of formula 1 as emitter host.
The emitter host compound has at least three aromatic rings, which are independently selected from carbocyclic rings and heterocyclic rings.
Other compounds that can be used as the emitter host is an anthracene matrix compound represented by formula 400 below:
Formula 400

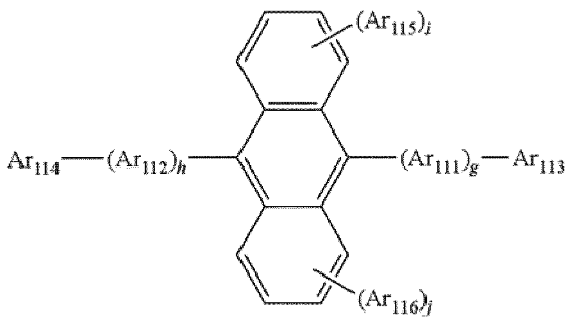

In formula 400, Arm and $Ar_{112}$ may be each independently a substituted or unsubstituted $C_6$-$C_{60}$ arylene group; $Ar_{113}$ to $Ar_{116}$ may be each independently a substituted or unsubstituted $C_1$-$C_{10}$ alkyl group or a substituted or unsubstituted $C_6$-$C_{60}$ arylene group; and g, h, i, and j may be each independently an integer from 0 to 4.
In some embodiments, Arm and $Ar_{112}$ in formula 400 may be each independently one of a phenylene group, a naphthalene group, a phenanthrenylene group, or a pyrenylene group; or a phenylene group, a naphthalene group, a phenanthrenylene group, a fluorenyl group, or a pyrenylene group, each substituted with at least one of a phenyl group, a naphthyl group, or an anthryl group.
In formula 400, g, h, i, and j may be each independently an integer of 0, 1, or 2.
In formula 400, $Ar_{113}$ to $Ar_{116}$ may be each independently one of

- a $C_1$-$C_{10}$ alkyl group substituted with at least one of a phenyl group, a naphthyl group, or an anthryl group;
- a phenyl group, a naphthyl group, an anthryl group, a pyrenyl group, a phenanthrenyl group, or a fluorenyl group;

- a phenyl group, a naphthyl group, an anthryl group, a pyrenyl group, a phenanthrenyl group, or a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof,
- a sulfonic acid group or a salt thereof, a phosphoric acid group or a salt thereof,
- a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a naphthyl group, an anthryl group, a pyrenyl group, a phenanthrenyl group, or
- a fluorenyl group

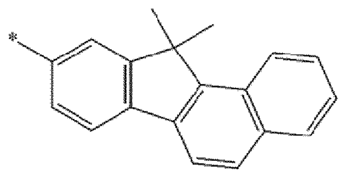

;

or

- formulas 7 or 8

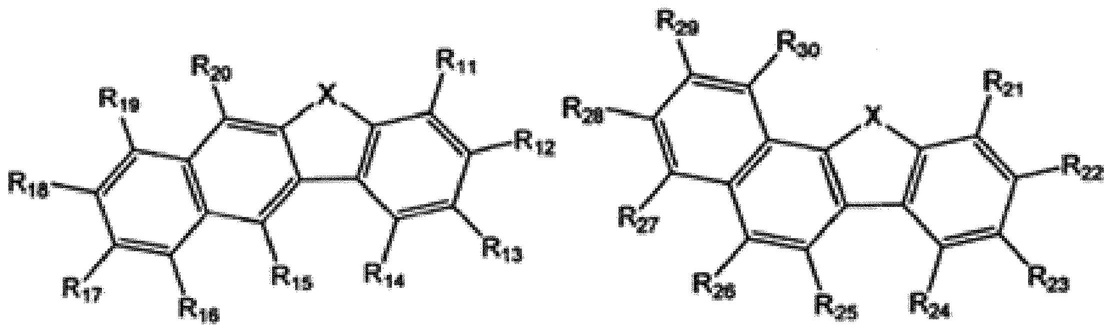

(7), (8).

Wherein in the formulas 7 and 8, X is selected form an oxygen atom and a sulfur atom, but embodiments of the invention are not limited thereto.

In the formula 7, any one of $R_{11}$ to $R_{14}$ is used for bonding to Arm. $R_{11}$ to $R_{14}$ that are not used for bonding to Arm and $R_{15}$ to $R_{20}$ are the same as $R_1$ to $R_8$.

In the formula 8, any one of $R_{21}$ to $R_{24}$ is used for bonding to $Ar_{111}$. $R_{21}$ to $R_{24}$ that are not used for bonding to Arm and $R_{25}$ to $R_{30}$ are the same as $R_1$ to $R_8$.

Preferably, the EML host comprises between one and three heteroatoms selected from the group consisting of N, O or S. More preferred the EML host comprises one heteroatom selected from S or O.

Emitter dopant

The dopant is mixed in a small amount to cause light emission, and may be generally a material such as a metal complex that emits light by multiple excitation into a triplet or more. The dopant may be, for example an inorganic, organic, or organic/inorganic compound, and one or more kinds thereof may be used.

The emitter may be a red, green, or blue emitter.

The dopant may be a fluorescent dopant, for example ter-fluorene, the structures are shown below. 4.4'-bis(4-diphenyl amiostyryl)biphenyl (DPAVBI, 2,5,8,11-tetra-tert-butyl perylene (TBPe), and Compound 8 below are examples of fluorescent blue dopants.

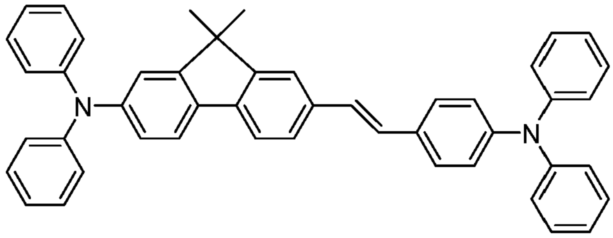

**Compound 8**

The dopant may be a phosphorescent dopant, and examples of the phosphorescent dopant may be an organic metal compound comprising Ir, Pt, Os, Ti, Zr, Hf, Eu, Tb, Tm, Fe, Co, Ni, Ru, Rh, Pd, or a combination thereof. The phosphorescent dopant may be, for example a compound represented by formula Z, but is not limited thereto:

$J_2MX$ (Z).

In formula Z, M is a metal, and J and X are the same or different, and are a ligand to form a complex compound with M.
The M may be, for example Ir, Pt, Os, Ti, Zr, Hf, Eu, Tb, Tm, Fe, Co, Ni, Ru, Rh, Pd or a combination thereof, and the J and X may be, for example a bidendate ligand.
One or more emission layers may be arranged between the anode and the cathode. To increase overall performance, two or more emission layers may be present.

Charge generation layer

A charge generation layer (also named CGL) may be arranged between the first and the second emission layer, and second and third emission layer, if present. Typically, the CGL comprises a n-type charge generation layer (also named n-CGL or electron generation layer) and a p-type charge generation layer (also named p-CGL or hole generation layer). An interlayer may be arranged between the n-type CGL and the p-type CGL.
In one aspect, the n-type CGL may comprise a matrix compound and a metal, metal salt or organic metal complex, preferably a metal. The metal may be selected from an alkali, alkaline earth or rare earth metal. The organic semiconductor layer comprising the composition according to the invention may be arranged between the first emission layer and the n-CGL and/or between the second and/or third emission layer and the cathode.
The p-type CGL may comprise a dipyrazino[2,3-f:2',3'-h]quinoxaline, a quinone compound or a radialene compound, preferably dipyrazino[2,3-f:2',3'-h]quinoxaline-2,3,6,7,10,11-hexacarbonitrile or a compound or formula (XX) and/or a compound of formula (XXIa) or (XXIb).

Electron transport layer (ETL)

According to another embodiment, the organic semiconductor layer that comprises the composition is an electron transport layer. In another embodiment the electron transport layer may consist ofthe composition according to the invention.
In another embodiment, the organic electronic device comprises an electron transport region of a stack of organic layers formed by two or more electron transport layers, wherein at least one electron transport layer comprises the composition.
The electron transport layer may include one or two or more different compounds of formula (1) and/or organic metal complexes.
The thickness of the electron transport layer may be from about 0.5 nm to about 100 nm, for example about 2 nm to about 40 nm. When the thickness of the electron transport layer is within these ranges, the electron transport layer may have improved electron transport ability without a substantial increase in operating voltage.

Electron injection layer (EIL)

According to another aspect of the invention, the organic electroluminescent device may further comprise an electron injection layer between the electron transport layer (first-ETL) and the cathode.
The electron injection layer (EIL) may facilitate injection of electrons from the cathode.
According to another aspect of the invention, the electron injection layer comprises:

(i) an electropositive metal selected from alkali metals, alkaline earth metals and rare earth metals in substantially elemental form, preferably selected from Li, Na, K, Rb, Cs, Mg, Ca, Sr, Ba, Eu and Yb, more preferably from Li, Na, Mg, Ca, Sr and Yb, even more preferably from Li and Yb, most preferably Yb; and/or

(ii) an alkali metal complex and/or alkali metal salt, preferably the Li complex and/or salt, more preferably a Li quinolinolate, even more preferably a lithium 8-hydroxyquino-linolate, most preferably the alkali metal salt and/or complex of the second electron transport layer (second-ETL) is identical with the alkali metal salt and/or complex of the injection layer.

The electron injection layer may include at least one selected from LiF, NaCl, CsF, $Li_2O$, and BaO.

A thickness of the EIL may be from about 0.1 nm to about 10 nm, or about 0.3 nm to about 9 nm. When the thickness of the electron injection layer is within these ranges, the electron injection layer may have satisfactory electron injection ability without a substantial increase in operating voltage.

The electron injection layer may comprise or consist of the composition according to the invention .

Cathode

A material for the cathode may be a metal, an alloy, or an electrically conductive compound that have a low work function, or a combination thereof. Specific examples of the material for the cathode may be lithium (Li), magnesium (Mg), aluminum (Al), aluminum-lithium (Al-Li), calcium (Ca), magnesium-indium (Mg-In), magnesium-silver (Mg-Ag), silver (Ag) etc. In order to manufacture a top-emission light-emitting device having a reflective anode deposited on a substrate, the cathode may be formed as a light-transmissive electrode from, for example, indium tin oxide (ITO), indium zinc oxide (IZO) or silver (Ag).

In devices comprising a transparent metal oxide cathode or a reflective metal cathode, the cathode may have a thickness from about 50 nm to about 100 nm, whereas semitransparent metal cathodes may be as thin as from about 5 nm to about 15 nm.

Substrate

A substrate may be further disposed under the anode or on the cathode. The substrate may be a substrate that is used in a general organic light emitting diode and may be a glass substrate or a transparent plastic substrate with strong mechanical strength, thermal stability, transparency, surface smoothness, ease of handling, and water resistance.

Hereinafter, the embodiments are illustrated in more detail with reference to examples.

**Description of the Drawings**

These and/or other aspects and advantages of the present invention will become apparent and more readily appreciated from the following description of the exemplary embodiments, taken in conjunction with the accompanying drawings, of which:

FIG. 1 is a schematic sectional view of an organic light-emitting diode (OLED), according to an exemplary embodiment of the present invention with an emission layer, one electron transport layer and an electron injection layer;

FIG. 2 is a schematic sectional view of an organic light-emitting diode (OLED), according to an exemplary embodiment of the present invention with an emission layer and two electron transport layers;

FIG. 3 is a schematic sectional view of an OLED, according to an exemplary embodiment of the present invention with an emission layer and three electron transport layers;

FIG. 4 is a schematic sectional view of an organic light-emitting diode (OLED), according to an exemplary embodiment of the present invention with an emission layer and one electron transport layer;

FIG. 5 is a schematic sectional view of an organic light-emitting diode (OLED), according to an exemplary embodiment of the present invention with an emission layer and two electron transport layers;

FIG. 6 is a schematic sectional view of an OLED, according to an exemplary embodiment of the present invention with an emission layer and three electron transport layers.

Reference will now be made in detail to the exemplary aspects, examples of which are illustrated in the accompanying drawings, wherein like reference numerals refer to the like elements throughout. The exemplary embodiments are described below, in order to explain the aspects, by referring to the figures.

Herein, when a first element is referred to as being formed or disposed "on" a second element, the first element can be disposed directly on the second element, or one or more other elements may be disposed there between. When a first element is referred to as being formed or disposed "directly on" a second element, no other elements are disposed

there between.

The term "contacting sandwiched" refers to an arrangement of three layers whereby the layer in the middle is in direct contact with the two adjacent layers.

The organic light emitting diodes according to an embodiment of the present invention may include a hole transport region; an emission layer; and a first electron transport layer comprising the composition according to the invention.

FIG. 1 is a schematic sectional view of an organic light-emitting diode 100, according to an exemplary embodiment of the present invention. The OLED 100 comprises an emission layer 150, an electron transport layer (ETL) 161 comprising the composition according to the invention, and an electron injection layer 180, whereby the first electron transport layer 161 is disposed directly on the emission layer 150 and the electron injection layer 180 is disposed directly on the first electron transport layer 161.

FIG. 2 is a schematic sectional view of an organic light-emitting diode 100, according to an exemplary embodiment of the present invention. The OLED 100 comprises an emission layer 150 and an electron transport layer stack (ETL) 160 comprising a first electron transport layer 161, and a second electron transport layer 162 comprising the composition according to the invention, whereby the second electron transport layer 162 is disposed directly on the first electron transport layer 161.

FIG. 3 is a schematic sectional view of an organic light-emitting diode 100, according to an exemplary embodiment of the present invention. The OLED 100 comprises an emission layer 150 and an electron transport layer stack (ETL) 160 comprising a first electron transport layer 161, a second electron transport layer 162, and a third electron transport layer 163, whereby the second electron transport layer 162 is disposed directly on the first electron transport layer 161 and the third electron transport layer 163 is disposed directly on the first electron transport layer 162. The first and/or the second and/or the third electron transport layer comprise the composition according to the invention.

FIG. 4 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, one first electron transport layer (ETL) 161, an electron injection layer (EIL) 180, and a cathode electrode 190. The first electron transport layer (ETL) 161 comprises the composition according to the invention. The electron transport layer (ETL) 161 is formed directly on the EML 150.

FIG. 5 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, an electron transport layer stack (ETL) 160, an electron injection layer (EIL) 180, and a cathode electrode 190. The electron transport layer (ETL) 160 comprises a first electron transport layer 161 and a second electron transport layer 162, wherein the first electron transport layer is arranged near to the anode (120) and the second electron transport layer is arranged near to the cathode (190). The first and/or the second electron transport layer comprise the composition according to the invention.

FIG. 6 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, an electron transport layer stack (ETL) 160, an electron injection layer (EIL) 180, and a second cathode electrode 190. The electron transport layer stack (ETL) 160 comprises a first electron transport layer 161, a second electron transport layer 162 and a third electron transport layer 163. The first electron transport layer 161 is formed directly on the emission layer (EML) 150. The first, second and/or third electron transport layer comprise the composition according to the invention.

Hereinafter, the embodiments are illustrated in more detail with reference to examples. Reference will now be made in detail to the exemplary aspects.

Preparation of compounds of formula 1

Compounds of formula 1 may be prepared as described in US 2015171340. LiQ is commercially available (CAS 25387-93-3). Metal borates may be synthesized as described in WO2013079676A1.

General procedure for fabrication of OLEDs

For top emission devices, Examples 1 to 4 and comparative example 1, a glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare a first electrode. 100 nm Ag were deposited on the glass substrate at a pressure of $10^{-5}$ to $10^{-7}$ mbar to form the anode.

Then, 92 vol.-% Biphenyl-4-yl(9,9-diphenyl-9H-fluoren-2-yl)-[4-(9-phenyl-9H-carbazol-3-yl)phenyl]-amine (CAS 1242056-42-3) with 8 vol.-% 2,2',2"-(cyclopropane-1,2,3-triylidene)tris(2-(p-cyanotetrafluorophenyl)acetonitrile)

was vacuum deposited on the anode, to form a HIL having a thickness of 10 nm.

Then, Biphenyl-4-yl(9,9-diphenyl-9H-fluoren-2-yl)-[4-(9-phenyl-9H-carbazol-3-yl) phenyl]-amine was vacuum deposited on the HIL, to form a HTL having a thickness of 118 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% H09 (Sun Fine Chemicals, South Korea) as EML host and 3 vol.-% BD200 (Sun Fine Chemicals, South Korea) as fluorescent blue dopant were deposited on the EBL, to form a blue-emitting EML with a thickness of 20 nm.

Then the hole blocking layer is formed with a thickness of 5 nm by depositing 2,4-diphenyl-6-(4',5',6'-triphenyl-[1,1':2',1":3",1‴:3‴,1⁗-quinquephenyl]-3⁗-yl)-1,3,5-triazine on the emission layer.

Then, the electron transporting layer is formed on the hole blocking layer according to Examples 1 to 4 and comparative example 1 with a the thickness of 31 nm. The electron transport layer comprises 50 wt.-% matrix compound and 50 wt.-% of alkali organic complex, see Table 1.

Then, the electron injection layer is formed on the electron transporting layer by deposing Yb with a thickness of 2 nm.

Ag is evaporated at a rate of 0.01 to 1 Å/s at $10^{-7}$ mbar to form a cathode with a thickness of 11 nm.

A cap layer of Biphenyl-4-yl(9,9-diphenyl-9H-fluoren-2-yl)-[4-(9-phenyl-9H-carbazol-3-yl)phenyl]-amine is formed on the cathode with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/$m^2$ using an Instrument Systems CAS-140CT array spectrometer (calibrated by Deutsche Akkreditierungsstelle (DAkkS)) for each of the voltage values. The cd/A efficiency at 10 mA/$cm^2$ is determined by interpolating the luminance-voltage and current-voltage characteristics, respectively.

Lifetime LT of the device is measured at ambient conditions (20°C) and 30 mA/$cm^2$, using a Keithley 2400 sourcemeter, and recorded in hours.

The brightness of the device is measured using a calibrated photo diode. The lifetime LT is defined as the time till the brightness of the device is reduced to 97 % of its initial value.

Top emission devices

In Table 1 is shown the performance of organic electronic devices comprising an organic semiconductor layer comprising compound of formula 1 and an alkali organic complex.

In comparative example 1, compound ETM-1 was used as matrix compound:

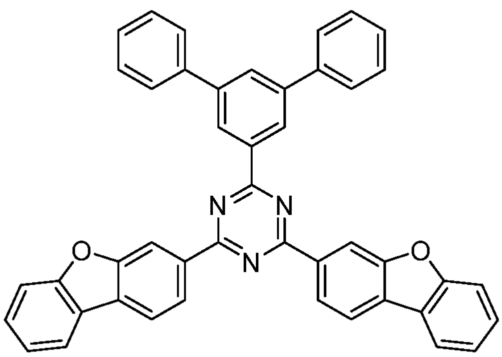

ETM-1.

Compound ETM-1 is free of carbazole groups. The organic semiconductor layer comprises 50 vol.-% ETM-1 and 50 vol.-% LiQ. The operating voltage is 3.55 V and the cd/A efficiency is 7.3 cd/A. The lifetime LT97 at 30 mA/cm2 is 62 hours.

In Example 1, the organic semiconductor layer comprises 50 vol.-% compound of formula 1 of MX1

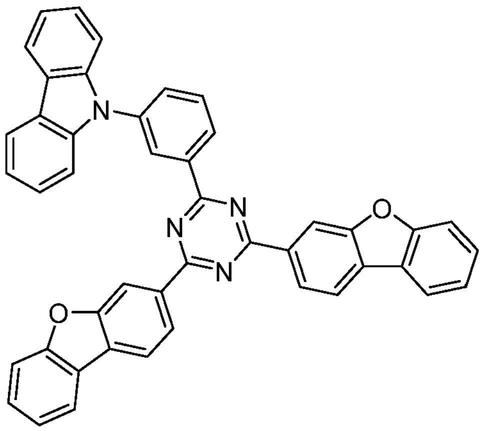

MX1

and 50 vol.-% LiQ. The operating voltage is 3.6 V and the cd/A efficiency is 7.0 cd/A. The lifetime is improved to 78 hours.

In Example 2, the organic semiconductor layer comprises 50 vol.-% compound of formula 1 of MX2

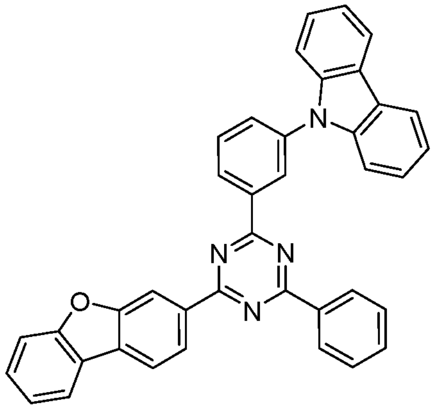

MX2

and 50 vol.-% LiQ. The operating voltage is 3.5 V and the cd/A efficiency is 7.0 cd/A. The lifetime is improved to 79 hours.

In Example 3, the organic semiconductor layer comprises 50 vol.-% compound of formula 1 of MX3

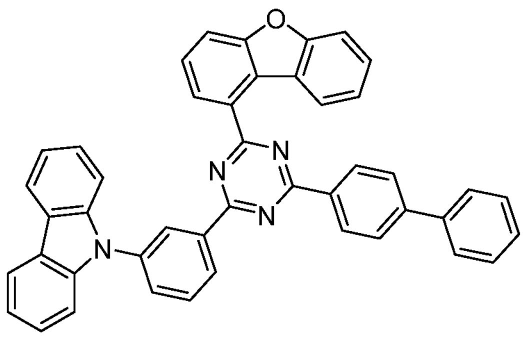

MX3

and 50 vol.-% LiQ. The operating voltage is 3.6 V and the cd/A efficiency is 7.2 cd/A. The lifetime is improved to 100 hours.

In Example 4, the organic semiconductor layer comprises 50 vol.-% compound of formula 1 of MX4

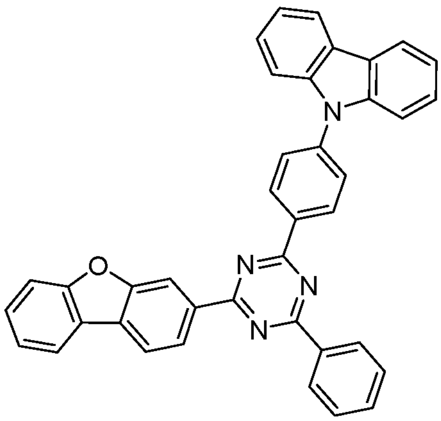

MX4

and 50 vol.-% LiQ. The operating voltage is 3.5 V and the cd/A efficiency is 7.1 cd/A. The lifetime is improved to 91 hours.

Table 1: Performance data of organic electroluminescent device comprising an organic semiconductor layer comprising compound of formula 1 and an alkali organic complex

| | Matrix compound | Concentration of matrix compound (vol.-%) | Alkali organic complex | Concentration of alkali organic complex (vol.-%) | Thickness electron transport layer (nm) | Operating voltage at 10 mA/cm$^2$ (V) | cd/A efficiency at 10 mA/cm$^2$ (cd/A) | LT97 at 30 mA/cm$^2$ (h) |
|---|---|---|---|---|---|---|---|---|
| Comparative example 1 | 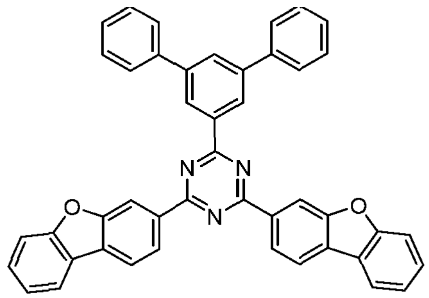 ETM-1 | 50 | LiQ | 50 | 31 | 3.55 | 7.3 | 62 |
| Example 1 | 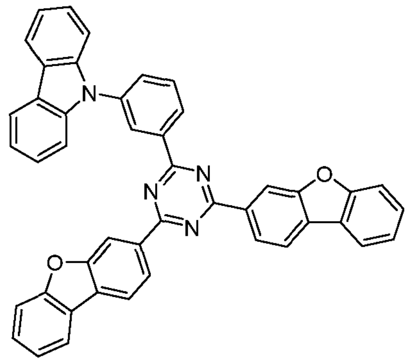 MX1 | 50 | LiQ | 50 | 31 | 3.6 | 7.0 | 78 |

(continued)

| | Matrix compound | Concentration of matrix compound (vol.-%) | Alkali organic complex | Concentration of alkali organic complex (vol.-%) | Thickness electron transport layer (nm) | Operating voltage at 10 mA/cm$^2$ (V) | cd/A efficiency at 10 mA/cm$^2$ (cd/A) | LT97 at 30 mA/cm$^2$ (h) |
|---|---|---|---|---|---|---|---|---|
| Example 2 | 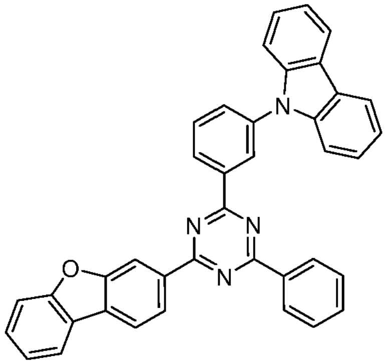 MX2 | 50 | LiQ | 50 | 31 | 3.5 | 7.0 | 79 |
| Example 3 | 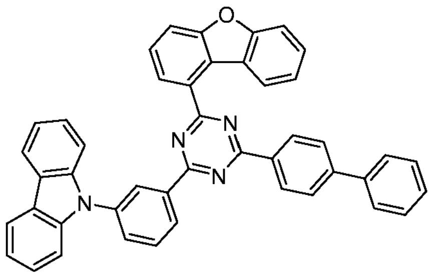 MX3 | 50 | LiQ | 50 | 31 | 3.6 | 7.2 | 100 |

(continued)

| | Matrix compound | Concentration of matrix compound (vol.-%) | Alkali organic complex | Concentration of alkali organic complex (vol.-%) | Thickness electron transport layer (nm) | Operating voltage at 10 mA/cm$^2$ (V) | cd/A efficiency at 10 mA/cm$^2$ (cd/A) | LT97 at 30 mA/cm$^2$ (h) |
|---|---|---|---|---|---|---|---|---|
| Example 4 | 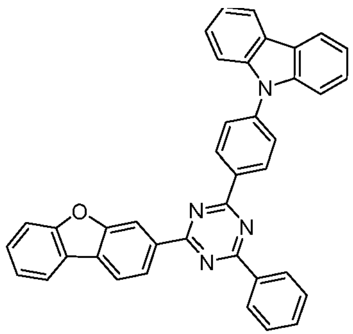 MX4 | 50 | LiQ | 50 | 31 | 3.5 | 7.1 | 91 |

In summary, improved lifetime may be achieved when the organic semiconductor layer comprises a compound of formula 1 and a organic metal complex.

## Claims

**1.** A composition comprising

a) a compound of formula 1:

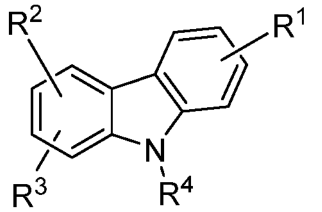

(1),

wherein

$R^1$ and $R^2$ are independently selected from H, D, CN, halogen, $C_1$ to $C_{12}$ alkyl, $C_1$ to $C_{12}$ alkoxy, substituted or unsubstituted $C_6$ to $C_{18}$ aryl, substituted or unsubstituted $C_3$ to $C_{24}$ heteroaryl, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to C12 alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to $C_{24}$ aryl, $C_3$ to $C_{24}$ heteroaryl;

$R^3$ or $R^4$ has the general formula 2:

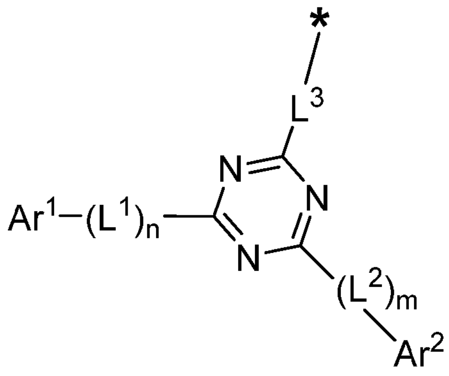

(2),

wherein the asterisk symbol "*" represents the binding position of the group $R^3$ or $R^4$;

$L^1$, $L^2$ and $L^3$ are independently selected from substituted or unsubstituted $C_6$ to $C_{13}$ arylene or substituted or unsubstituted $C_3$ to $C_{12}$ heteroarylene, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{18}$ aryl, perhalogenated $C_6$ to $C_{18}$ aryl, $C_3$ to $C_{24}$ heteroaryl, CN, halogen;

n is 1 or 0; m is 1 or 0;

$Ar^1$ is selected from substituted or unsubstituted $C_5$ to $C_{24}$ heteroaryl, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to C24 aryl, C3 to C24 heteroaryl; and wherein $Ar^1$ is free of a spiro-group;

$Ar^2$ has the general formula 3:

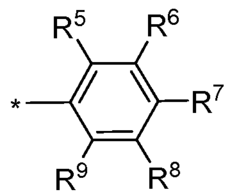

(3),

wherein the asterisk symbol "*" represents the binding position of the group $Ar^2$ to the moiety $L^2$;
$R^5$ to $R^9$ are independently H, D, CN, halogen, $C_1$ to $C_{12}$ alkyl, $C_1$ to $C_{12}$ alkoxy, substituted or unsubstituted $C_6$ to $C_{12}$ aryl, substituted or unsubstituted $C_3$ to $C_{12}$ heteroaryl,
wherein the substituted aryl ring or substituted heteroaryl ring is substituted with one or more $C_1$ to $C_{12}$ alkyl, $C_6$ to $C_{12}$ aryl or $C_3$ to $C_{12}$ heteroaryl groups, wherein the substituents are bonded by a single bond to the substituted aryl ring or substituted heteroaryl ring;
or

$Ar^2$ is selected from a fused ring system comprising one to four substituted or unsubstituted 6 membered aryl rings and zero to two substituted or unsubstituted 5 to 7 membered heteroaryl rings, wherein the substituted aryl ring is substituted with $C_1$ to $C_{12}$ alkyl groups and the substituted heteroaryl ring is substituted with one or more $C_1$ to $C_{12}$ alkyl, $C_6$ to $C_{12}$ aryl or $C_3$ to $C_{12}$ heteroaryl groups;

with the provision that:

i) $R^3$ is formula 2 and $R^4$ is selected from $C_1$ to $C_{12}$ alkyl, substituted or unsubstituted $C_6$ to $C_{18}$ aryl, substituted or unsubstituted $C_3$ to $C_{24}$ heteroaryl, wherein the substituents are selected from $C_1$ to $C_{12}$ alkyl, perhalogenated $C_1$ to $C_{12}$ alkyl, $C_1$ to $C_{12}$ alkoxy, perhalogenated $C_1$ to $C_{12}$ alkoxy, $C_6$ to $C_{24}$ aryl, perhalogenated $C_6$ to $C_{24}$ aryl, $C_3$ to $C_{24}$ heteroaryl, CN, halogen, P(=O)R'R", C(=O)R' or C(=O)OR', wherein R' and R" are independently selected from $C_1$ to $C_{16}$ alkyl, $C_6$ to C24 aryl, $C_3$ to $C_{24}$ heteroaryl;
or
ii) $R^4$ is formula 2 and $R^3$ is H or D; and

b) at least one organic metal complex, wherein the metal of the organic metal complex is selected from the group comprising alkali, alkaline earth or rare earth metal.

2. The composition according to claim 1, wherein the metal of the organic metal complex is selected from alkali or alkaline earth metal, more preferably the metal is lithium, magnesium or calcium.

3. The composition according to claim 1 or 2, wherein the organic metal complex comprises at least one ligand, wherein the ligand is selected from a quinolate or borate group.

4. The composition according to any of the preceding claims 1 to 3, wherein $R^1$ and $R^2$ are independently selected from H, D, unsubstituted $C_6$ to $C_{18}$ aryl, or unsubstituted $C_3$ to $C_{24}$ heteroaryl; preferably $R^1$ and $R^2$ are independently selected from H, D, or unsubstituted $C_6$ to $C_{18}$ aryl; further preferred $R^1$ and $R^2$ are independently selected from H, D, or unsubstituted $C_6$ aryl, and in addition preferred $R^1$ is selected from H or D and $R^2$ is a unsubstituted $C_6$ aryl, or $R^2$ is selected from H or D and $R^1$ is an unsubstituted $C_6$ aryl.

5. The composition according to any of the preceding claims 1 to 4, wherein $Ar^1$ is selected from substituted or unsubstituted $C_5$ to $C_{24}$ heteroaryl comprising at least one heteroatom selected from O, S, Se or N, preferably the at least one heteroatom is selected from O, S, Se and in addition preferred the at least one heteroatom is O.

6. The composition according to any of the preceding claims 1 to 5, wherein $Ar^1$ has the general formula 4:

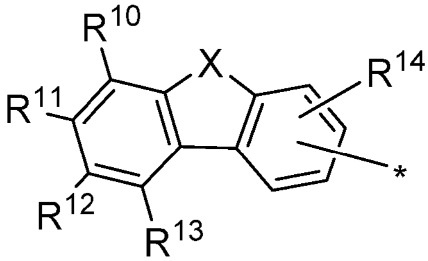

(4),

wherein the asterisk symbol "*" represents the binding position of the group $Ar^1$ to the moiety $L^1$;

X is selected from O, S, Se or $NR^{15}$, wherein $R_{15}$ is independently selected from $C_1$ to $C_{12}$ alkyl, perhalogenated $C_1$ to $C_{12}$ alkyl, $C_6$ to $C_{24}$ aryl, perhalogenated $C_6$ to C24 aryl, $C_3$ to $C_{24}$ heteroaryl;
$R^{10}$ to $R^{14}$ are independently selected from H, D, CN, halogen, $C_1$ to $C_{12}$ alkyl, $C_1$ to $C_{12}$ alkoxy, $C_6$ to $C_{18}$ aryl or $C_3$ to $C_{24}$ heteroaryl;

wherein preferably $R^{10}$ and $R^{11}$, $R^{11}$ and $R^{12}$ or $R^{12}$ and $R^{13}$ form a 5 to 7 membered substituted or unsubstituted aryl ring, or a 5 to 7 membered substituted or unsubstituted heteroaryl ring, and in addition preferred $R^{10}$ and $R^{11}$, $R^{11}$ and $R^{12}$ or $R^{12}$ and $R^{13}$ form two fused 5 to 7 membered substituted or unsubstituted aryl rings, or two fused 5 to 7 membered substituted or unsubstituted heteroaryl rings,
wherein the substituents are selected from $C_1$ to $C_{12}$ alkyl, $C_6$ to $C_{18}$ aryl or $C_3$ to $C_{24}$ heteroaryl groups.

**7.** The composition according to any of the preceding claims 1 to 6, wherein X of general formula 4 is selected from O, S or $NR^{15}$, more preferred X is selected from O or S, and also preferred X is O.

**8.** The composition according to any of the preceding claims 1 to 7, wherein $Ar^1$ is selected from the group comprising D1 to D16:

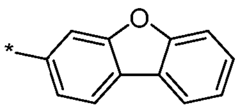

(D1),

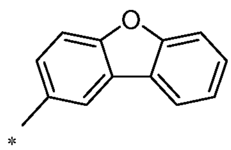

(D2),

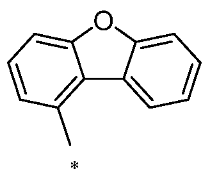

(D3),

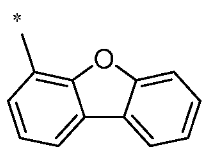

(D4),

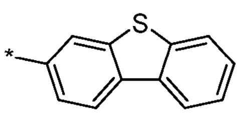

(D5),

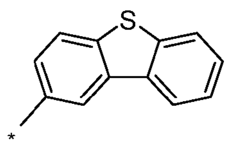

(D6),

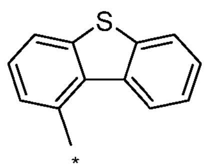

(D7),

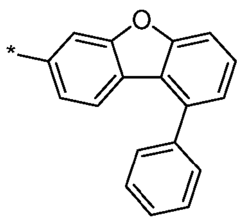

(D8),

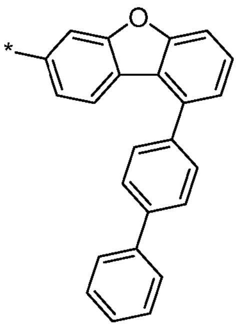 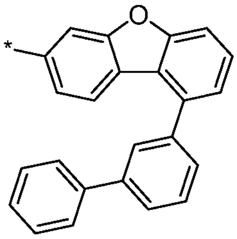 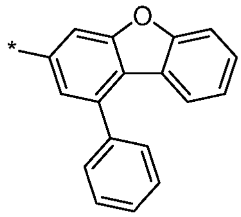 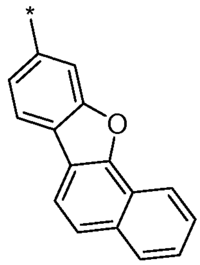

(D9), (D10), (D11), (D12),

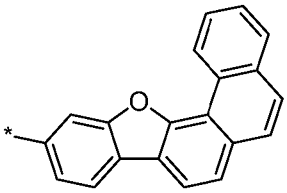 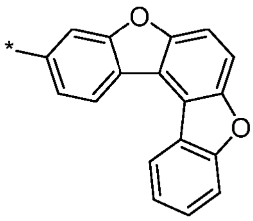 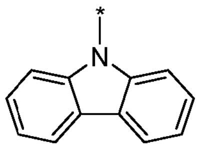 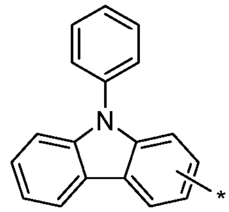

(D13), (D14), (D15), (D16),

preferably $Ar^1$ is selected from D1 to D14, also preferred $Ar^1$ is selected from D1, D3, D5, D7, D8 to D14, more preferably $Ar^1$ is selected from D1 or D3, most preferred $Ar^1$ is selected from D1.

**9.** The composition according to any of the preceding claims 1 to 8, wherein $Ar^2$ is selected from the group comprising E1 to E44:

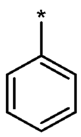 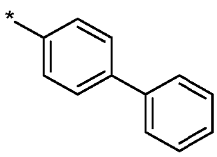 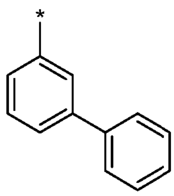 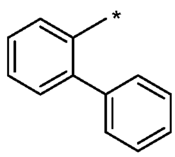 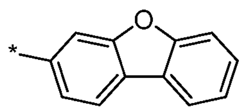 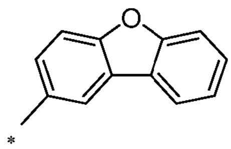

(E1), (E2), (E3, (E4), (E5), (E6),

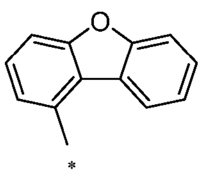 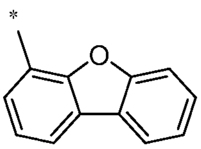 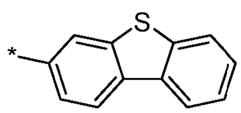 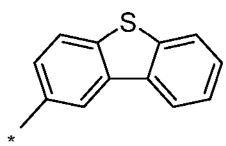 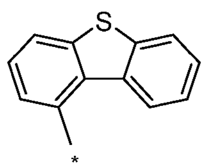

(E7), (E8), (E9), (E10), (E11),

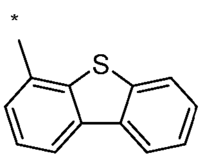 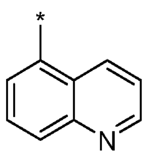 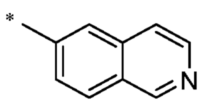 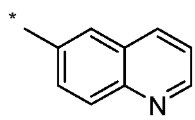 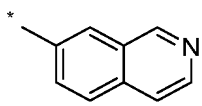

(E12), (E13), (E14), (E15), (E16),

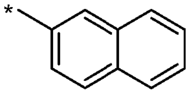 (E17), 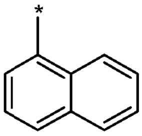 (E18), 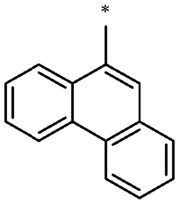 (E19), 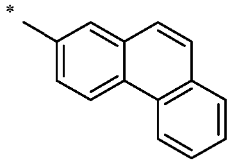 (E20), 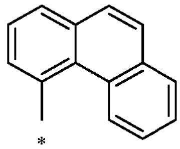 (E21),

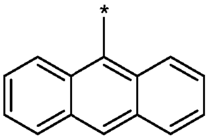 (E22), 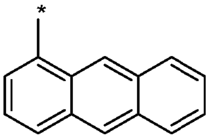 (E23), 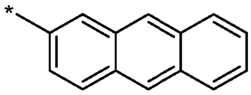 (E24), 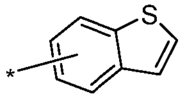 (E25), 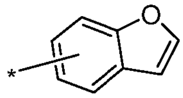 (E26),

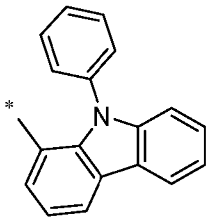 (E27), 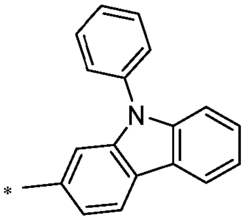 (E28), 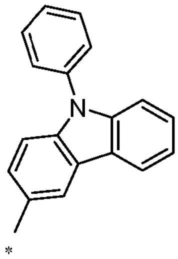 (E29), 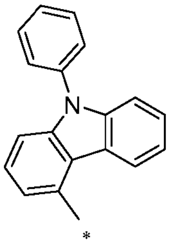 (E30), 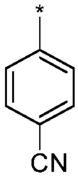 (E31), 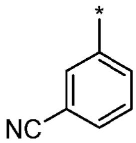 (E32),

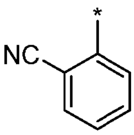 (E33), 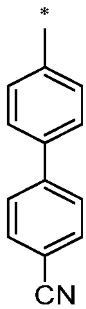 (E34), 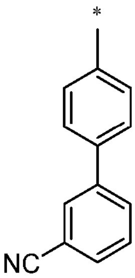 (E35), 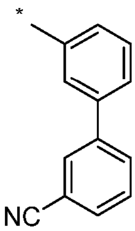 (E36), 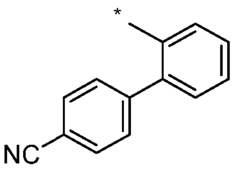 (E37), 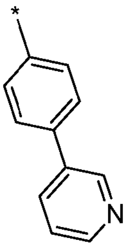 (E38), 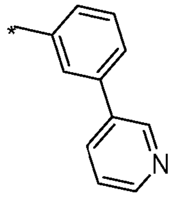 (E39),

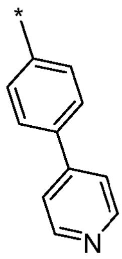 (E40), 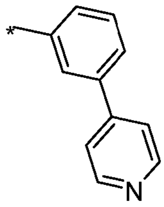 (E41), 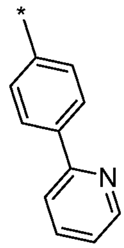 (E42), 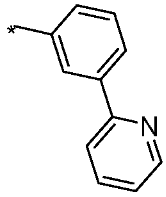 (E43), 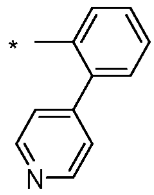 (E44);

preferably $Ar^2$ is selected from E1 to E26 and E31 to E44; more preferably $Ar^2$ is selected from E1to E12, E17 to E19 and E31 to E37; also preferred $Ar^2$ is selected from E1 to E12, E17 to E19 and E31 to E37; most preferred $Ar^2$ is selected from E1 to E4 and E17 to E18.

**10.** The composition according to any of the preceding claims 1 to 9, wherein

e) $L^1$, $L^2$ and/or $L^3$ are independently selected from the group comprising F1 to F11:

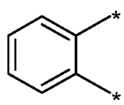 (F1), 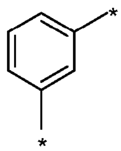 (F2), 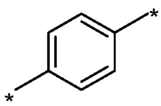 (F3), 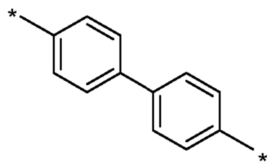 (F4),

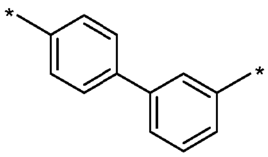 (F5), 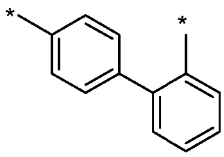 (F6), 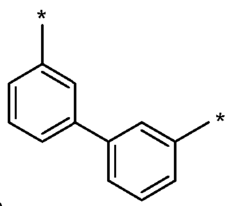 (F7), 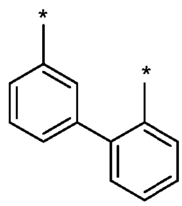 (F8),

,

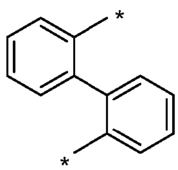 (F9), 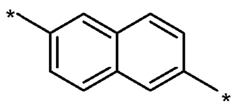 (F10), 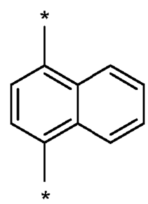 (F11);

preferably $L^1$, $L^2$ and/or $L^3$ are selected from F2, F3, F4, F5 or F7, more preferably $L^1$, $L^2$ and/or $L^3$ are selected from F2 , F3, F5 or F7, more preferred $L^1$, $L^2$ and/or $L^3$ is selected from F2 or F3; or
f) for $L^1$ n is 1 and for $L^2$m is 0, $L^1$ is selected from F1 to F11, preferably $L^1$ is selected from F2, F3, F4, F5 or F7, more preferably $L^1$ is selected from F2 , F3, F5 or F7, more preferred $L^1$ is selected from F2 or F3; or
g) for $L^1$ n is 0 and for $L^2$ m is 1, $L^2$ is selected from F1 to F11, preferably $L^2$ is selected from F2, F3, F4, F5 or F7, more preferably $L^2$ is selected from F2 , F3, F5 or F7, more preferred $L^2$ is selected from F2 or F3; or
h) for $L^1$ n is 0 and for $L^2$ m is 0 and $L^3$ is selected from F1 to F11, preferably $L^3$ is selected from F2, F3, F4, F5 or F7, more preferably $L^3$ is selected from F2 , F3, F5 or F7, more preferred $L^3$ is selected from F2 or F3.

**11.** The composition according to any of the preceding claims 1 to 10, wherein the compounds of formula 1 are selected

from the group comprising G1 to G31:

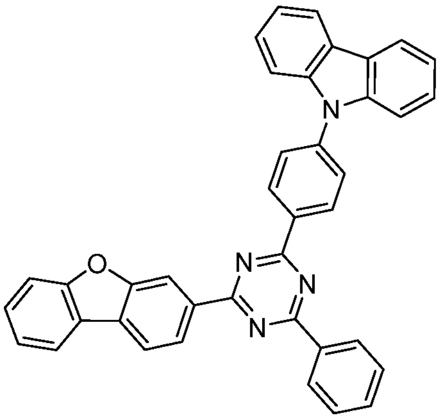

(G1),

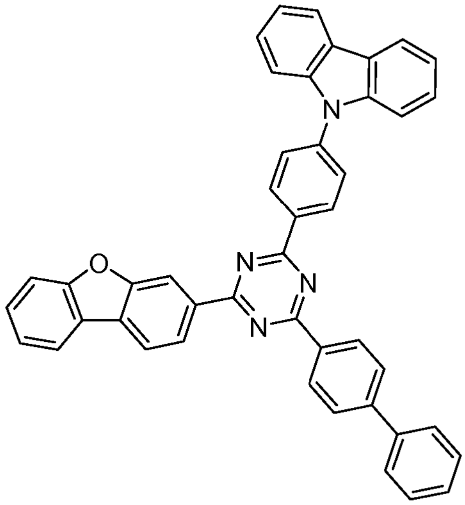

(G2),

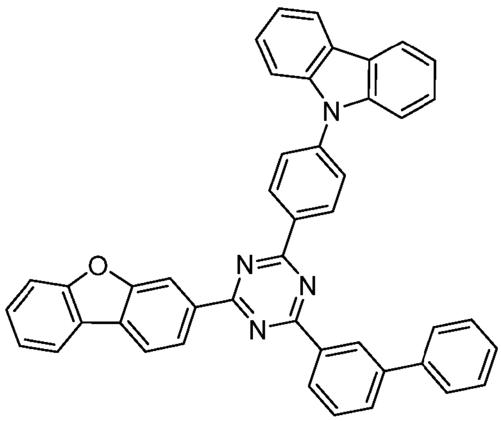

(G3),

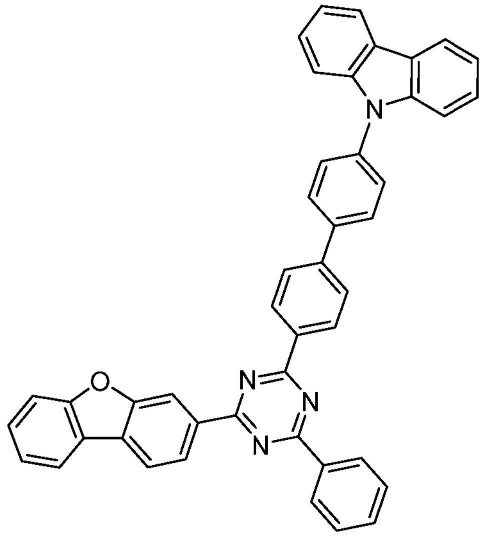

(G4),

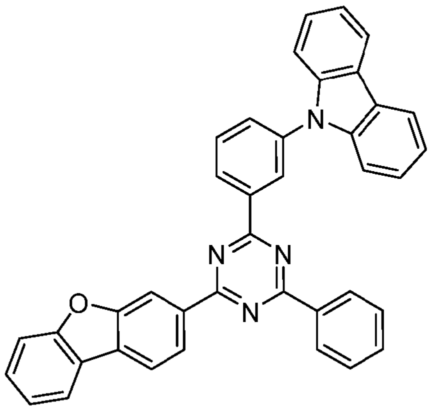

(G5),

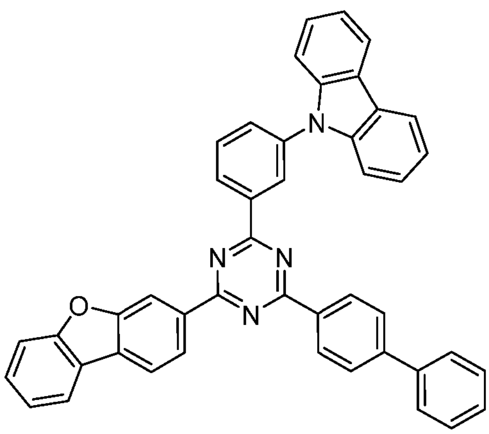

(G6),

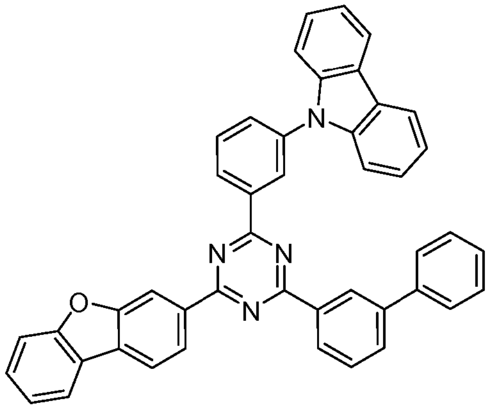

(G7),

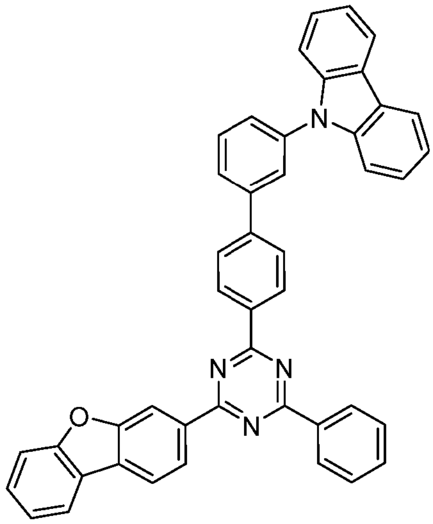

(G8),

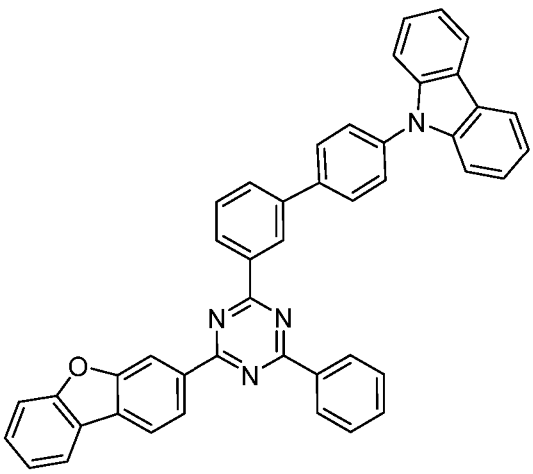

(G9),

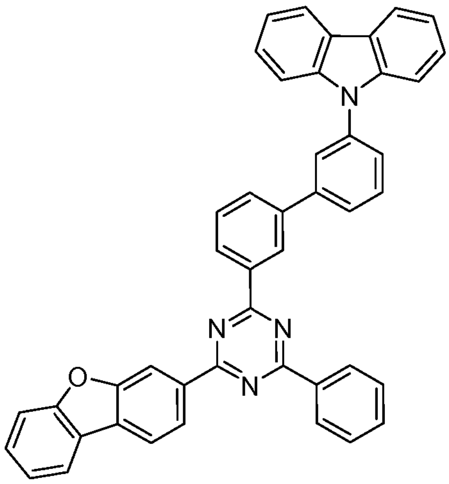

(G10),

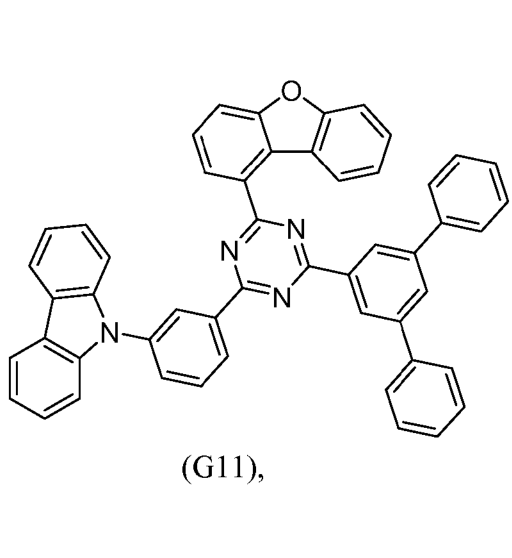

(G11),

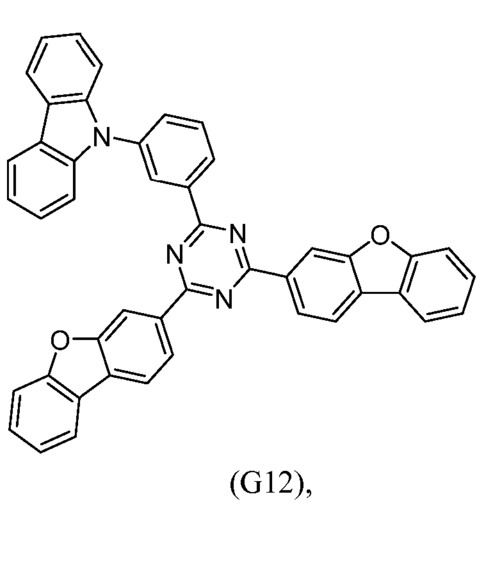

(G12),

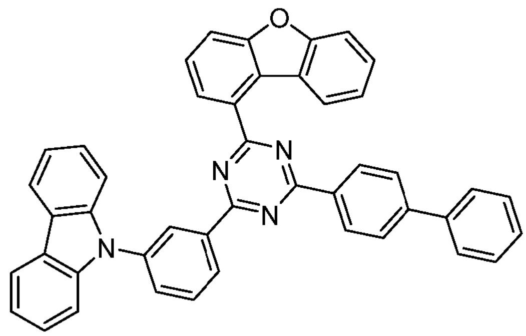

(G13),

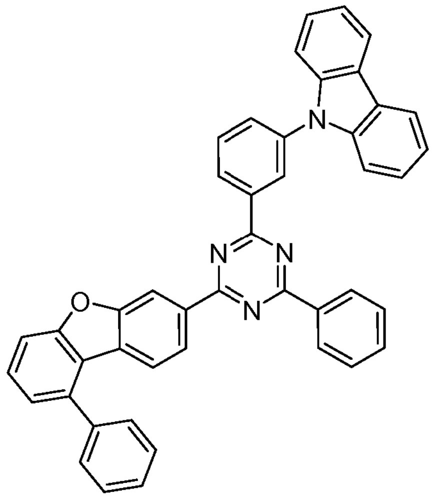

(G14),

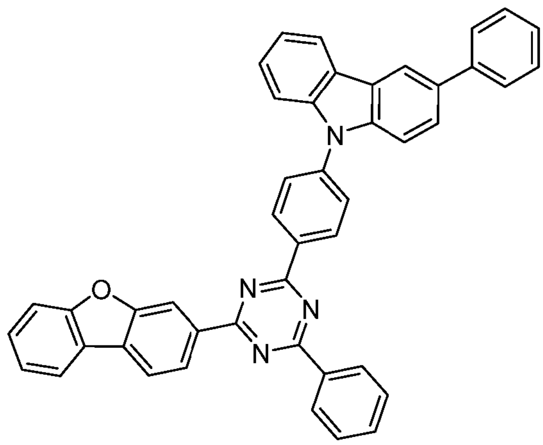

(G15),

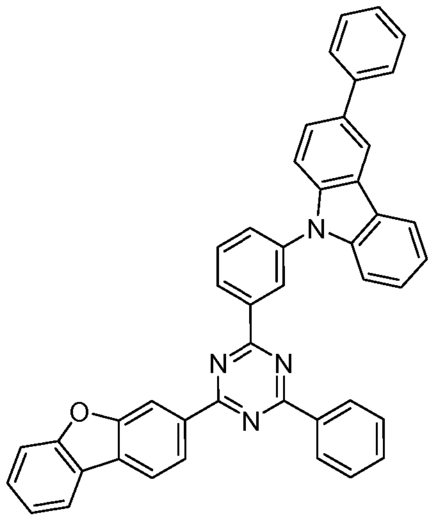

(G16),

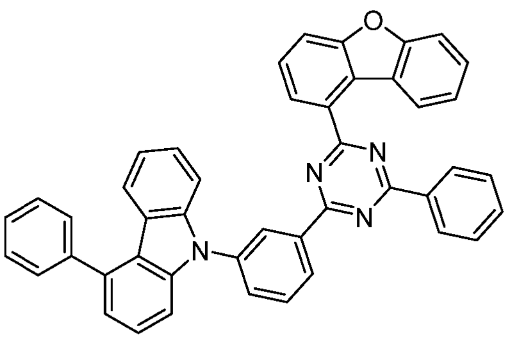

(G17),

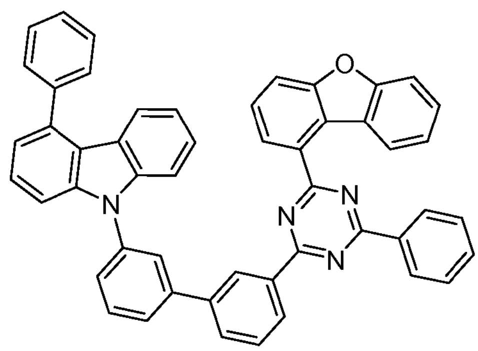

(G18),

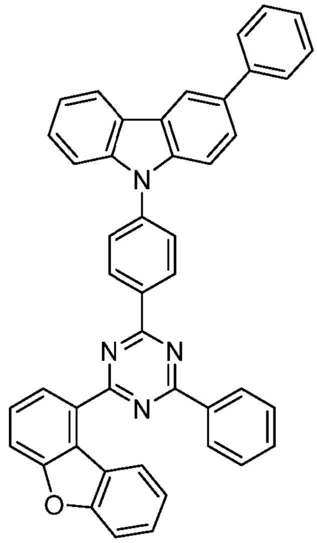

(G19),

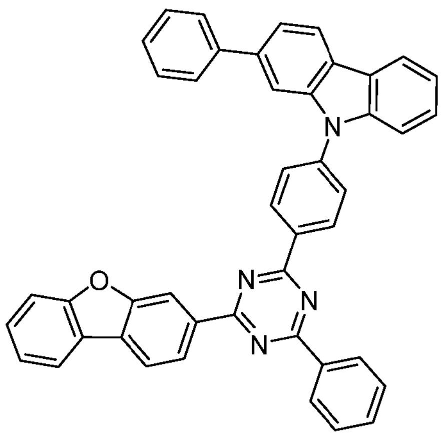

(G20),

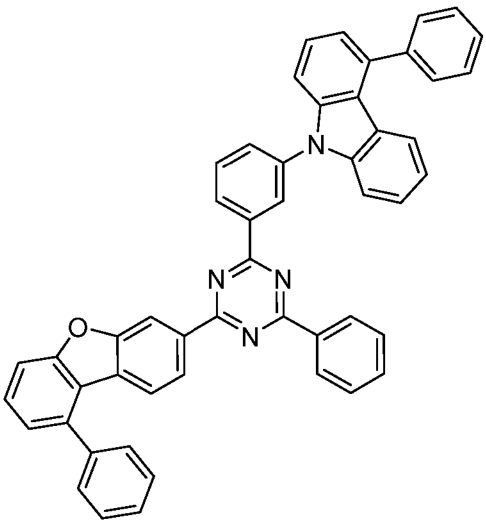

(G21),

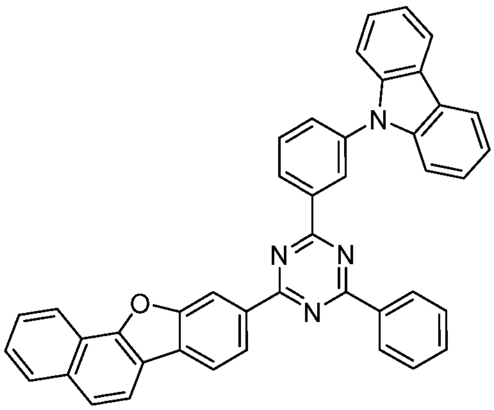

(G22),

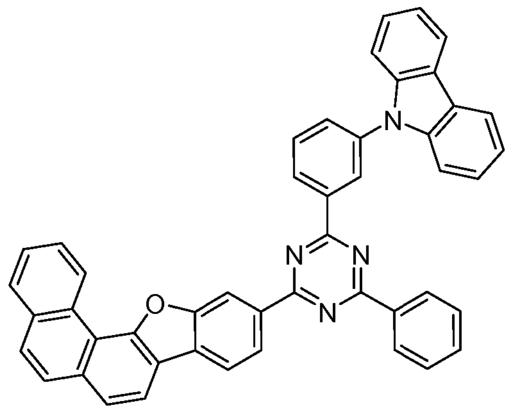

(G23),

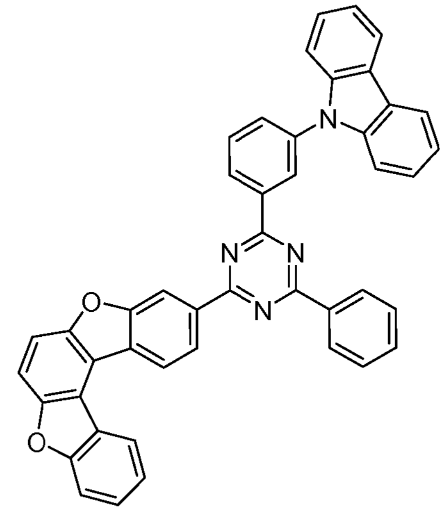

(G24),

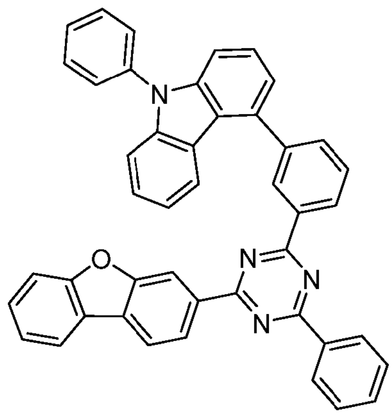

(G25),

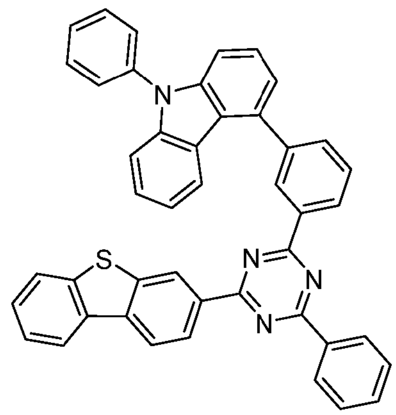

(G26),

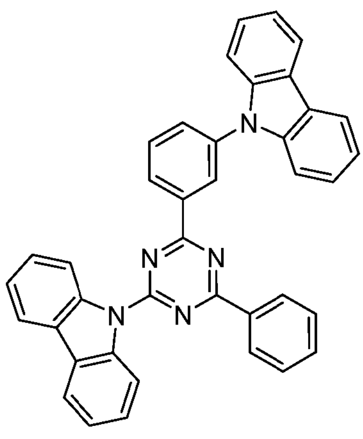

(G27),

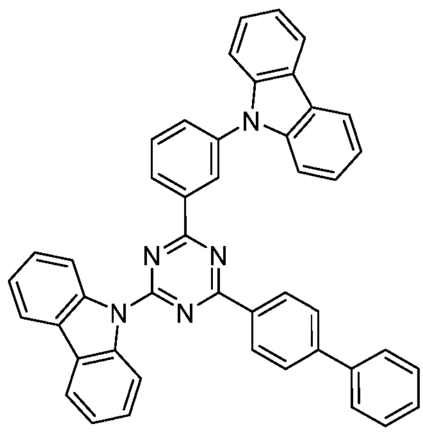

(G28),

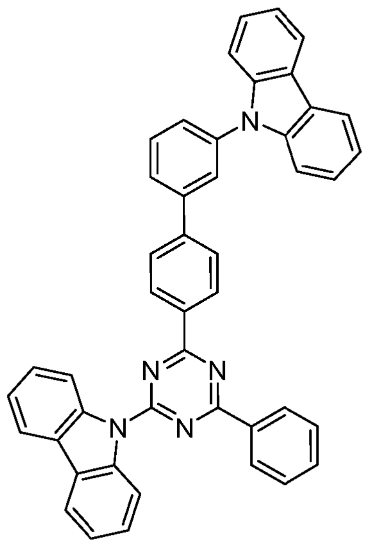

(G29),

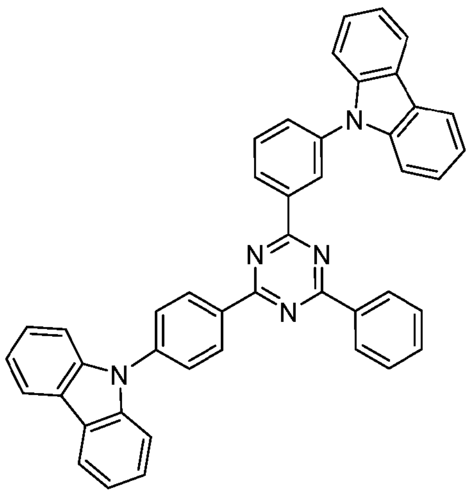

(G30),

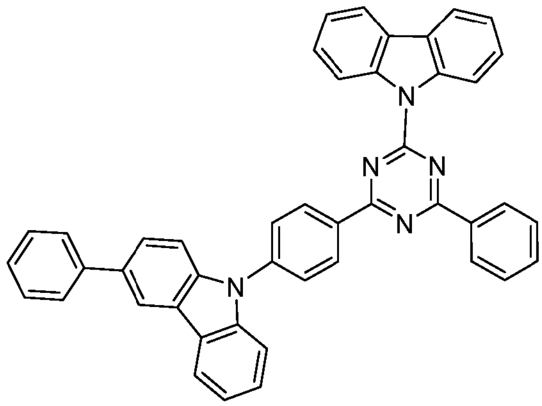

(G31).

**12.** An organic semiconductor layer comprising at least one composition according to any of the preceding claims 1 to 11.

**13.** An organic electronic device comprising at least one organic semiconductor layer according to claim 12.

**14.** The organic electronic device according to claim 13, further comprising at least one anode and at least one cathode, preferably the organic semiconductor layer is arranged between the anode and the cathode.

**15.** The organic electronic device according to any of the preceding claims 13 to 14, wherein the organic electronic device is a light emitting device, thin film transistor, a battery, a display device or a photovoltaic cell, preferably a light emitting device.

**Patentansprüche**

**1.** Zusammensetzung, umfassend

a) eine Verbindung der Formel 1:

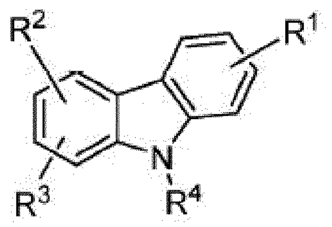

(1),

wobei

$R^1$ und $R^2$ unabhängig aus H, D, CN, Halogen, $C_1$-bis $C_{12}$-Alkyl, $C_1$- bis $C_{12}$-Alkoxy, substituiertem oder unsubstituiertem $C_6$- bis $C_{18}$-Aryl, substituiertem oder unsubstituiertem $C_3$- bis $C_{24}$-Heteroaryl ausgewählt sind, wobei die Substituenten aus $C_1$-bis $C_{12}$-Alkyl, perhalogeniertem $C_1$-bis $C_{12}$-Alkyl, $C_1$- bis $C_{12}$-Alkoxy, perhalogeniertem $C_1$- bis $C_{12}$-Alkoxy, $C_6$- bis $C_{24}$-Aryl, perhalogeniertem $C_6$- bis $C_{24}$-Aryl, $C_3$- bis $C_{24}$-Heteroaryl, CN, Halogen, P(=O)R'R", C(=O)R' oder C(=O)OR' ausgewählt sind, wobei R' und R" unabhängig aus $C_1$- bis $C_{16}$-Alkyl, $C_6$- bis $C_{24}$-Aryl und $C_3$- bis $C_{24}$-Heteroaryl ausgewählt sind;
$R^3$ oder $R^4$ die allgemeine Formel 2 aufweist:

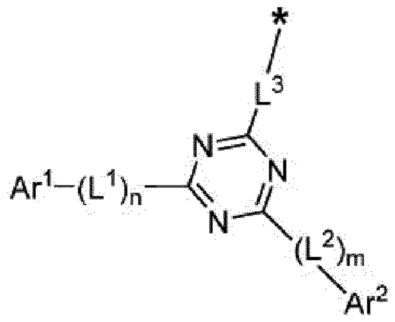

(2),

wobei das Sternchensymbol "*" die Bindungsposition der Gruppe $R^3$ oder $R^4$ anzeigt;
$L^1$, $L^2$ und $L^3$ unabhängig aus substituiertem oder unsubstituiertem $C_6$- bis $C_{13}$-Arylen oder substituiertem oder unsubstituiertem $C_3$- bis $C_{12}$-Heteroarylen ausgewählt sind, wobei die Substituenten aus $C_1$-bis $C_{12}$-Alkyl, perhalogeniertem $C_1$- bis $C_{12}$-Alkyl, $C_1$- bis $C_{12}$-Alkoxy, perhalogeniertem $C_1$-bis $C_{12}$-Alkoxy,$C_6$- bis $C_{18}$-Aryl, perhalogeniertem $C_6$- bis $C_{18}$-Aryl, $C_3$- bis $C_{24}$-Heteroaryl, CN, Halogen ausgewählt sind; n für 1 oder 0 steht; m für 1 oder 0 steht;
$Ar^1$ aus substituiertem oder unsubstituiertem $C_5$- bis $C_{24}$-Heteroaryl ausgewählt ist, wobei die Substituenten aus $C_1$- bis $C_{12}$-Alkyl, perhalogeniertem $C_1$- bis $C_{12}$-Alkyl, $C_1$- bis $C_{12}$-Alkoxy, perhalogeniertem $C_1$- bis $C_{12}$-Alkoxy, $C_6$- bis $C_{24}$-Aryl, perhalogeniertem $C_6$-bis $C_{24}$-Aryl, $C_3$- bis $C_{24}$-Heteroaryl, CN, Halogen, P(=O)R'R", C(=O)R' oder C(=O)OR' ausgewählt sind, wobei R' und R" unabhängig aus $C_1$- bis $C_{16}$-Alkyl, $C_6$- bis $C_{24}$-Aryl und $C_3$- bis $C_{24}$-Heteroaryl ausgewählt sind; und wobei $Ar^1$ spirogruppenfrei ist;
$Ar^2$ die allgemeine Formel 3 aufweist:

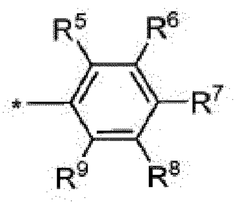

(3),

wobei das Sternchensymbol "*" die Bindungsposition der Gruppe $Ar^2$ an die Gruppierung $L^2$ anzeigt;
$R^5$ bis $R^9$ unabhängig für H, D, CN, Halogen, $C_1$-bis $C_{12}$-Alkyl, $C_1$- bis $C_{12}$-Alkoxy,substituiertes oder unsubstituiertes $C_6$- bis $C_{12}$-Aryl, substituiertes oder unsubstituiertes $C_3$- bis $C_{12}$-Heteroaryl stehen,
wobei der substituierte Arylring bzw. substituierte Heteroarylring durch eine oder mehrere $C_1$- bis $C_{12}$-Alkyl-, $C_6$- bis $C_{12}$-Aryl- oder $C_3$- bis $C_{12}$-Heteroarylgruppen substituiert ist, wobei die Substituenten über eine Einfachbindung an den substituierten Arylring bzw. substituierten Heteroarylring gebunden sind;
oder

$Ar^2$ aus einem anellierten Ringssystem mit einem bis vier substituierten oder unsubstituierten 6-gliedrigen Arylringen und null bis zwei substituierten oder unsubstituierten 5- bis 7-gliedrigen Heteroarylringen ausgewählt ist, wobei der substituierte Arylring durch $C_1$- bis $C_{12}$-Alkylgruppen substituiert ist und der substituierte Heteroarylring durch eine oder mehrere $C_1$- bis $C_{12}$-Alkyl-, $C_6$- bis $C_{12}$-Aryl- oder $C_3$- bis $C_{12}$-Heteroaryl-gruppen substituiert ist;

mit der Maßgabe, dass:

i) $R^3$ für Formel 2 steht und $R^4$ aus $C_1$- bis $C_{12}$-Alkyl, substituiertem oder unsubstituiertem $C_6$- bis $C_{18}$-Aryl, substituiertem oder unsubstituiertem $C_3$-bis $C_{24}$-Heteroaryl ausgewählt ist,
wobei die Substituenten aus $C_1$- bis $C_{12}$-Alkyl, perhalogeniertem $C_1$- bis $C_{12}$-Alkyl, $C_1$- bis $C_{12}$-Alkoxy, perhalogeniertem $C_1$- bis $C_{12}$-Alkoxy, $C_6$- bis $C_{24}$-Aryl, perhalogeniertem $C_6$-bis $C_{24}$-Aryl, $C_3$- bis $C_{24}$-Heteroaryl, CN, Halogen, P(=O)R'R", C(=O)R' oder C(=O)OR' ausgewählt sind,
wobei R' und R" unabhängig aus $C_1$- bis $C_{16}$-Alkyl, $C_6$- bis $C_{24}$-Aryl und $C_3$- bis $C_{24}$-Heteroaryl ausgewählt sind; oder

ii) $R^4$ für Formel 2 steht und $R^3$ für H oder D steht; und

b) mindestens einen organischen Metallkomplex, wobei das Metall des organischen Metallkomplexes aus der Gruppe umfassend Alkali-, Erdalkali- oder Seltenerdmetall ausgewählt ist.

**2.** Zusammensetzung nach Anspruch 1, wobei das Metall des organischen Metallkomplexes aus Alkali- oder Erdalkalimetall ausgewählt ist, weiter bevorzugt das Metall Lithium, Magnesium oder Calcium ist.

**3.** Zusammensetzung nach Anspruch 1 oder 2, wobei der organische Metallkomplex mindestens einen Liganden umfasst, wobei der Ligand aus einer Chinolat- oder Boratgruppe ausgewählt ist.

**4.** Zusammensetzung nach einem der vorhergehenden Ansprüche 1 bis 3, wobei $R^1$ und $R^2$ unabhängig aus H, D, unsubstituiertem $C_6$- bis $C_{18}$-Aryl oder unsubstituiertem $C_3$- bis $C_{24}$-Heteroaryl ausgewählt sind; vorzugsweise $R^1$ und $R^2$ unabhängig aus H, D oder unsubstituiertem $C_6$- bis $C_{18}$-Aryl ausgewählt sind; weiter bevorzugt $R^1$ und $R^2$ unabhängig aus H, D oder unsubstituiertem $C_6$-Aryl ausgewählt sind, und zusätzlich bevorzugt $R^1$ aus H oder D ausgewählt ist und $R^2$ für ein unsubstituiertes $C_6$-Aryl steht oder $R^2$ aus H oder D ausgewählt ist und $R^1$ für ein unsubstituiertes $C_6$-Aryl steht.

**5.** Zusammensetzung nach einem der vorhergehenden Ansprüche 1 bis 4, wobei $Ar^1$ aus substituiertem oder unsubstituiertem $C_5$- bis $C_{24}$-Heteroaryl mit mindestens einem Heteroatom, das aus O, S, Se oder N ausgewählt ist, ausgewählt ist, vorzugsweise das mindestens eine Heteroatom aus O, S, Se ausgewählt ist und zusätzlich bevorzugt das mindestens eine Heteroatom O ist.

**6.** Zusammensetzung nach einem der vorhergehenden Ansprüche 1 bis 5, wobei $Ar^1$ die allgemeine Formel 4 aufweist:

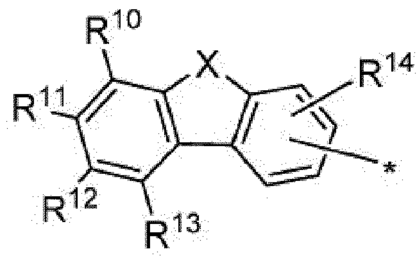

(4),

wobei das Sternchensymbol "*" die Bindungsposition der Gruppe $Ar^1$ an die Gruppierung $L^1$ anzeigt;

X aus O, S, Se oder $NR^{15}$ ausgewählt ist, wobei $R^{15}$ unabhängig aus $C_1$- bis $C_{12}$-Alkyl, perhalogeniertem $C_1$- bis $C_{12}$-Alkyl, $C_6$- bis $C_{24}$-Aryl, perhalogeniertem $C_6$- bis $C_{24}$-Aryl, $C_3$- bis $C_{24}$-Heteroaryl ausgewählt ist,
$R^{10}$ bis $R^{14}$ unabhängig aus H, D, CN, Halogen, $C_1$-bis $C_{12}$-Alkyl, $C_1$- bis $C_{12}$-Alkoxy,$C_6$-bis $C_{18}$-Aryl oder $C_3$- bis $C_{24}$-Heteroaryl ausgewählt sind;

wobei vorzugsweise $R^{10}$ und $R^{11}$, $R^{11}$ und $R^{12}$ oder $R^{12}$ und $R^{13}$ einen 5- bis 7-gliedrigen substituierten oder unsubstituierten Arylring oder einen 5- bis 7-gliedrigen substituierten oder unsubstituierten Heteroarylring bilden und zusätzlich bevorzugt $R^{10}$ und $R^{11}$, $R^{11}$ und $R^{12}$ oder $R^{12}$ und $R^{13}$ zwei anellierte 5- bis 7-gliedrige substituierte oder unsubstituierte Arylringe oder 2 anellierte 5- bis 7-gliedrige substituierte oder unsubstituierte Heteroarylring bilden,
wobei die Substituenten aus $C_1$- bis $C_{12}$-Alkyl-, $C_6$- bis $C_{18}$-Aryl- oder $C_3$- bis $C_{24}$-Heteroaryl-gruppen ausgewählt sind.

**7.** Zusammensetzung nach einem der vorhergehenden Ansprüche 1 bis 6, wobei X der allgemeinen Formel 4 aus O, S oder $NR^{15}$ ausgewählt ist, weiter bevorzugt X aus O oder S ausgewählt ist und auch bevorzugt X für O steht.

**8.** Zusammensetzung nach einem der vorhergehenden Ansprüche 1 bis 7, wobei $Ar^1$ aus der Gruppe umfassend D1 bis D16 ausgewählt ist:

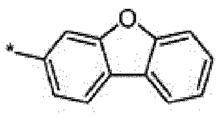
(D1),
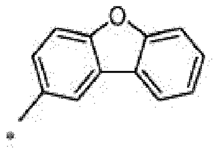
(D2),
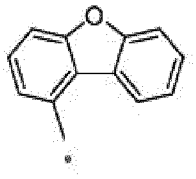
(D3),
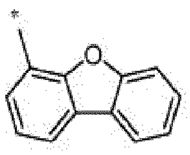
(D4),

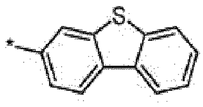
(D5),
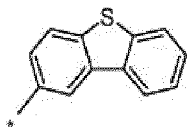
(D6),
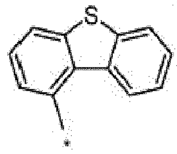
(D7),
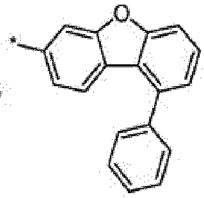
(D8),

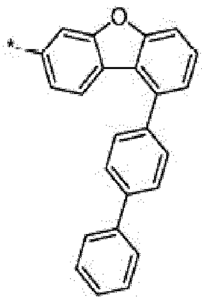
(D9),
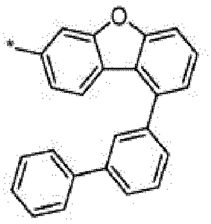
(D10),
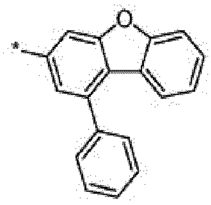
(D11),
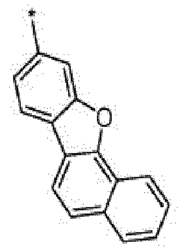
(D12),

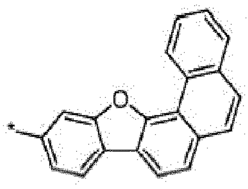
(D13),
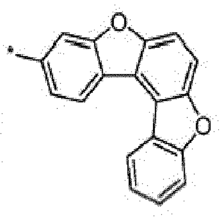
(D14),
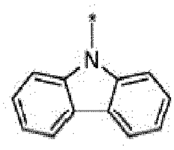
(D15),
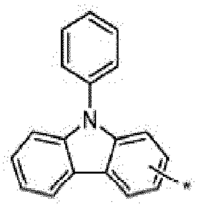
(D16),

vorzugsweise $Ar^1$ aus D1 bis D14 ausgewählt ist, auch bevorzugt $Ar^1$ aus D1, D3, D5, D7, D8 bis D14 ausgewählt ist, weiter bevorzugt $Ar^1$ aus D1 oder D3 ausgewählt ist, ganz besonders bevorzugt $Ar^1$ aus D1 ausgewählt ist.

**9.** Zusammensetzung nach einem der vorhergehenden Ansprüche 1 bis 8, wobei $Ar^2$ aus der Gruppe umfassend E1 bis E44 ausgewählt ist:

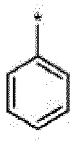
(E1),
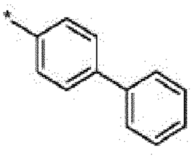
(E2),
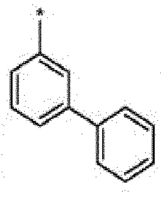
(E3,
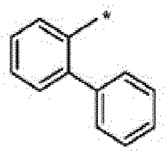
(E4),
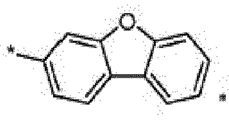
(E5),
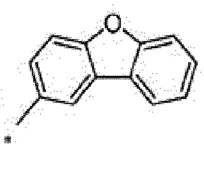
(E6),

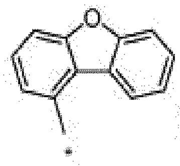 (E7), 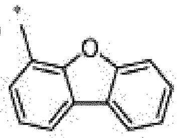 (E8), 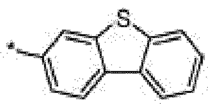 (E9), 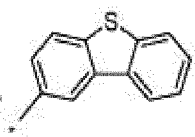 (E10), 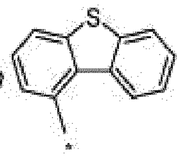 (E11),

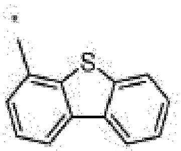 (E12), 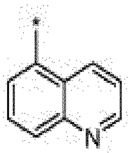 (E13), 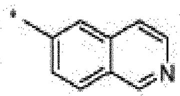 (E14), 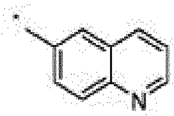 (E15), 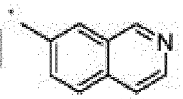 (E16),

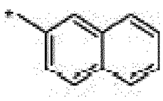 (E17), 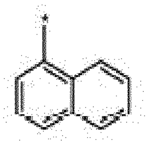 (E18), 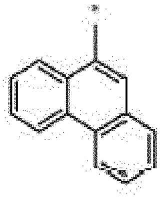 (E19), 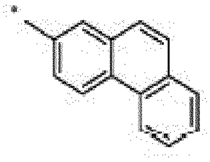 (E20), 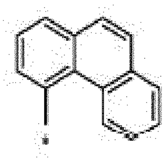 (E21),

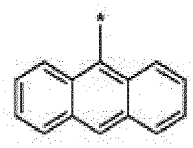 (E22), 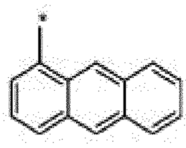 (E23), 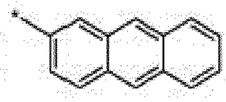 (E24), 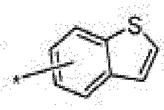 (E25), 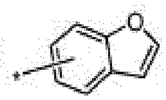 (E26),

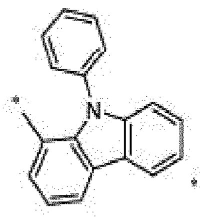 (E27), 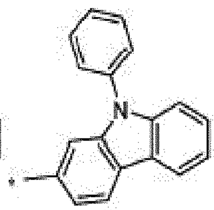 (E28), 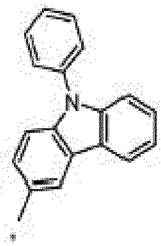 (E29), 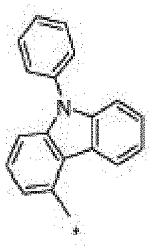 (E30), 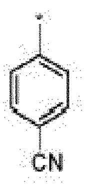 (E31), 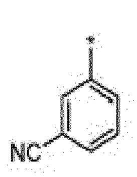 (E32),

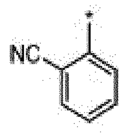 (E33), 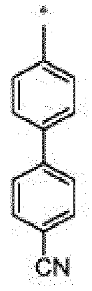 (E34), 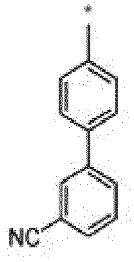 (E35), 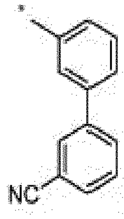 (E36), 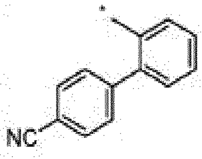 (E37), 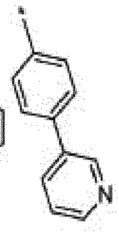 (E38), 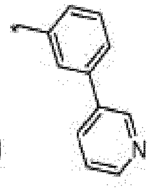 (E39),

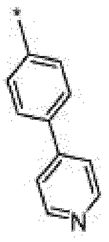 (E40), 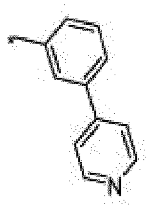 (E41), 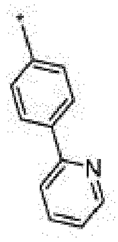 (E42), 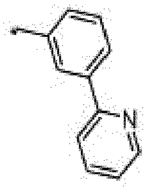 (E43), 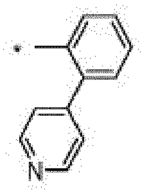 (E44);

vorzugsweise $Ar^2$ aus E1 bis E26 und E31 bis E44 ausgewählt ist; weiter bevorzugt $Ar^2$ aus E1 bis E12, E17 bis E19 und E31 bis E37 ausgewählt ist; auch bevorzugt $Ar^2$ aus E1 bis E12, E17 bis E19 und E31 bis E37 ausgewählt ist; ganz besonders bevorzugt $Ar^2$ aus E1 bis E4 und E17 bis E18 ausgewählt ist.

**10.** Zusammensetzung nach einem der vorhergehenden Ansprüche 1 bis 9, wobei

e) $L^1$, $L^2$ und/oder $L^3$ unabhängig aus der Gruppe umfassend F1 bis F11 ausgewählt sind:

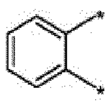 (F1), 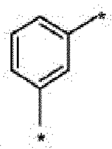 (F2), 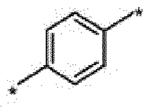 (F3), 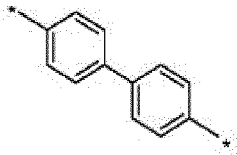 (F4),

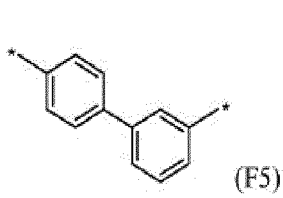 (F5), 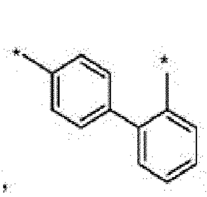 (F6), 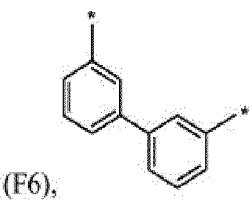 (F7), 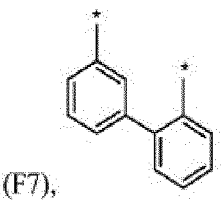 (F8),

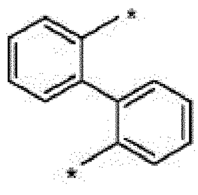 (F9), 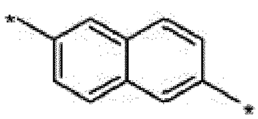 (F10), 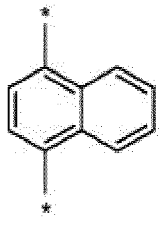 (F11);

vorzugsweise $L^1$, $L^2$ und/oder $L^3$ aus F2, F3, F4, F5 oder F7 ausgewählt sind, weiter bevorzugt $L^1$, $L^2$ und/oder $L^3$ aus F2, F3, F5 oder F7 ausgewählt sind, weiter bevorzugt $L^1$, $L^2$ und/oder $L^3$ aus F2 oder F3 ausgewählt sind; oder

f) für $L^1$ n für 1 steht und für $L^2$ m für 0 steht, $L^1$ aus F1 bis F11 ausgewählt ist, vorzugsweise $L^1$ aus F2, F3, F4, F5 oder F7 ausgewählt ist, weiter bevorzugt $L^1$ aus F2, F3, F5 oder F7 ausgewählt ist, weiter bevorzugt $L^1$ aus F2 oder F3 ausgewählt ist; oder

g) für $L^1$ n für 0 steht und für $L^2$ m für 1 steht, $L^2$ aus F1 bis F11 ausgewählt ist, vorzugsweise $L^2$ aus F2, F3, F4, F5 oder F7 ausgewählt ist, weiter bevorzugt $L^2$ aus F2, F3, F5 oder F7 ausgewählt ist, weiter bevorzugt $L^2$ aus F2 oder F3 ausgewählt ist; oder

h) für $L^1$ n für 0 steht und für $L^2$ m für 0 steht und $L^3$ aus F1 bis F11 ausgewählt ist, vorzugsweise $L^3$ aus F2, F3, F4, F5 oder F7 ausgewählt ist, weiter bevorzugt $L^3$ aus F2, F3, F5 oder F7 ausgewählt ist, weiter bevorzugt $L^3$ aus F2 oder F3 ausgewählt ist.

**11.** Zusammensetzung nach einem der vorhergehenden Ansprüche 1 bis 10, wobei die Verbindungen der Formel 1 aus der Gruppe umfassend G1 bis G31 ausgewählt sind:

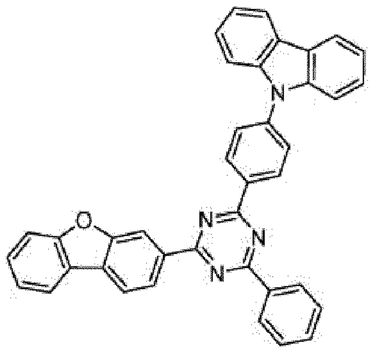

(G1),

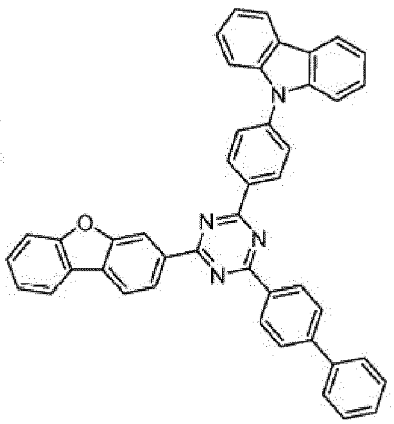

(G2),

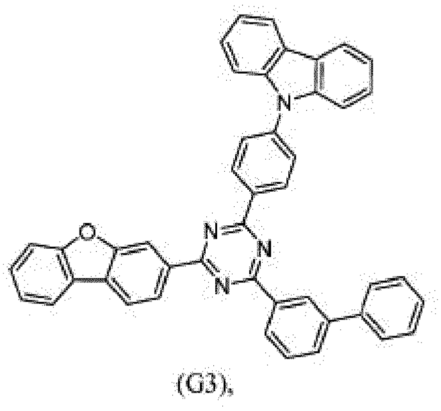

(G3),

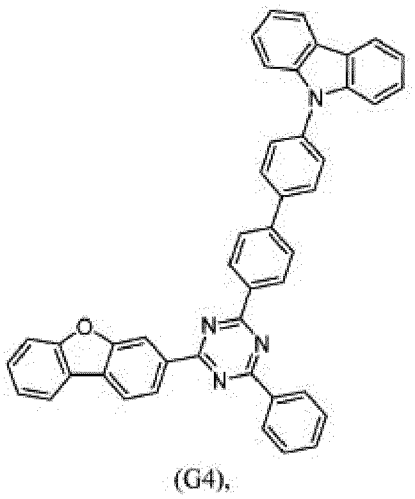

(G4),

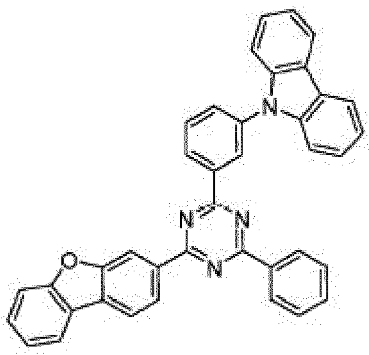

(G5),

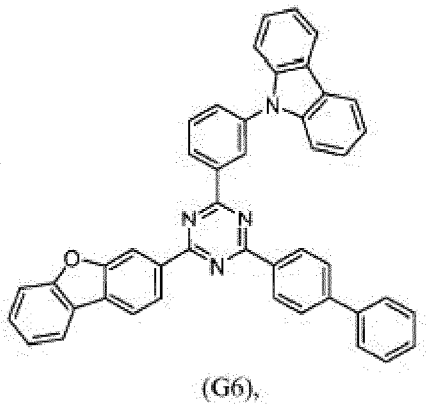

(G6),

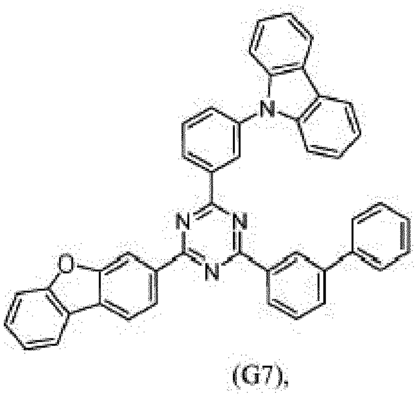

(G7),

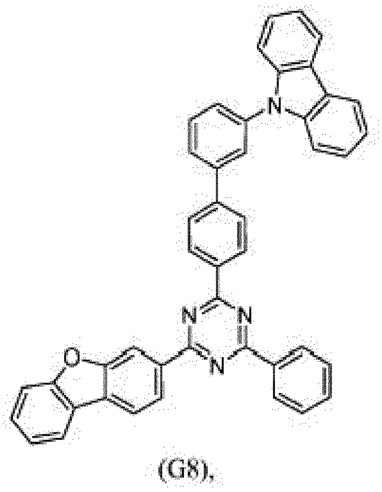

(G8),

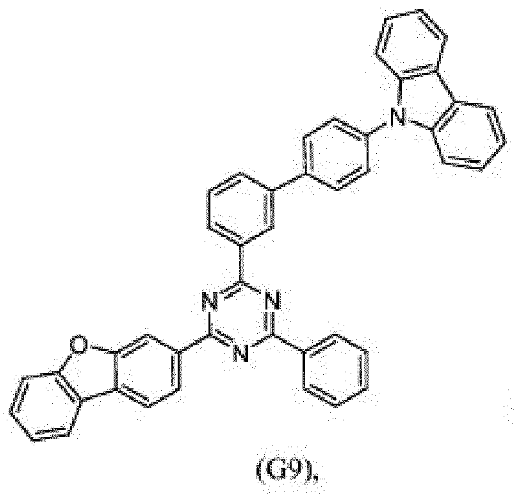

(G9),

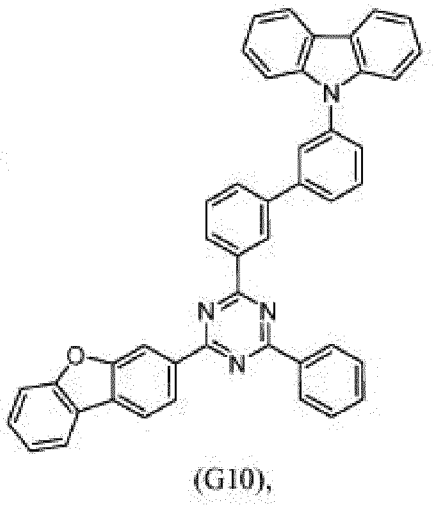

(G10),

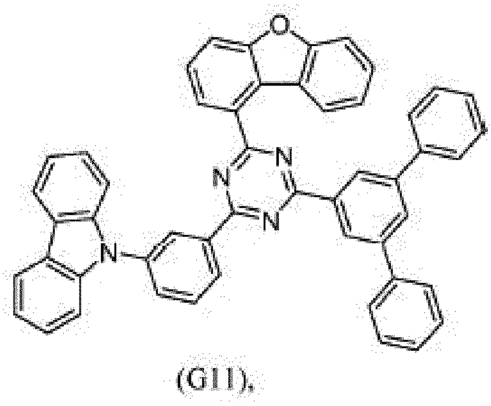

(G11),

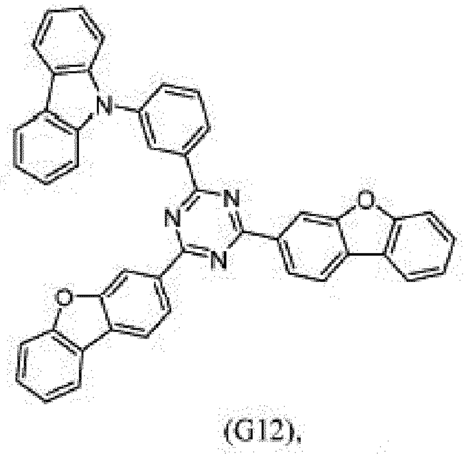

(G12),

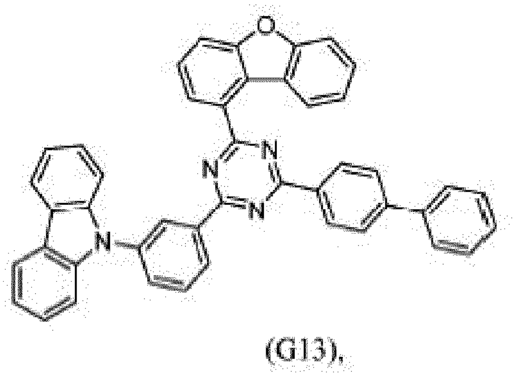

(G13),

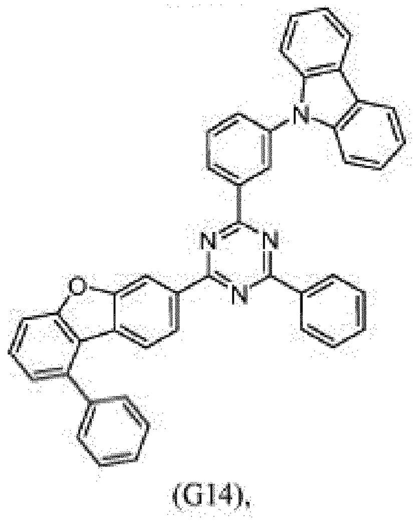

(G14),

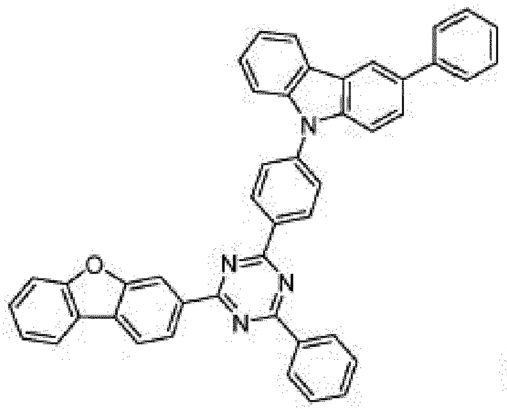

(G15),

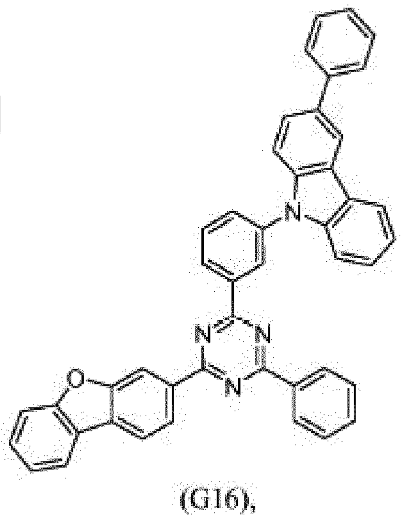

(G16),

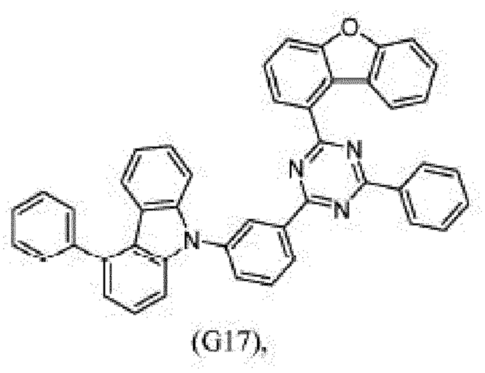
(G17),

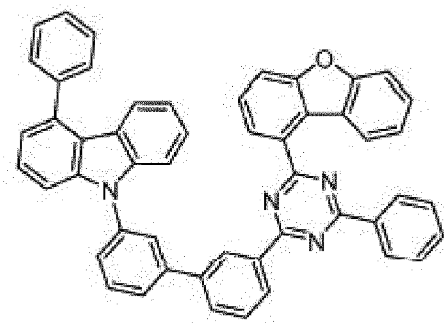
(G18),

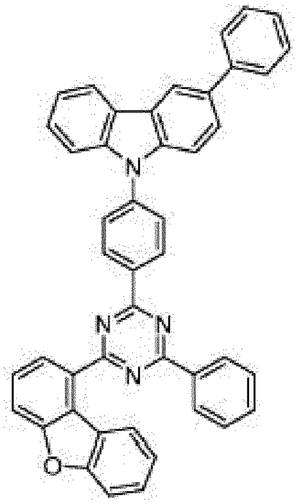
(G19),

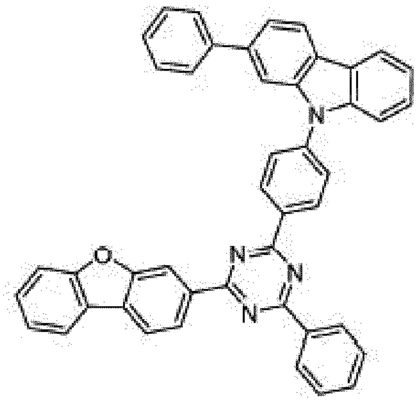
(G20),

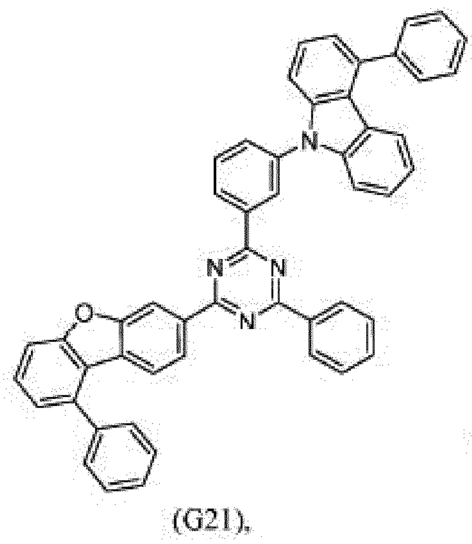
(G21),

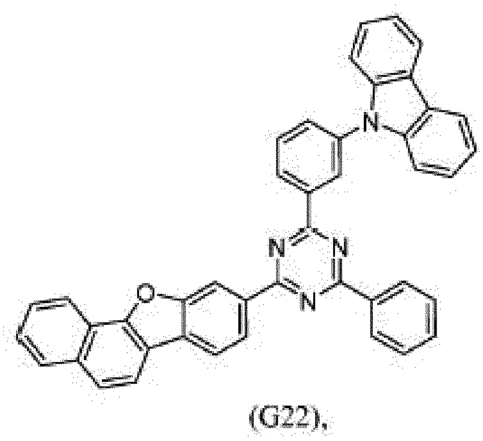
(G22),

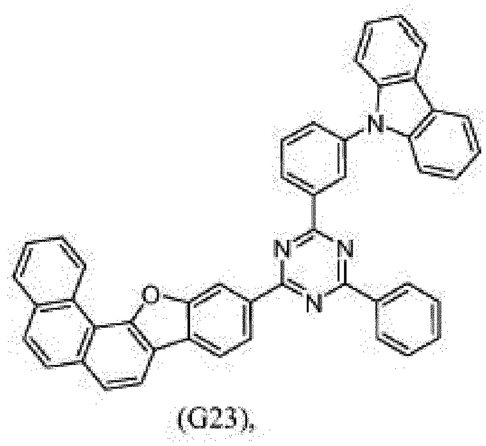

(G23),

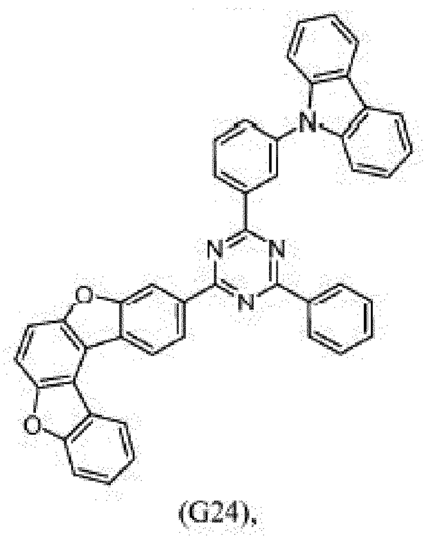

(G24),

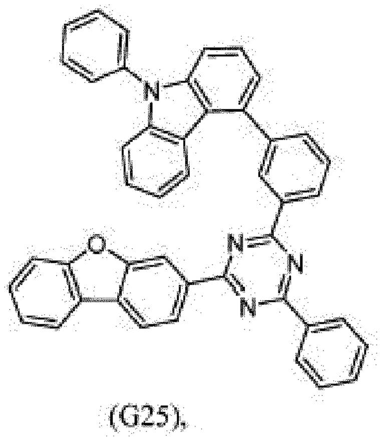

(G25),

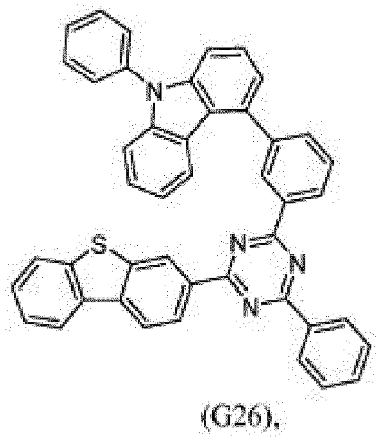

(G26),

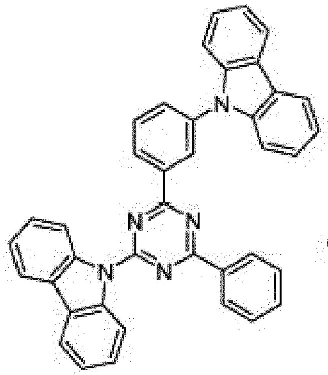

(G27),

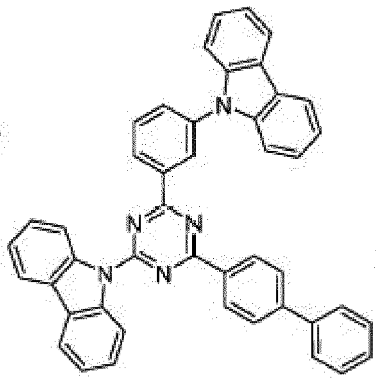

(G28),

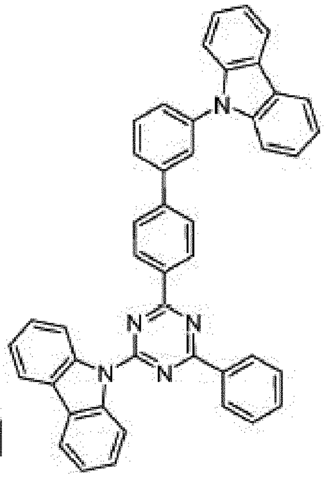

(G29),

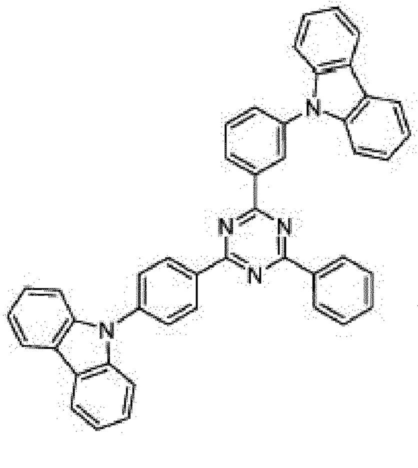

(G30),

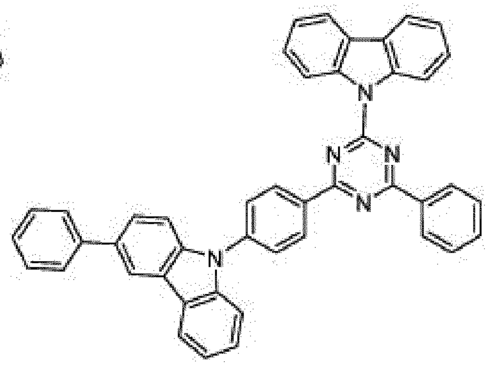

(G31).

12. Organische Halbleiterschicht, umfassend mindestens eine Zusammensetzung nach einem der vorhergehenden Ansprüche 1 bis 11.

13. Organische elektronische Vorrichtung, umfassend mindestens eine organische Halbleiterschicht nach Anspruch 12.

14. Organische elektronische Vorrichtung nach Anspruch 13, ferner umfassend mindestens eine Anode und mindestens eine Kathode, wobei die organische Halbleiterschicht vorzugsweise zwischen der Anode und der Kathode angeordnet ist.

15. Organische elektronische Vorrichtung nach einem der vorhergehenden Ansprüche 13 bis 14, wobei es sich bei der organischen elektronischen Vorrichtung um eine lichtemittierende Vorrichtung, einen Dünnschichttransistor, eine Batterie, eine Anzeigevorrichtung oder eine Photovoltaikzelle, vorzugsweise eine lichtemittierende Vorrichtung, handelt.

**Revendications**

1. Composition comprenant

a) un composé de formule 1 :

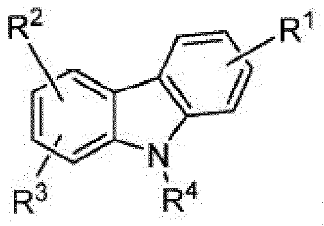

(1),

$R^1$ étant indépendamment choisis parmi H, D, CN, halogène, $C_1$ à $C_{12}$ alkyle, $C_1$ à $C_{12}$ alcoxy, $C_6$ à $C_{18}$ aryle substitué ou non substitué, $C_3$ à $C_{24}$ hétéroaryle substitué ou non substitué,
et $R^2$ les substituants étant choisis parmi $C_1$ à $C_{12}$ alkyle, $C_1$ à $C_{12}$ alkyle perhalogéné, $C_1$ à $C_{12}$ alcoxy, $C_1$ à $C_{12}$ alcoxy perhalogéné, C6 à C24 aryle, C6 à $C_{24}$ aryle perhalogéné, $C_3$ à $C_{24}$ hétéroaryle, CN, halogène, P(=O)R'R", C(=O)R' et C(=O)OR', R' et R" étant indépendamment choisis parmi $C_1$ à $C_{16}$ alkyle, $C_6$ à C24 aryle, C3 à C24 hétéroaryle ;
$R^3$ ou $R^4$ possédant la formule générale 2 :

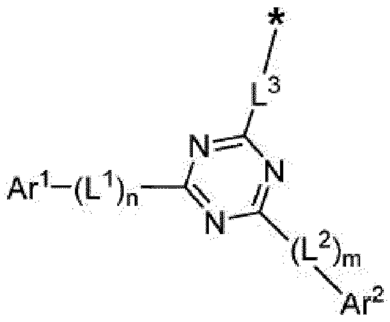

(2),

le symbole astérisque « * » représentant la position de liaison du groupe $R^3$ ou $R^4$ ;
$L^1$, étant indépendamment choisis parmi $C_6$ à $C_{13}$ arylène substitué ou non substitué et $C_3$ à $C_{12}$ hétéroarylène substitué ou non substitué,
$L^2$ et $L^3$ les substituants étant choisis parmi $C_1$ à $C_{12}$ alkyle, $C_1$ à $C_{12}$ alkyle perhalogéné, $C_1$ à $C_{12}$ alcoxy, $C_1$ à $C_{12}$ alcoxy perhalogéné, $C_6$ à $C_{18}$ aryle, $C_6$ à $C_{18}$ aryle perhalogéné, $C_3$ à $C_{24}$ hétéroaryle, CN, halogène ;
n étant 1 ou 0 ;
m étant 1 ou 0 ;
$Ar^1$ étant choisi parmi $C_5$ à $C_{24}$ hétéroaryle substitué ou non substitué, les substituants étant choisis parmi $C_1$ à $C_{12}$ alkyle, $C_1$ à $C_{12}$ alkyle perhalogéné, $C_1$ à $C_{12}$ alcoxy, $C_1$ à $C_{12}$ alcoxy perhalogéné, $C_6$ à $C_{24}$ aryle, $C_6$ à $C_{24}$ aryle perhalogéné, $C_3$ à $C_{24}$ hétéroaryle, CN, halogène, P(=O)R'R", C(=O)R' et C(=O)OR', R' et R" étant indépendamment choisis parmi $C_1$ à $C_{16}$ alkyle, $C_6$ à $C_{24}$ aryle, $C_3$ à $C_{24}$ hétéroaryle ; et $Ar^1$ étant exempt de groupe spiro ;
$Ar^2$ possédant la formule générale 3 :

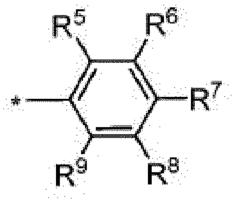

(3),

le symbole astérisque « * » représentant la position de liaison du groupe $Ar^2$ au fragment L2 ;

$R^5$ à $R^9$ étant indépendamment H, D, CN, halogène,
$C_1$ alkyle, $C_1$ à $C_{12}$ alcoxy, $C_6$ à $C_{12}$ aryle substitué ou non substitué, $C_3$ à $C_{12}$ hétéroaryle substitué ou non substitué,
à $C_{12}$ le cycle aryle substitué ou le cycle hétéroaryle substitué étant substitué par un ou plusieurs groupes $C_1$ à $C_{12}$ alkyle, $C_6$ à $C_{12}$ aryle ou $C_3$ à $C_{12}$ hétéroaryle, les substituants étant liés par une simple liaison au cycle aryle substitué ou au cycle hétéroaryle substitué ;
ou
$Ar^2$ étant choisi parmi un système cyclique condensé comprenant un à quatre cycles aryle à 6 chaînons substitués ou non substitués et zéro à deux cycles hétéroaryle à 5 à 7 chaînons substitués ou non substitués, le cycle aryle substitué étant substitué par des groupes $C_1$ à $C_{12}$ alkyle et le cycle hétéroaryle substitué étant substitué par un ou plusieurs groupes $C_1$ à $C_{12}$ alkyle, $C_6$ à $C_{12}$ aryle ou $C_3$ à $C_{12}$ hétéroaryle ;

à la condition que :

i) $R^3$ soit la formule 2 et $R^4$ soit choisi parmi $C_1$ à $C_{12}$ alkyle, $C_6$ à $C_{18}$ aryle substitué ou non substitué, $C_3$ à $C_{24}$ hétéroaryle substitué ou non substitué,
les substituants étant choisis parmi $C_1$ à $C_{12}$ alkyle, $C_1$ à $C_{12}$ alkyle perhalogéné, $C_1$ à $C_{12}$ alcoxy, $C_1$ à $C_{12}$ alcoxy perhalogéné, $C_6$ à C24 aryle, $C_6$ à $C_{24}$ aryle perhalogéné, $C_3$ à $C_{24}$ hétéroaryle, CN, halogène, P(=O)R'R", C(=O)R' et C(=O)OR',

R' et R" étant indépendamment choisis parmi $C_1$ à $C_{16}$ alkyle, $C_6$ à $C_{24}$ aryle, $C_3$ à $C_{24}$ hétéroaryle ; ou

ii) $R^4$ soit la formule 2 et $R^3$ soit H ou D ; et

b) au moins un complexe métallique organique, le métal du complexe métallique organique étant choisi dans le groupe comprenant un métal alcalin, un métal alcalino-terreux et un métal des terres rares.

**2.** Composition selon la revendication 1, le métal du complexe métallique organique étant choisi parmi un métal alcalin et un métal alcalino-terreux, plus préférablement le métal étant le lithium, le magnésium ou le calcium.

**3.** Composition selon la revendication 1 ou 2, le complexe métallique organique comprenant au moins un ligand, le ligand étant choisi parmi un groupe quinolate et un groupe borate.

**4.** Composition selon l'une quelconque des revendications précédentes 1 à 3, $R^1$ et $R^2$ étant indépendamment choisis parmi H, D, $C_6$ à $C_{18}$ aryle non substitué, et $C_3$ à $C_{24}$ hétéroaryle non substitué ; préférablement $R^1$ et $R^2$ étant indépendamment choisis parmi H, D et $C_6$ à $C_{18}$ aryle non substitué ; de manière plus préférée $R^1$ et $R^2$ étant indépendamment choisis parmi H, D et $C_6$ aryle non substitué, et de plus de manière préférée $R^1$ étant choisi parmi H et D et $R^2$ étant un $C_6$ aryle non substitué, ou $R^2$ étant choisi parmi H et D et $R^1$ étant un $C_6$ aryle non substitué.

**5.** Composition selon l'une quelconque des revendications précédentes 1 à 4, $Ar^1$ étant choisi parmi $C_5$ à $C_{24}$ hétéroaryle substitué ou non substitué comprenant au moins un hétéroatome choisi parmi O, S, Se et N, préférablement l'au moins un hétéroatome étant choisi parmi O, S, Se et de plus de manière préférée l'au moins un hétéroatome étant O.

**6.** Composition selon l'une quelconque des revendications précédentes 1 à 5, $Ar^1$ possédant la formule générale 4 :

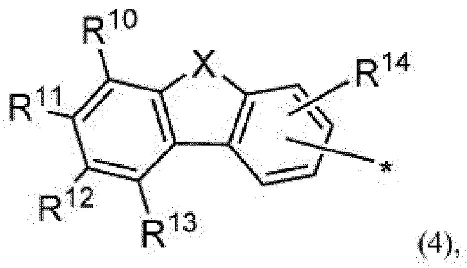

(4),

le symbole astérisque « * » représentant la position de liaison du groupe $Ar^1$ au fragment $L^1$ ;

X étant choisi parmi O, S, Se et $NR^{15}$, $R^{15}$ étant indépendamment choisi parmi $C_1$ à $C_{12}$ alkyle, $C_1$ à $C_{12}$ alkyle perhalogéné, $C_6$ à C24 aryle, $C_6$ à $C_{24}$ aryle perhalogéné, $C_3$ à $C_{24}$ hétéroaryle ;

$R^{10}$ à $R^{14}$ étant indépendamment choisis parmi H, D, CN, halogène, $C_1$ à $C_{12}$ alkyle, $C_1$ à $C_{12}$ alcoxy, $C_6$ à $C_{18}$ aryle et $C_3$ à $C_{24}$ hétéroaryle ;

préférablement $R^{10}$ et $R^{11}$, $R^{11}$ et $R^{12}$ ou $R^{12}$ et $R^{13}$ formant un cycle aryle substitué ou non substitué à 5 à 7 chaînons, ou un cycle hétéroaryle substitué ou non substitué à 5 à 7 chaînons, et de plus de manière préférée $R^{10}$ et $R^{11}$, $R^{11}$ et $R^{12}$ ou $R^{12}$ et $R^{13}$ formant deux cycles aryle substitués ou non substitués à 5 à 7 chaînons condensés, ou deux cycles hétéroaryle substitués ou non substitués à 5 à 7 chaînons condensés,
les substituants étant choisis parmi les groupes $C_1$ à $C_{12}$ alkyle, $C_6$ à $C_{18}$ aryle et $C_3$ à $C_{24}$ hétéroaryle.

**7.** Composition selon l'une quelconque des revendications précédentes 1 à 6, X de la formule générale 4 étant choisi parmi O, S et $NR^{15}$, de manière plus préférée X étant choisi parmi O et S, et également de manière préférée X étant O.

**8.** Composition selon l'une quelconque des revendications précédentes 1 à 7, $Ar^1$ étant choisi dans le groupe comprenant D1 à D16 :

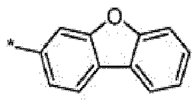
(D1),
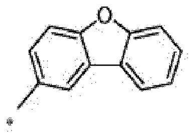
(D2),
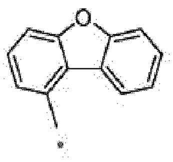
(D3),
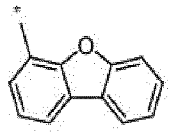
(D4),

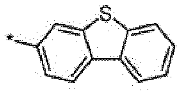
(D5),
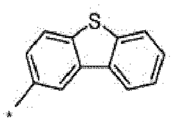
(D6),
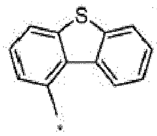
(D7),
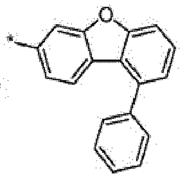
(D8),

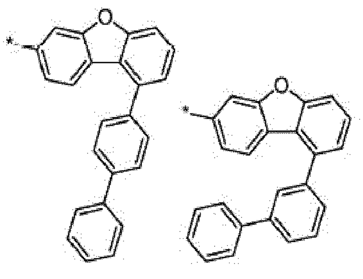
(D9), (D10),
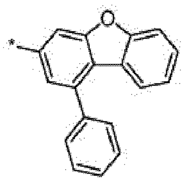
(D11),
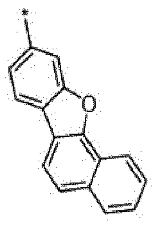
(D12),

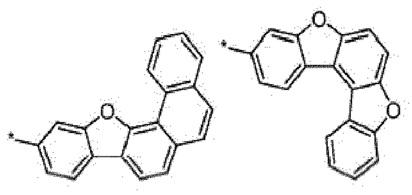
(D13), (D14),
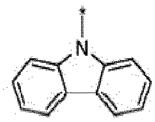
(D15),
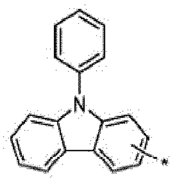
(D16),

préférablement $Ar^1$ étant choisi parmi D1 à D14, également de manière préférée $Ar^1$ étant choisi parmi D1, D3, D5, D7, D8 à D14, plus préférablement $Ar^1$ étant choisi parmi D1 et D3, de la manière la plus préférée $Ar^1$ étant choisi parmi D1.

**9.** Composition selon l'une quelconque des revendications précédentes 1 à 8, $Ar^2$ étant choisi dans le groupe comprenant E1 à E44 :

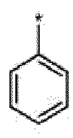
(E1),
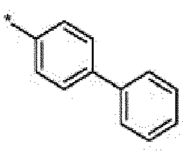
(E2),
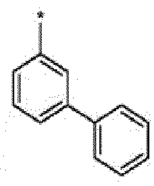
(E3,
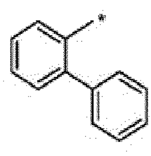
(E4),
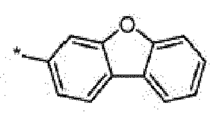
(E5),
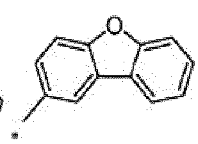
(E6),

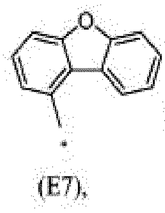 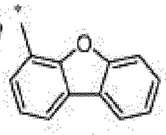 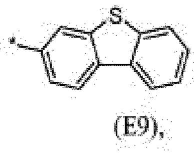 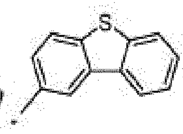 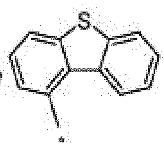

(E7), (E8), (E9), (E10), (E11),

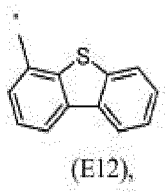 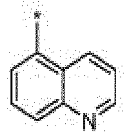 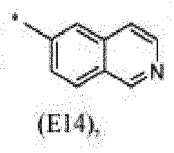 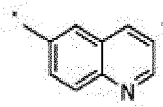 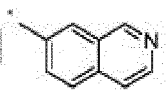

(E12), (E13), (E14), (E15), (E16),

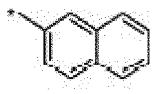 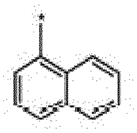 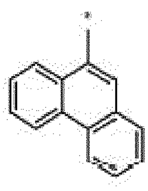 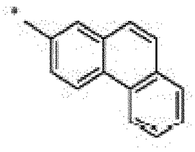 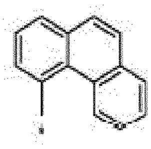

(E17), (E18), (E19), (E20), (E21),

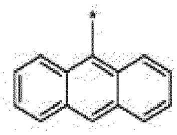 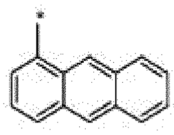 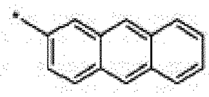 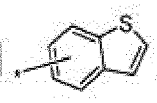 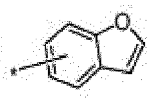

(E22), (E23), (E24), (E25), (E26),

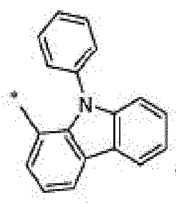 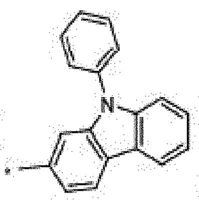 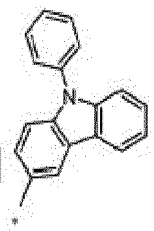 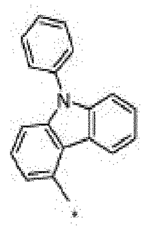 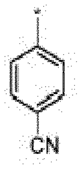 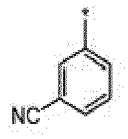

(E27), (E28), (E29), (E30), (E31), (E32),

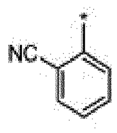 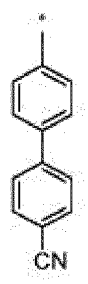 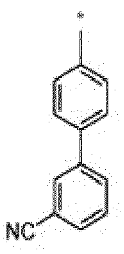 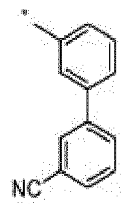 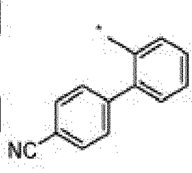 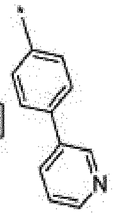 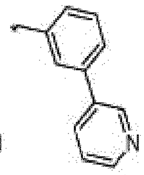

(E33), (E34), (E35), (E36), (E37), (E38), (E39),

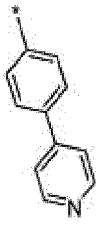 (E40), 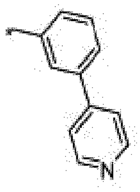 (E41), 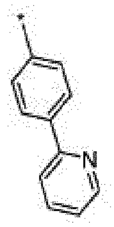 (E42), 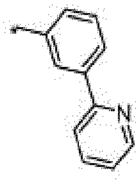 (E43), 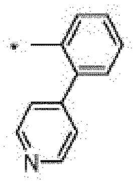 (E44);

préférablement $Ar^2$ étant choisi parmi E1 à E26 et E31 à E44 ; plus préférablement $Ar^2$ étant choisi parmi E1 à E12, E17 à E19 et E31 à E37 ; également de manière préférée $Ar^2$ étant choisi parmi E1 à E12, E17 à E19 et E31 à E37 ; de la manière la plus préférée $Ar^2$ étant choisi parmi E1 à E4 et E17 à E18.

**10.** Composition selon l'une quelconque des revendications précédentes 1 à 9,

e) $L^1$, $L^2$ et/ou $L^3$ étant indépendamment choisis dans le groupe comprenant F1 à F11 :

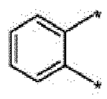 (F1), 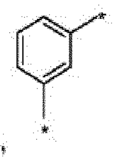 (F2), 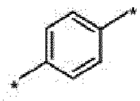 (F3), 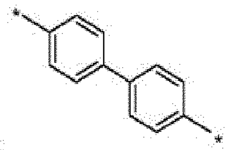 (F4),

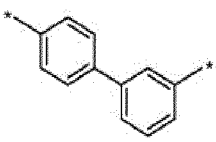 (F5), 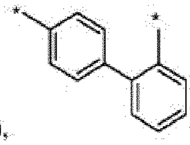 (F6), 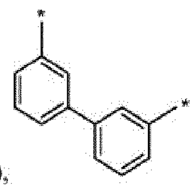 (F7), 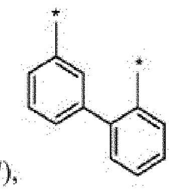 (F8),

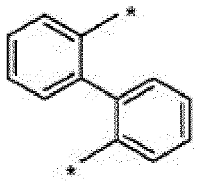 (F9), 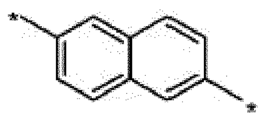 (F10), 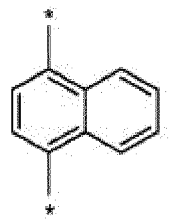 (F11);

préférablement $L^1$, $L^2$ et/ou $L^3$ étant choisis parmi F2, F3, F4, F5 et F7, plus préférablement $L^1$, $L^2$ et/ou $L^3$ étant choisis parmi F2, F3, F5 et F7, de manière plus préférée $L^1$, $L^2$ et/ou $L^3$ étant choisis parmi F2 et F3 ; ou
f) pour $L^1$ n étant 1 et pour $L^2$ m étant 0, $L^1$ étant choisi parmi F1 à F11, préférablement $L^1$ étant choisi parmi F2, F3, F4, F5 et F7, plus préférablement $L^1$ étant choisi parmi F2, F3, F5 et F7, de manière plus préférée $L^1$ étant choisi parmi F2 et F3 ; ou
g) pour $L^1$ n étant 0 et pour $L^2$ m étant 1, $L^2$ étant choisi parmi F1 à F11, préférablement $L^2$ étant choisi parmi F2, F3, F4, F5 et F7, plus préférablement $L^2$ étant choisi parmi F2, F3, F5 et F7, de manière plus préférée $L^2$ étant choisi parmi F2 et F3 ; ou
h) pour $L^1$ n étant 0 et pour $L^2$ m étant 0 et $L^3$ étant choisi parmi F1 à F11, préférablement $L^3$ étant choisi parmi F2, F3, F4, F5 et F7, plus préférablement $L^3$ étant choisi parmi F2, F3, F5 et F7, de manière plus préférée $L^3$ étant choisi parmi F2 et F3.

**11.** Composition selon l'une quelconque des revendications précédentes 1 à 10, les composés de formule 1 étant

choisis dans le groupe comprenant G1 à G31 :

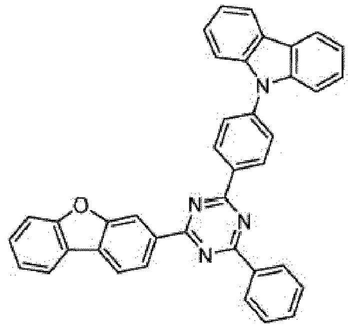

(G1),

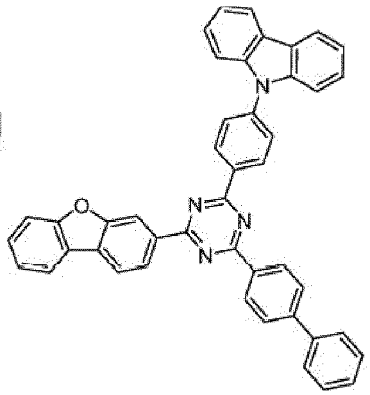

(G2),

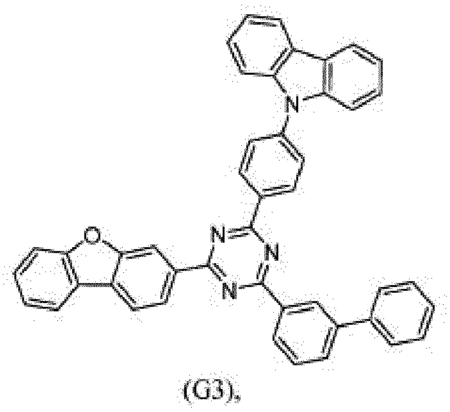

(G3),

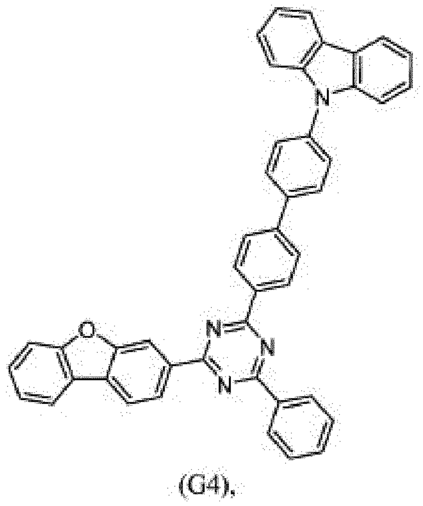

(G4),

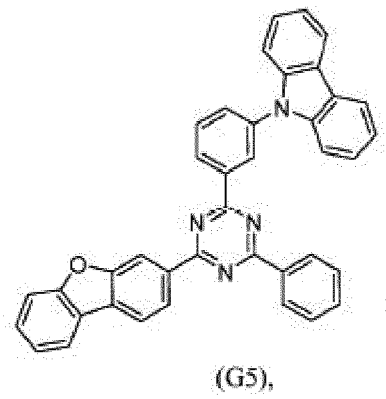

(G5),

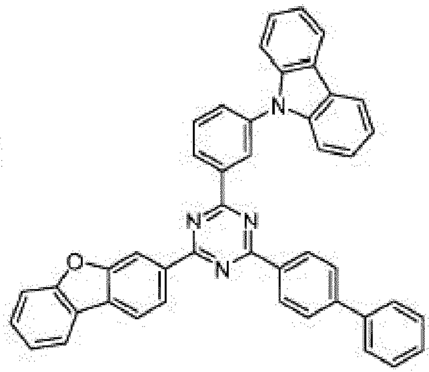

(G6),

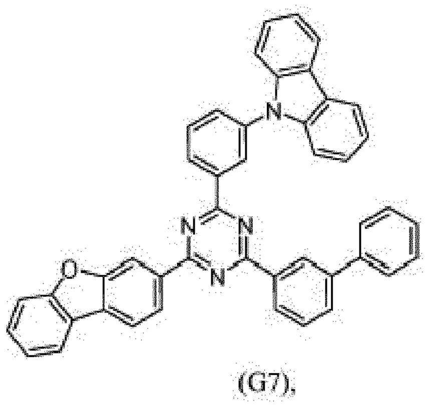

(G7),

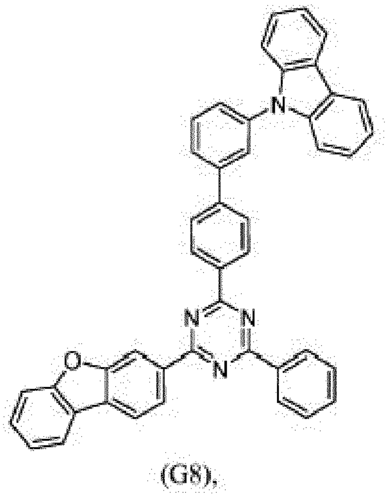

(G8),

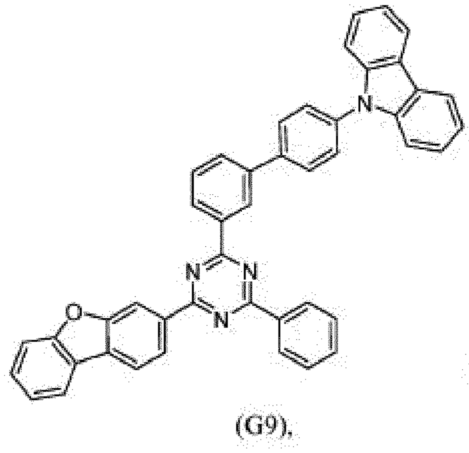

(G9),

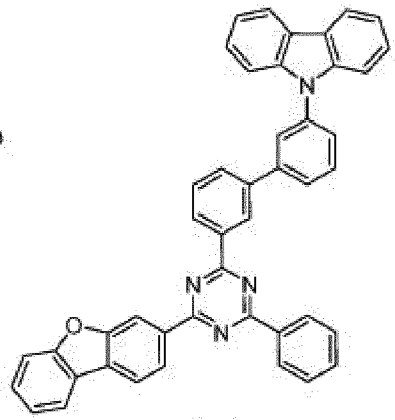

(G10),

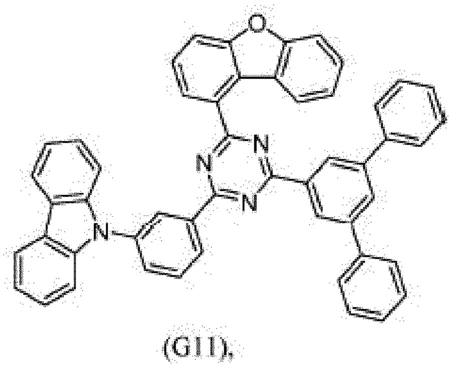

(G11),

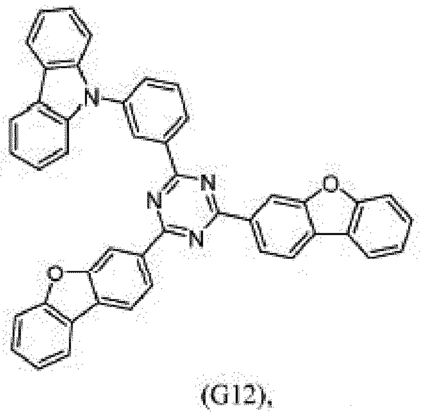

(G12),

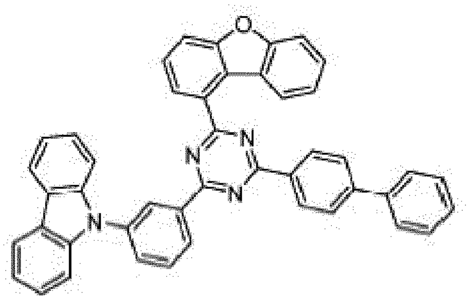

(G13),

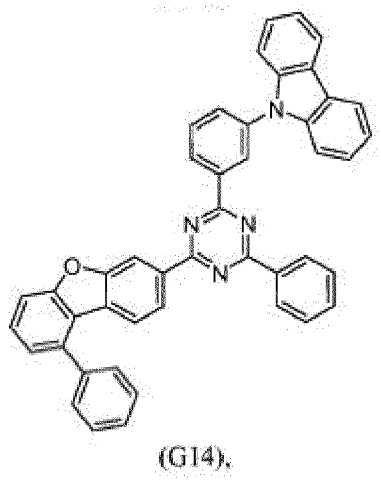

(G14),

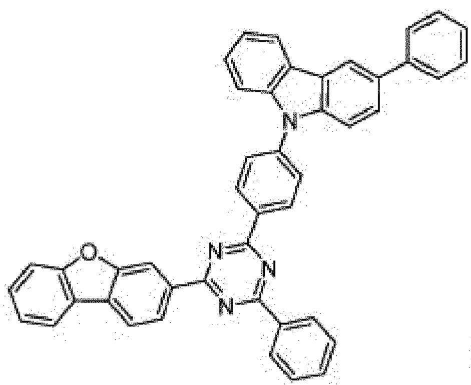

(G15),

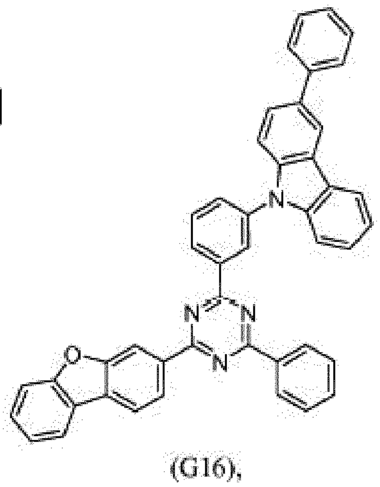

(G16),

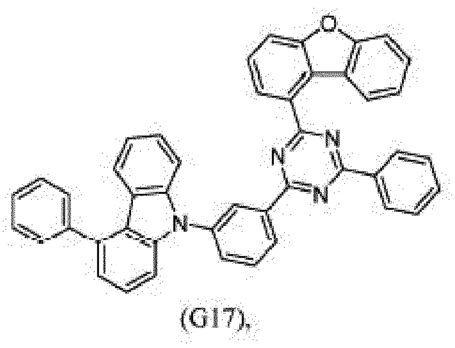

(G17),

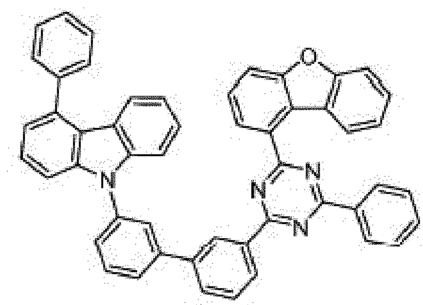

(G18),

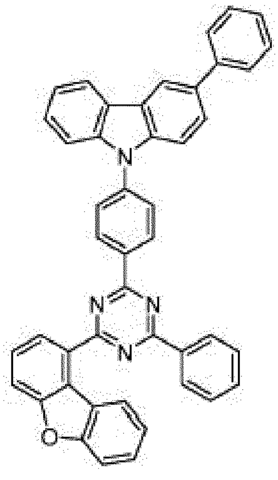

(G19),

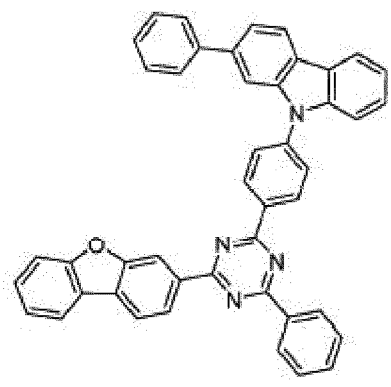

(G20),

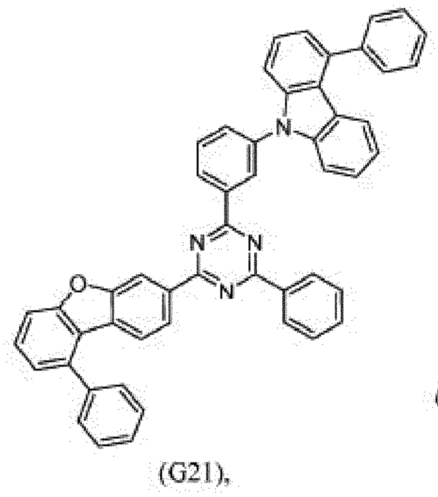

(G21),

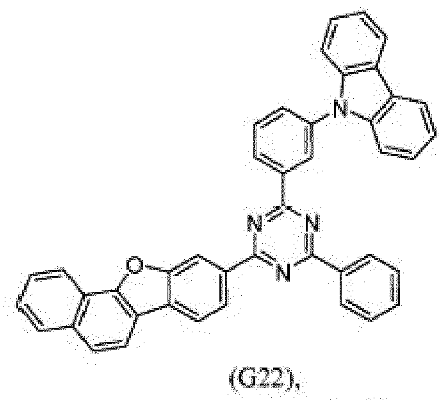

(G22),

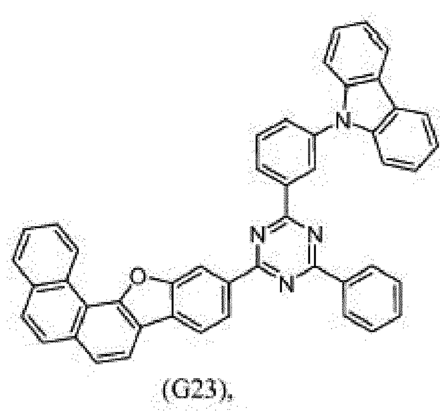

(G23),

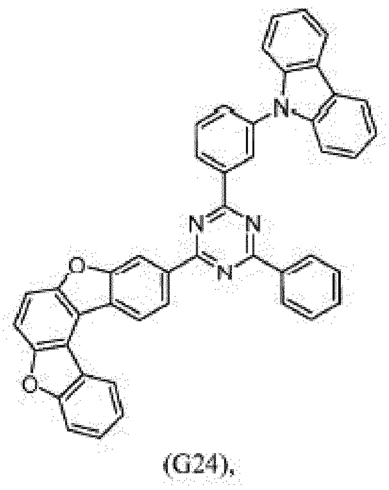

(G24),

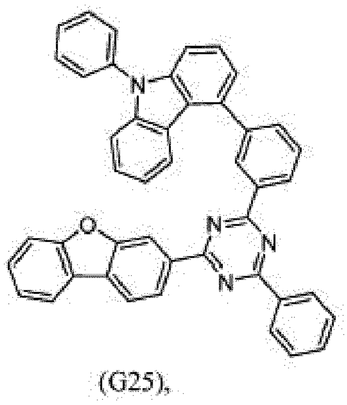

(G25),

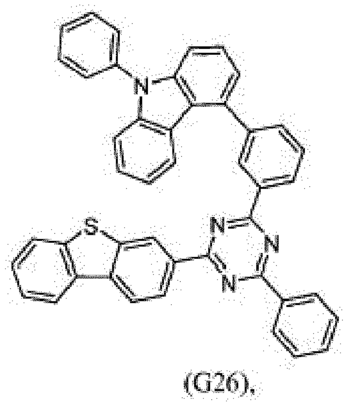

(G26),

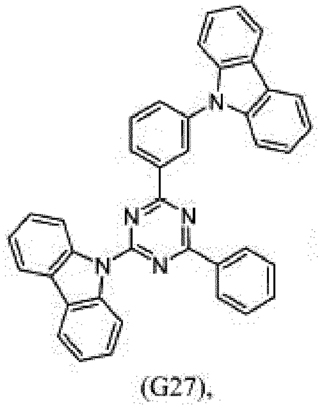

(G27),

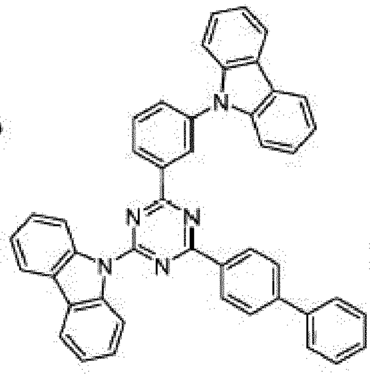

(G28),

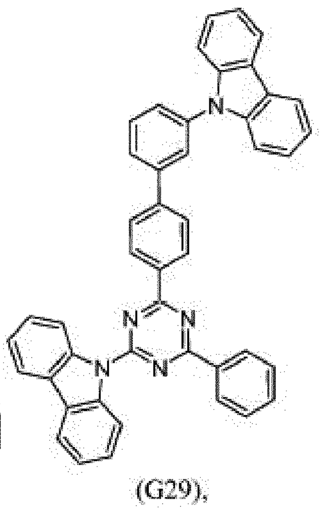

(G29),

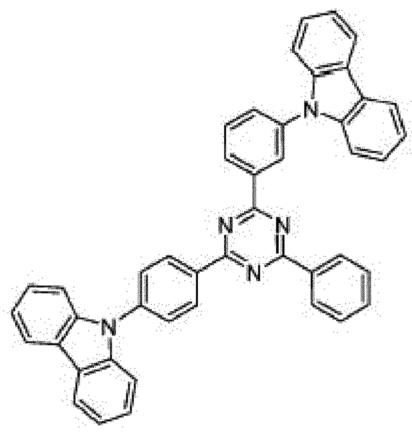

(G30),

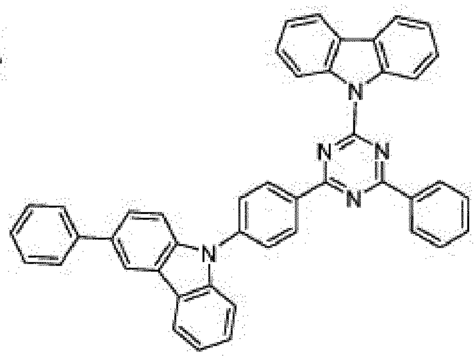

(G31).

12. Couche semi-conductrice organique comprenant au moins une composition selon l'une quelconque des revendications précédentes 1 à 11.

13. Dispositif électronique organique comprenant au moins une couche semi-conductrice organique selon la revendication 12.

14. Dispositif électronique organique selon la revendication 13, comprenant en outre au moins une anode et au moins une cathode, préférablement la couche semi-conductrice organique étant agencée entre l'anode et la cathode.

**15.** Dispositif électronique organique selon l'une quelconque des revendications précédentes 13 et 14, le dispositif électronique organique étant un dispositif luminescent, un transistor en film mince, une batterie, un dispositif d'affichage ou une cellule photovoltaïque, préférablement un dispositif luminescent.

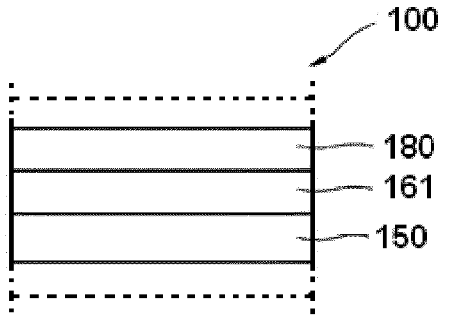


FIG. 1

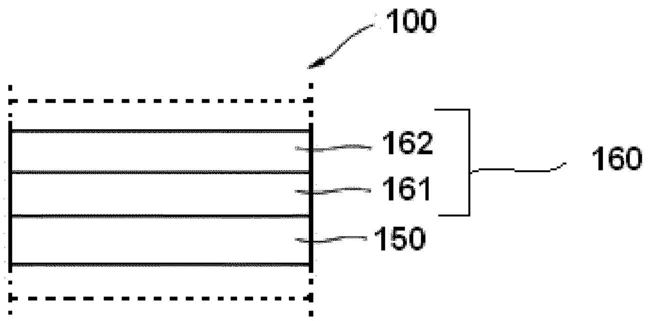


FIG. 2

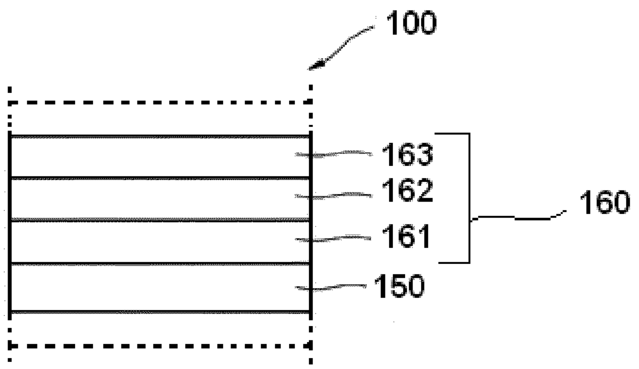


FIG. 3

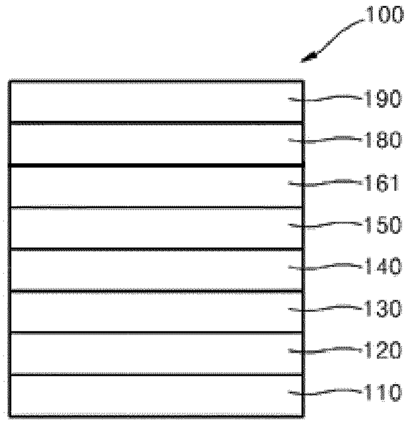


FIG. 4

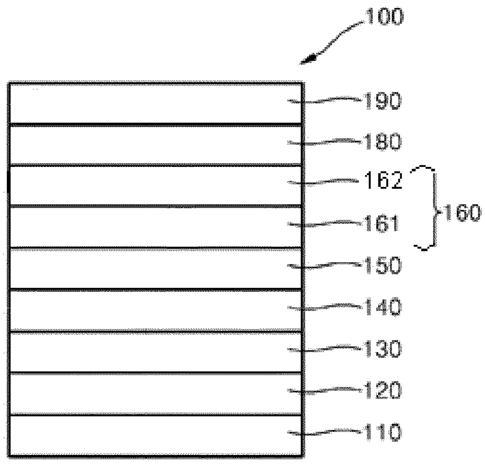


FIG. 5

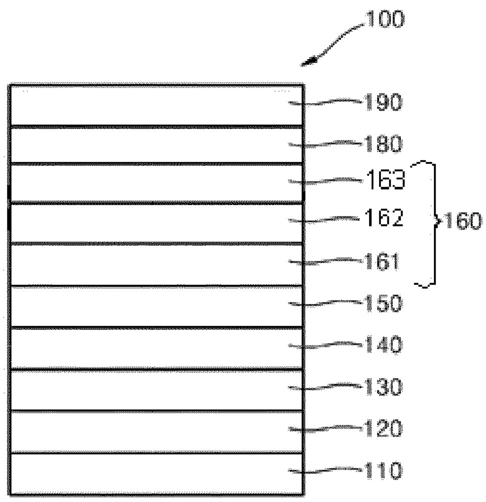


FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150171340 A1
- WO 2019009591 A1
- WO 2018110958 A1
- WO 2013079217 A1
- WO 2013079676 A1
- US 6140763 A
- US 6614176 B
- US 2016248022 A
- US 2015171340 A


### Non-patent literature cited in the description


- *CHEMICAL ABSTRACTS,* 25387-93-3
- *CHEMICAL ABSTRACTS,* 1242056-42-3
- *CHEMICAL ABSTRACTS,* 1198399-61-9